(19) Europäisches Patentamt
European Patent Office
Office européen des brevets

(11) Veröffentlichungsnummer: **0 384 276 B1**

(12) # EUROPÄISCHE PATENTSCHRIFT

(45) Veröffentlichungstag der Patentschrift: **10.11.93**

(51) Int. Cl.[5]: **C09B 62/507**, D06P 1/384, C07C 317/28, C09B 29/01

(21) Anmeldenummer: **90102848.0**

(22) Anmeldetag: **14.02.90**

(54) **Wasserlösliche Azoverbindungen, Verfahren zu ihrer Herstellung und ihre Verwendung als Farbstoffe.**

(30) Priorität: **18.02.89 DE 3905074**

(43) Veröffentlichungstag der Anmeldung: **29.08.90 Patentblatt 90/35**

(45) Bekanntmachung des Hinweises auf die Patenterteilung: **10.11.93 Patentblatt 93/45**

(84) Benannte Vertragsstaaten: **BE CH DE ES FR GB IT LI**

(56) Entgegenhaltungen:
**DE-A- 2 126 143**
**DE-A- 3 502 991**

(73) Patentinhaber: **HOECHST AKTIENGESELLSCHAFT**
**D-65926 Frankfurt(DE)**

(72) Erfinder: **Springer, Hartmut, Dr.**
**Am Erdbeerstein 27**
**D-6240 Königstein/Taunus(DE)**
Erfinder: **Hussong, Kurt, Dr.**
**Am Flachsland 56**
**D-6233 Kelkheim (Taunus)(DE)**

**EP 0 384 276 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung liegt auf dem Gebiet der faserreaktiven Farbstoffe.

Es wurden neue wasserlösliche Azoverbindungen entsprechend der allgemeinen Formel (1)

$$D - N = N - K \quad (1)$$

gefunden, die wertvolle faserreaktive Farbstoffeigenschaften besitzen.

In dieser Formel (1) bedeuten:

D ist ein Rest der allgemeinen Formel (2)

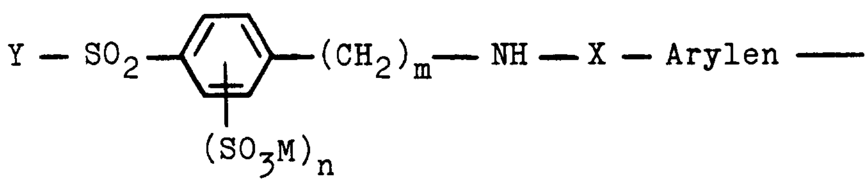

(2)

in welcher

Y die Vinylgruppe oder eine Gruppe der allgemeinen Formel (3)

$$-CH_2-CH_2-Z \quad (3)$$

ist, in welcher

Z ein Substituent ist, der durch ein Alkali unter Bildung der Vinylgruppe eliminierbar ist,

X die Carbonyl- oder Sulfonylgruppe ist,

Arylen ein Phenylen- oder Naphthylenrest ist, die beide durch eine Sulfogruppe, eine Alkylgruppe von 1 bis 4 C-Atomen, wie Ethyl- und insbesondere Methylgruppe, eine Alkoxygruppe von 1 bis 4 C-Atomen, wie Ethoxy- und insbesondere Methoxygruppe, eine Carboxygruppe oder ein Halogenatom, wie Chlor- und Bromatom, substituiert sein können,

m die Zahl 1 oder 2 ist,

n für die Zahl Null oder 1, bevorzugt Null, steht (wobei im Fall n = Null die Gruppe ein Wasserstoffatom bedeutet) und

M ein Wasserstoffatom oder ein salzbildendes Metallatom, wie insbesondere ein Alkalimetallatom, wie beispielsweise Natrium, Kalium oder Lithium, ist;

K ist ein Rest einer einfach ankuppelbaren wasserlöslichen Kupplungskomponente, die noch eine Azogruppe enthalten kann, oder der Rest einer doppelankuppelbaren wasserlöslichen Kupplungskomponente, jeweils aus der Reihe der Aminobenzole, der Phenole, insbesondere deren Sulfonsäuren und Carbonsäuren, der Naphthole, insbesondere deren Sulfonsäuren und Carbonsäuren, der Aminonaphthole, insbesondere deren Sulfonsäuren, der Acylamino-naphthole, insbesondere deren Sulfonsäuren, mit dem Acylrest einer Alkan- oder Alkencarbonsäure mit jeweils 1 bis 4 bzw. 2 bis 4 C-Atomen im Alkyl- bzw. Alkenylrest oder einer aromatischen Carbonsäure, wie der Benzoesäure, oder einer aromatischen Sulfonsäure, wie der Benzol- oder Toluolsulfonsäure, oder einer N-substituierten Carbaminsäure, wie der N-Phenylureido-Rest, oder aus der Reihe der Dihydroxynaphthalinsulfonsäuren, der Phenylazo- und Naphthylazo-aminonaphtholsulfonsäuren, der 5-Pyrazolone und 5-Aminopyrazole, der Acetoacetylarylide, der 2-Hydroxy-6-pyridone und der Hydroxychinoline, wobei K neben den in Farbstoffen üblichen Substituenten auch eine faserreaktive Gruppe enthalten kann, wie bspw. eine Gruppe $-SO_2-Y$ mit Y der obigen Bedeutung oder eine 4-Fluor- oder 4-Chlor-6-amino-s-triazin-2-ylamino-Gruppe, deren 6-ständige Aminogruppe durch Alkyl von 1 bis 4 C-Atomen und/oder Phenyl mono- oder disubstituiert sein kann, wobei der Phenylrest durch Subtituenten aus der Gruppe Sulfo, Carboxy, Methoxy, Ethoxy, Methyl, Chlor, Brom und $-SO_2-Y$ mit Y der obigen Bedeutung substituiert sein kann.

Ist Arylen ein Phenylenrest, so ist dieser bevorzugt unsubstituiert, und die Gruppe X ist an diesen Benzolring bevorzugt in meta- oder para-Stellung zur freien Bindung, die zur Azogruppe führt, gebunden. Ist Arylen ein Naphthylenrest, so ist X bevorzugt die Sulfonylgruppe, und der Naphthylenrest ist bevorzugt durch eine Sulfogruppe in ortho-Stellung zur freien Bindung, die zur Azogruppe führt, substituiert.

Alkalisch eliminierbare Substituenten Z sind beispielsweise Halogenatome, wie das Bromatom und Chloratom, Estergruppen organischer Carbon- und Sulfonsäuren, wie ein Alkanoyloxyrest von 2 bis 5 C-Atomen, beispielsweise die Acetyloxygruppe, oder ein Sulfobenzoyloxy-, Benzoyloxy-, Phenylsulfonyloxy- oder Toluylsulfonyloxy-Rest, desweiteren beispielsweise die sauren Estergruppen der Phosphorsäure, der Schwefelsäure und der Thioschwefelsäure (Phosphato- bzw. Sulfato- bzw. Thiosulfatogruppen), ebenso Dialkylaminogruppen mit Alkylgruppen von jeweils 1 bis 4 C-Atomen, wie die Dimethylamino- und Diethylaminogruppe.

Bevorzugt ist Y die Vinylgruppe und insbesondere die β-Sulfatoethyl-Gruppe.

Die Gruppen "Sulfo", "Carboxy", "Thiosulfato", "Phosphato" und "Sulfato" schließen sowohl deren Säureform als auch deren Salzform ein. Demgemäß bedeuten Sulfogruppen Gruppen entsprechend der allgemeinen Formel $-SO_3M$ , Carboxygruppen Gruppen entsprechend der allgemeinen Formel -COOM , Thiosulfatogruppen Gruppen entsprechend der allgemeinen Formel $-S-SO_3M$ , Phosphatogruppen Gruppen entsprechend der allgemeinen Formel $-OPO_3M_2$ und Sulfatogruppen entsprechend Gruppen der allgemeinen Formel $-OSO_3M$ , jeweils mit M der obengenannten Bedeutung.

Von den erfindungsgemäßen Verbindungen der allgemeinen Formel (1) können beispielsweise solche Verbindungen hervorgehoben werden, in welchen K einen Rest der nachstehenden Formel (4a), (4b), (4c), (4d), (4e), (4f), (4g), (4h), (4i), (4k), (4m), (4n), (4p), (4q), (4r), (4s), (4t) (4v), (4w) und (4x) bedeuten:

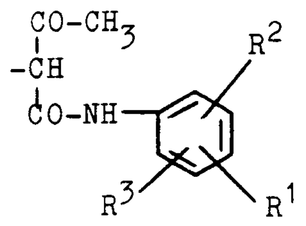

(4a)

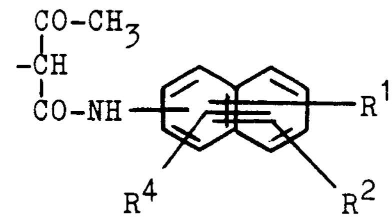

(4b)

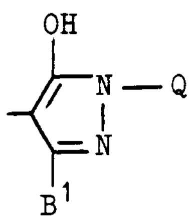

(4c)

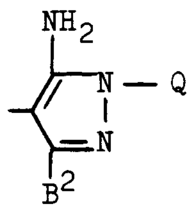

(4d)

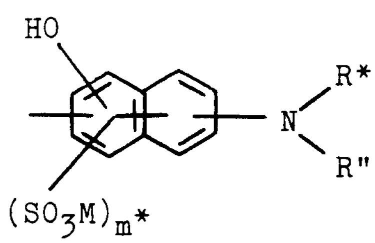

(4e)

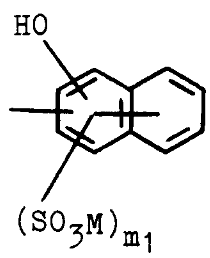

(4f)

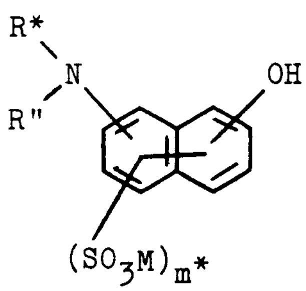

(4g)

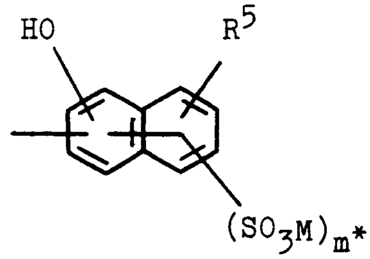

(4h)

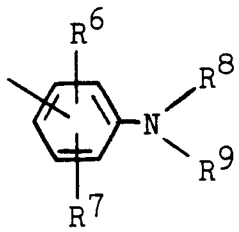

(4i)

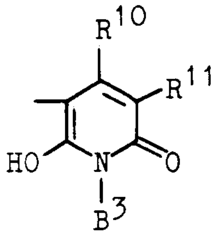

(4k)

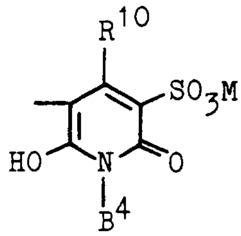

(4m)

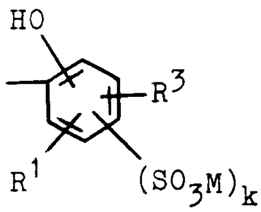

(4n)

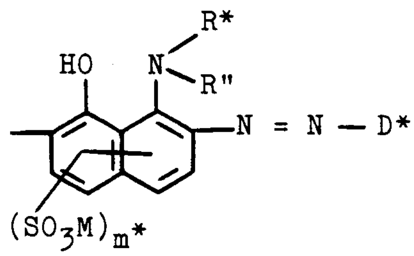

(4p)

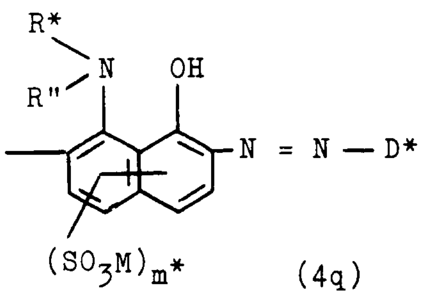

(4q)

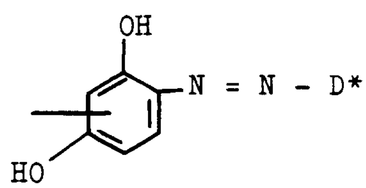

(4r)

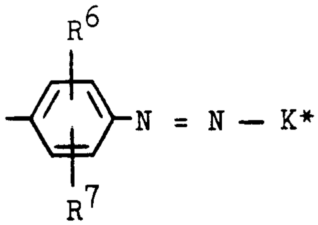

(4s)

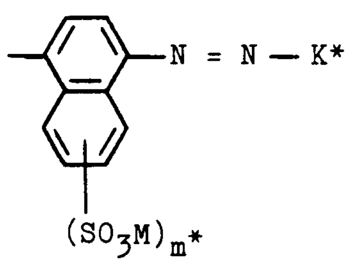

(4t)

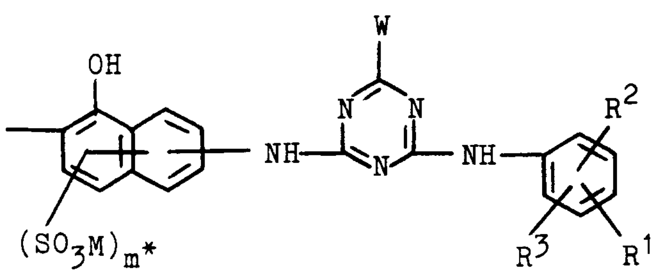

(4v)

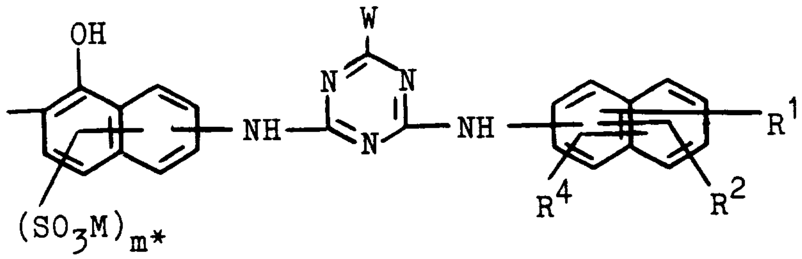

(4w)

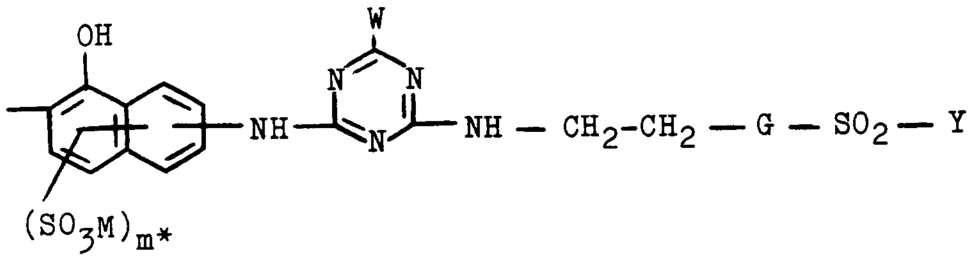

(4x)

In diesen Formeln bedeuten:

- $R^1$ ist ein Wasserstoffatom oder eine Carboxy- oder Sulfogruppe oder eine Gruppe der allgemeinen Formel $-G^*-SO_2-Y$ , in welcher
- Y eine der obengenannten Bedeutungen hat und
- $G^*$ eine direkte Bindung, die Methylen- oder Ethylengruppe oder die Propylenoxygruppe der Formel $-O-(CH_2)_3-$oder

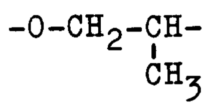

ist;

- $R^2$ ist ein Wasserstoffatom, eine Alkylgruppe von 1 bis 4 C-Atomen, wie insbesondere eine Methyl- oder Ethylgruppe, eine Alkoxygruppe von 1 bis 4 C-Atomen, wie insbesondere eine Methoxy- oder Ethoxygruppe, ein Chlor- oder Bromatom oder eine Carboxy- oder Sulfogruppe;
- $R^3$ ist ein Wasserstoffatom, eine Alkylgruppe von 1 bis 4 C-Atomen, wie insbesondere die Methyl- oder Ethylgruppe, eine Alkoxygruppe von 1 bis 4 C-Atomen, wie insbesondere die Methoxy- oder Ethoxygruppe, ein Chlor- oder Bromatom;
- $R^4$ ist ein Wasserstoffatom, eine Sulfo- oder Carboxygruppe;
- $B^1$ ist eine Alkylgruppe von 1 bis 4 C-Atomen, wie insbesondere die Methylgruppe, eine Carboxygruppe, eine Carbalkoxygruppe von 2 bis 5 C-Atomen, die Carbamoylgruppe oder ein gegebenenfalls durch Sulfo, Carboxy, Methyl, Ethyl, Methoxy, Ethoxy und/oder Chlor substituierter Phenylrest;
- $B^2$ ist eine Alkylgruppe von 1 bis 4 C-Atomen, wie insbesondere die Methylgruppe, eine Carboxygruppe, eine Carbalkoxygruppe von 2 bis 5 C-Atomen, die Carbamoylgruppe oder ein Phenylrest, der durch 1 oder 2 Substituenten aus der Gruppe Alkyl von 1 bis 4 C-Atomen, Alkoxy von 1 bis 4 C-Atomen, Chlor, Brom und Sulfo substituiert sein kann;
- Q ist ein Phenylrest, der substituiert sein kann, wie beispielsweise durch 1, 2 oder 3, bevorzugt 1 oder 2, Substituenten aus der Gruppe Chlor, Brom, Methyl, Ethyl, Methoxy, Ethoxy, Carboxy, Sulfo und Alkanoylamino, wie Acetylamino, und/oder durch eine Gruppe der allgemeinen Formel $-SO_2-Y$ mit Y der obengenannten Bedeutung, oder ist ein Naphthylrest, der durch 1, 2 oder 3 Sulfogruppen und gegebenenfalls durch eine Alkylgruppe von 1 bis 4 C-Atomen, eine Alkoxygruppe von 1 bis 4 C-Atomen, ein Chloratom

oder eine Alkanoylaminogruppe von 2 bis 5 C-Atomen und/oder durch eine Gruppe der allgemeinen Formel $-SO_2-Y$ mit Y der obengenannten Bedeutung substituiert sein kann;

R* ist ein Wasserstoffatom oder eine Alkylgruppe von 1 bis 4 C-Atomen, die durch einen Phenylrest oder eine durch Sulfo und/oder $-SO_2-Y$ mit Y der obigen Bedeutung substituierten Phenylrest substituiert sein kann;

R'' ist ein Wasserstoffatom, eine Alkylgruppe von 1 bis 4 C-Atomen, die durch einen Phenylrest substituiert sein kann, oder ist ein Phenylrest, der durch 1 oder 2 Substituenten aus der Gruppe Alkyl von 1 bis 4 C-Atomen, Alkoxy von 1 bis 4 C-Atomen, Chlor, Brom, Sulfo und $-SO_2-Y$ mit Y der obigen Bedeutung substituiert sein kann;

$R^5$ ist die Phenylureidogruppe, eine Alkanoylaminogruppe von 2 bis 5 C-Atomen, Alkenoylaminogruppe von 3 bis 5 C-Atomen, wie Acetylamino-, Propionylamino- oder Acryloylaminogruppe, oder eine Benzoylaminogruppe, die durch Substituenten aus der Gruppe Chlor, Methyl, Methoxy, Nitro, Sulfo, Carboxy und $-SO_2-Y$ mit Y der obigen Bedeutung substituiert sein kann, bevorzugt der Acetylamino- oder Benzoylamino-Rest;

$R^6$ ist ein Wasserstoffatom oder eine Alkylgruppe von 1 bis 4 C-Atomen, eine Alkoxygruppe von 1 bis 4 C-Atomen, eine Sulfogruppe, eine Carboxygruppe, eine Carbalkoxygruppe von 2 bis 5 C-Atomen, ein Halogenatom, wie Brom- oder Chloratom, oder eine Alkoxygruppe von 1 bis 4 C-Atomen, die durch eine Hydroxy-, Acetyloxy-, Carboxy-, Carbamoyl- oder Cyangruppe oder ein Halogenatom, wie Chloratom, substituiert ist;

$R^7$ ist ein Wasserstoffatom, eine Alkylgruppe von 1 bis 4 C-Atomen, eine Alkoxygruppe von 1 bis 4 C-Atomen, ein Halogenatom, wie Brom- oder Chloratom, die Cyangruppe, die Trifluormethylgruppe, eine Alkoxygruppe von 1 bis 4 C-Atomen, die durch eine Hydroxy-, Acetyloxy-, Carboxy-, Carbamoyl- oder Cyangruppe oder durch ein Halogenatom, wie Chloratom, substituiert ist, oder ist eine Alkanoylaminogruppe von 2 bis 5 C-Atomen, die durch Chlor, Brom, Alkoxy von 1 bis 4 C-Atomen, Phenoxy, Phenyl, Hydroxy, Carboxy oder Sulfo substituiert sein kann, oder ist eine Alkenoylaminogruppe von 3 bis 5 C-Atomen, die durch Chlor, Brom, Carboxy oder Sulfo substituiert sein kann, oder ist die Benzoylaminogruppe, die im Benzolkern substituiert sein kann, beispielsweise durch Chlor, Methyl und/oder Sulfo, oder ist eine Alkylsulfonylgruppe von 1 bis 4 C-Atomen oder die Phenylsulfonylgruppe, die im Benzolkern substituiert sein kann, beispielsweise durch Chlor, Methyl und/oder Sulfo, oder ist eine Alkylsulfonylaminogruppe von 1 bis 4 C-Atomen, die durch Hydroxy, Sulfato, Chlor, Brom oder Alkoxy von 1 bis 4 C-Atomen substituiert sein kann, oder ist die Phenylsulfonylaminogruppe, die im Benzolkern substituiert sein kann, beispielsweise durch Chlor, Methyl und/oder Sulfo, oder ist die Carbamoylgruppe, die am Stickstoffatom durch 1 oder 2 Substituenten mono- oder disubstituiert sein kann, wobei die Substituenten der Gruppe Alkyl von 1 bis 4 C-Atomen, durch beispielsweise Hydroxy, Sulfo, Carboxy, Sulfato oder Phenyl substituiertes Alkyl von 1 bis 4 C-Atomen, Cycloalkyl mit 5 bis 8 C-Atomen, Phenyl und durch beispielsweise Chlor, Sulfo, Methyl, Methoxy und/oder Carboxy substituiertes Phenyl angehören, oder ist die Sulfamoylgruppe, die am Stickstoffatom durch 1 oder 2 Substituenten mono- oder disubstituiert sein kann, wobei die Substituenten der Gruppe Alkyl von 1 bis 4 C-Atomen, durch beispielsweise Hydroxy, Sulfo, Carboxy, Sulfato oder Phenyl substituiertes Alkyl von 1 bis 4 C-Atomen, Cycloalkyl mit 5 bis 8 C-Atomen, Phenyl und durch beispielsweise Chlor, Sulfo, Methyl, Methoxy und/oder Carboxy substituiertes Phenyl angehören, oder ist die Ureidogruppe oder eine Ureidogruppe, die am endständigen Stickstoffatom durch 1 oder 2 Substituenten mono- oder disubstituiert sein kann, wobei die Substituenten der Gruppe Alkyl von 1 bis 4 C-Atomen, durch beispielsweise Hydroxy, Sulfo, Carboxy, Sulfato oder Phenyl substituiertes Alkyl von 1 bis 4 C-Atomen, Cycloalkyl mit 5 bis 8 C-Atomen, Phenyl und durch beispielsweise Chlor, Sulfo, Methyl, Methoxy und/oder Carboxy substituiertes Phenyl angehören;

$R^8$ ist ein Wasserstoffatom oder eine Alkylgruppe von 1 bis 4 C-Atomen, die durch beispielsweise Hydroxy, Sulfo, Carboxy, Sulfato, eine Gruppe $-SO_2-Y$ mit Y der obigen Bedeutung oder Phenyl substituiert sein kann, oder ist eine Alkenylgruppe von 2 bis 4 C-Atomen, die durch eine Carboxy- oder Sulfogruppe oder durch ein Chlor- oder Bromatom substituiert sein kann, oder ist ein Cycloalkylrest von 5 bis 8 C-Atomen;

$R^9$ ist ein Wasserstoffatom oder eine Alkylgruppe von 1 bis 4 C-Atomen, die substituiert sein kann, beispielsweise durch Hydroxy, Sulfo, Carboxy, Sulfato, Phenyl oder $-SO_2-Y$ mit Y obiger Bedeutung, oder ist eine Alkenylgruppe von 2 bis 5 C-Atomen, die durch eine

Carboxy- oder Sulfogruppe oder $-SO_2-Y$ mit Y obiger Bedeutung oder durch ein Chlor- oder Bromatom substituiert sein kann, oder

$R^9$ ist ein Cycloalkylrest von 5 bis 8 C-Atomen oder ein Phenylrest, der substituiert sein kann, beispielsweise durch Substituenten aus der Gruppe Chlor, Sulfo, Methyl, Methoxy, Carboxy und $-SO_2-Y$ mit Y obiger Bedeutung, oder

$R^9$ ist ein Naphthylrest, der durch 1, 2 oder 3 Sulfogruppen und gegebenenfalls durch eine oder zwei Gruppen $-SO_2-Y$ mit Y obiger Bedeutung oder ein Chloratom, eine Alkoxygruppe von 1 bis 4 C-Atomen, eine Alkylgruppe von 1 bis 4 C-Atomen, eine Alkanoylaminogruppe von 2 bis 5 C-Atomen oder eine gegebenenfalls durch Sulfo substituierte Benzoylaminogruppe substituiert ist, oder ist ein heterocyclischer Rest, der einen oder zwei ankondensierte carbocyclische Ringe aufweisen kann, wobei die carbocyclischen Ringe noch substituiert sein können und der heterocyclische Rest an den C-Atomen und/oder an den Heteroatomen durch gegebenenfalls substituierte Alkylgruppen von 1 bis 4 C-Atomen und/oder gegebenenfalls substituierte Phenylreste substituiert sein kann, oder

$R^8$ und $R^9$ stellen zusammen mit dem Stickstoffatom und gegebenenfalls einem weiteren Heteroatom einen gesättigten heterocyclischen Rest dar, wie beispielsweise den Piperidino-, Morpholino- oder Piperazinorest;

$R^{10}$ ist ein Wasserstoffatom oder eine Alkylgruppe von 1 bis 4 C-Atomen oder eine durch Alkoxy von 1 bis 4 C-Atomen oder Cyan substituierte Alkylgruppe von 1 bis 4 C-Atomen;

$R^{11}$ ist ein Wasserstoffatom oder eine Sulfogruppe oder eine Sulfoalkylgruppe mit einem Alkylenrest von 1 bis 4 C-Atomen, wie Sulfomethylgruppe, oder eine Cyan- oder Carbamoylgruppe;

$B^3$ ist ein Wasserstoffatom oder eine Alkylgruppe von 1 bis 6 C-Atomen, bevorzugt von 1 bis 4 C-Atomen, die durch Phenyl, Sulfo, Sulfophenyl oder $-SO_2-Y$ mit Y obiger Bedeutung substituiert sein kann;

$B^4$ ist ein Wasserstoffatom, eine Alkylgruppe von 1 bis 4 C-Atomen, eine durch eine Alkoxygruppe von 1 bis 4 C-Atomen, wie Methoxygruppe, oder eine durch eine Sulfo-, Carboxy-, Sulfato-, Acetylamino-, Benzoylamino- oder Cyangruppe oder eine Gruppe der Formel $-SO_2-Y$ mit Y obiger Bedeutung substituierte Alkylgruppe von 1 bis 4 C-Atomen oder ist eine Alkenylgruppe von 2 bis 4 C-Atomen, die Cyclohexylgruppe, die Phenylgruppe oder eine durch Substituenten aus der Gruppe Carboxy, Sulfo, Benzoylamino, Acetylamino, $-SO_2-Y$ mit Y obiger Bedeutung und Chlor substituierte Phenylgruppe;

k ist die Zahl Null oder 1 (wobei im Falle k = Null diese Gruppe für ein Wasserstoffatom steht);

m* steht für die Zahl 1 oder 2;

$m_1$ steht für die Zahl 1, 2 oder 3;

D* hat eine der für die allgemeine Formel (2) angegebenen Bedeutungen, hierbei bevorzugt die gleiche Bedeutung, oder ist ein Phenylrest, der durch 1, 2 oder 3, bevorzugt 1 oder 2, Substituenten aus der Gruppe Alkyl von 1 bis 4 C-Atomen, Alkoxy von 1 bis 4 C-Atomen, Chlor, Brom, Hydroxy, Carboxy, Sulfo, Carbamoyl, Sulfamoyl und Alkanoylamino, bevorzugt hiervon Methyl, Methoxy, Ethoxy, Chlor, Sulfo, Carboxy und Hydroxy und/oder durch eine Gruppe der Formel $-SO_2-Y$ mit Y der obengenannten Bedeutung substituiert sein kann, wobei bevorzugt einer dieser Substituenten eine Sulfo- oder Carboxygruppe ist, oder ist ein Naphthylrest, der durch 1, 2 oder 3 Sulfogruppen oder durch 1 oder 2 Sulfogruppen und 1 oder 2 Gruppen der allgemeinen Formel $-SO_2-Y$ mit Y der obengenannten Bedeutung oder nur durch eine solche Gruppe $-SO_2-Y$ substituiert ist;

K* ist ein Rest aus einer der oben genannten und definierten allgemeinen Formeln (4a) bis (4m), wobei K und K* zueinander gleiche oder voneinander verschiedene Bedeutungen besitzen können;

W ist ein Fluor- oder Chloratom oder eine Methoxy- oder Methylthiogruppe;

G ist eine Methylen- oder Phenylengruppe oder eine durch eine Sulfogruppe substituierte Phenylengruppe;

M hat eine der oben genannten Bedeutungen.

Die einzelnen Formelglieder können zueinander gleiche oder voneinander verschiedene Bedeutungen haben.

Die in den obigen Formeln (4e), (4f), (4g), (4h), (4i) und (4n) befindlichen freien Bindungen, welche zur Azogruppe führen, bzw. die Azogruppe in Formel (4p) und (4q) befinden sich in ortho-Stellung zur Hydroxy- bzw. Aminogruppe gebunden. Bevorzugt steht diese Hydroxygruppe in $\alpha$-Stellung an den Naphthalinrest

gebunden.

Alkylgruppen von 1 bis 4 C-Atomen sind bevorzugt die Ethyl- und insbesondere die Methylgruppe; Alkoxygruppen von 1 bis 4 C-Atomen sind bevorzugt die Ethoxy- und insbesondere die Methoxygruppe; Alkanoylaminogruppen von 2 bis 5 C-Atomen sind bevorzugt die Propionylaminogruppe und insbesondere die Acetylaminogruppe und Carbalkoxygruppen von 2 bis 5 C-Atomen, bevorzugt die Carbomethoxy- und Carbethoxygruppe.

Von den erfindungsgemäßen Verbindungen der allgemeinen Formel (1) sind insbesondere diejenigen bevorzugt, in welchen K einen Rest der allgemeinen Formel (4a), (4b), (4c), (4e), (4g), (4h), (4i), (4p), (4q), (4v), (4w) oder (4x) bedeutet, in welchen wiederum die einzelnen Formelglieder die folgenden bevorzugten Bedeutungen besitzen:

$B^1$ ist eine Carboxy- oder Methylgruppe;

Q ist ein Phenylrest, der durch 1 oder 2 Substituenten substituiert sein kann, die aus der folgenden Menge an Substituenten ausgewählt sind: 2 Methyl, 2 Methoxy, 1 Chlor oder Brom, 2 Sulfo, 1 Carboxy und 1 Vinylsulfonyl oder β-Sulfatoethylsulfonyl;

$R^1$ ist eine Carboxy-, Sulfo- oder β-Sulfatoethylsulfonyl-Gruppe;

$R^5$ ist die Acetylamino- oder Propionylaminogruppe oder eine Benzoylaminogruppe, die durch 1 oder 2 Substituenten aus der Gruppe Chlor, Methyl, Methoxy, Nitro, Sulfo und β-Sulfatoethylsulfonyl substituiert sein kann;

$R^8$ ist eine Alkylgruppe von 1 bis 4 C-Atomen, die durch Hydroxy, Sulfato oder β-Sulfatoethylsulfonyl substituiert sein kann;

R* und R'' sind beide ein Wasserstoffatom.

Insbesondere bevorzugt sind solche Verbindungen der allgemeinen Formel (1), in welchen K eine Gruppe der allgemeinen Formel $-SO_2-Y$ mit Y einem Rest der allgemeinen Formel (3) enthält, wobei Y bevorzugt eine β-Sulfatoethyl-Gruppe ist.

Die vorliegende Erfindung betrifft weiterhin Verfahren zur Herstellung der erfindungsgemäßen Azoverbindungen der allgemeinen Formel (1), beispielsweise durch Kupplungsreaktion der Diazoniumverbindung einer Aminoverbindung der allgemeinen Formel (5)

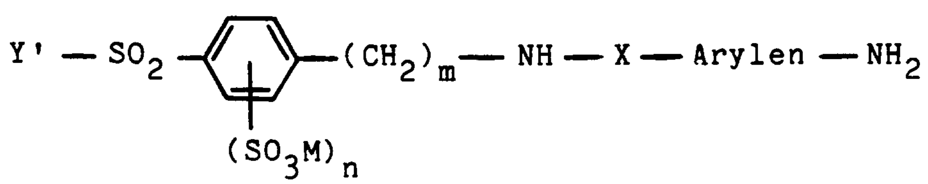

(5)

in welcher Y' eine der Bedeutungen von Y hat oder die β-Hydroxyethyl-Gruppe ist und Arylen, X, M, m und n die obengenannten Bedeutungen haben, mit einer Kupplungskomponente der allgemeinen Formel H-K mit K der obengenannten Bedeutung; sofern K, wie oben angegeben, eine bivalente Kupplungskomponente ist, kann eine Disazoverbindung, sofern diese erwünscht ist, durch Umsetzung dieser bivalenten Kupplungskomponente mit der zweifach äquimolaren Menge der Diazokomponente hergestellt werden. Im Falle der Verwendung einer Verbindung (5) mit Y' gleich einer β-Hydroxyethyl-Gruppe wird diese β-Hydroxyethyl-Gruppe in der gebildeten Azoverbindung in eine Gruppe Y der erfindungsgemäßen Azoverbindung (1), wie später noch angegeben, übergeführt.

Die Diazotierungs- und Kupplungsreaktionen erfolgen in üblicher und altbekannter Weise, so die Diazotierung des Amins (5) in der Regel bei einer Temperatur zwischen -5 °C und +15 °C und einem pH-Wert unterhalb von 2 mittels einer starken Säure und Alkalinitrit in bevorzugt wäßrigem Medium und die Kupplungsreaktion in der Regel bei einem pH-Wert zwischen 1 und 4,5 im Falle einer aminogruppenhaltigen Kupplungskomponente und bei einem pH-Wert zwischen 3 und 7,5 im Falle einer hydroxygruppenhaltigen Kupplungskomponente und bei einer Temperatur zwischen 0 und 30 °C in bevorzugt wäßrigem Medium.

Ist die Kupplungskomponente eine bivalente, doppelankuppelbare Verbindung, enthält sie beispielsweise eine kupplungsfähige Aminogruppe und gleichzeitig eine kupplungsfähige Hydroxygruppe, so kann zur Herstellung einer Disazoverbindung die Kupplung zunächst mit dem ersten Mol der Diazoniumverbindung des Amins im sauren pH-Bereich zur Monoazoverbindung erfolgen und die zweite Kupplungsreaktion mit dem zweiten Mol der Diazoniumverbindung des Amins anschließend im schwach sauren bis schwach alkalischen Bereich. Diese Verfahrensweise gilt beispielsweise für die Verbindungen entsprechend den allgemeinen Formeln (4p) und (4q), so durch Kupplung der Aminonaphtholsulfonsäure zunächst mit dem ersten Mol der Diazoniumverbindung des Amins der allgemeinen Formel (5) oder eines anderen aromatischen Amins entsprechend der allgemeinen Formel D*-$NH_2$ mit D* der obengenannten anderen Bedeutung als D im stark sauren Medium und sodann durch Kupplung der gebildeten Monoazoverbindung mit dem zweiten Mol einer Diazoniumverbindung eines Amins D*-$NH_2$ mit D* der obengenannten Bedeutung im schwach sauren, neutralen oder schwach alkalischen Bereich, wobei D* zwingend eine der für D angegebenen Bedeutungen besitzt, sofern die erste Kupplungsreaktion nicht mit einer Diazoniumverbindung eines Amins (5) durchgeführt wurde, so insbesondere zunächst bei einem pH-Wert von etwa 0,5 bis 2,5 und anschließend bei einem pH-Wert zwischen 4 und 6,5, wobei, sofern die Diazoniumverbindung der Aminoverbindung (5) in beiden Kupplungsreaktionen identisch ist, die erste und zweite Kupplungsreaktion in ein und demselben Ansatz, zunächst im angegebenen sauren Bereich und sodann im schwach sauren bis schwach alkalischen Bereich, durchgeführt werden kann. Zur Herstellung einer Disazoverbindung entsprechend der allgemeinen Formel (4r) erfolgt die Umsetzung der Kupplungskomponente Resorcin mit der bzw. den Diazoniumverbindung(en) vorteilhaft zunächst bei einem pH-Wert zwischen 0,8 und 2 und sodann bei einem pH-Wert zwischen 6 und 7,5.

Disazoverbindungen entsprechend der allgemeinen Formel (1), deren Rest K dem Rest einer Azoverbindung entspricht, welcher aus einer kupplungsfähigen Diazokomponente und einer Kupplungskomponente zusammengesetzt ist, wie beispielsweise der Rest entsprechend der allgemeinen Formel (4s) oder (4t), lassen sich auch erfindungsgemäß in der Weise herstellen, daß man zunächst die Diazoniumverbindung eines Amins (5) mit der aminogruppenhaltigen und somit diazotierbaren Kupplungskomponente, wie beispielsweise in den Formeln (4s) und (4t) die durch die Substituenten $R^6$ und $R^7$ substituierten Anilin- bzw. Sulfo-aminonaphthalin-Komponenten, kuppelt und in der so gebildeten Amino-Azoverbindung die Aminogruppe diazotiert und mit einer Kupplungskomponente, wie beispielsweise der Kupplungskomponente H-K* , zur Disazoverbindung kuppelt.

Alle diese Umsetzungsmöglichkeiten zur Synthese von Disazoverbindungen sind analog den in der Literatur bekannten oder dem Fachmann geläufigen Methoden zur Synthese von Disazoverbindungen.

Kupplungskomponenten, die zur Herstellung der erfindungsgemäßen Farbstoffe verwendet werden können und beispielsweise den allgemeinen Formeln (4a) bis (4n) entsprechen, sind beispielsweise:
Resorcin, 2-Ethoxy-phenol, 4-Methylphenol, 3-Sulfophenol, Salicylsäure, 3-Sulfo-1-naphthol, 4-Sulfo-1-naphthol, 5-Sulfo-1-naphthol, 3,6-Disulfo-8-naphthol, 4,6-Disulfo-8-naphthol,1-Naphthol-3,8-disulfonsäure, 1 Amino-8-naphthol-4-sulfonsäure, 1-Amino-8-naphthol-5-sulfonsäure, 1-Amino-8-naphthol-2,4-disulfonsäure, 2-Amino-5-naphthol-7-sulfonsäure, 2-Amino-5-naphthol-1,7-disulfonsäure, 1-Amino-5-naphthol-7-sulfonsäure, 2-Amino-8-naphthol-6-sulfonsäure, 2-Amino-8-naphthol-3,6-disulfonsäure, 2-Amino-8-naphthol-4,6-disulfonsäure, 1-Amino-8-naphthol-3,6- oder -4,6-disulfonsäure, 1-Acryloylamino-8-naphthol-3,6- oder -4,6-disulfonsäure, 1-Propionylamino-8-naphthol-3,6- oder -4,6-disulfonsäure, 1-Acetylamino-8-naphthol-4-sulfonsäure, 1-Acetylamino-8-naphthol-3,6- oder -4,6-disulfonsäure, 1-Benzoylamino-8-naphthol-3,6- oder -4,6-disulfonsäure, 2-Naphthol-5,7-disulfonsäure, 2-Naphthol-3,6- und -6,8-disulfonsäure, 1,8-Dihydroxynaphthalin-3,6-disulfonsäure, 1,8-Dihydroxynaphthalin-6-sulfonsäure, 1-Naphthol-3,6,8-trisulfonsäure,2-Acetylamino-5-naphthol-7-sulfonsäure, 2-Benzoylamino-8-naphthol-6-sulfonsäure, 2-(p'-Tosylamino)-5-naphthol-7-sulfonsäure, 2-Acetylamino-8-naphthol-3,6-disulfonsäure, 2-Acetylamino-5-naphthol-1,7-disulfonsäure, 3-Benzoylamino-8-naphthol-6-sulfonsäure, 2-Phenylsulfonylamino-5-naphthol-7-sulfonsäure, 2-(N-Methyl-N-acetyl)-amino-8-naphthol-6-sulfonsäure, N-Ethyl-N-benzylanilin-3-sulfonsäure, N,N-Bis-($\beta$-hydroxyethyl)-anilin, N,N-Bis-($\beta$-sulfatoethyl-)anilin, N,N-Bis-($\beta$-hydroxyethyl)-2-methoxy-5-chlor-anilin, N-($\beta$-Sulfatoethyl)-2,5-dimethoxyanilin, N-($\beta$-Sulfatoethyl)-2-chloranilin, Acetoacetyl-2-naphthylamid-5-sulfonsäure, N-Acetoacetylanilin-3- oder -4-sulfonsäure, N-Acetoacetyl-2-methoxy-5-sulfo-anilin, N-Acetoacetyl 4 methoxy-3-sulfoanilin, N-Acetoacetyl-2-methoxy-5-methyl-4-sulfo-anilin, N-Acetoacetyl-2,5-dimethoxy-4-sulfo-anilin, N-Acetoacetyl-2-methoxy-5-methyl-4-($\beta$-sulfatoethylsulfonyl)-anilin, N-Acetoacetyl-2,5-dimethoxy-4-($\beta$-sulfatoethylsulfonyl)-anilin, N-Acetoacetyl-2-methoxy-5-($\beta$-sulfatoethylsulfonyl)-anilin, N-Acetoacetyl-4-($\beta$-sulfatoethylsulfonyl)-anilin, N-Acetoacetyl-3-($\beta$-sulfatoethylsulfonyl)-anilin, 1-(4'-$\beta$-Sulfatoethylsulfonyl-phenyl)-3-methyl-pyrazolon(5), 1-(4'-$\beta$-Sulfatoethylsulfonyl-phenyl)-3-carboxy-pyrazolon(5), 1-(4'-Sulfophenyl)-3-methyl-pyrazolon(5), 1-(4'-Sulfophenyl)-3-carboxy-pyrazolon(5), 1-(2'-Chlor-5'sulfo-phenyl)-3-methyl- oder -3-carboxy-pyrazolon(5), 1-(3'-Sulfophenyl)-3-carboxypyrazolon(5),1-(2'-Methoxy-4'-sulfophenyl)-3-carboxy-pyrazolon(5), 1-(3'-Sulfophenyl)-3-methyl-5-amino-pyrazol, 1-(4'-Sulfo-phenyl)-3-methyl-5-amino-pyrazol, 1-(2'-Methoxy-5'-sulfo-phenyl)-3-methyl-5-aminopyrazol, 1-(2'-Methoxy-5'-methyl-4'-sulfophenyl)-3-methyl-5-aminopyrazol, 1-(2'-Chlor-5'-sulfo-phenyl)-3-methyl-5-aminopyrazol, 1-(3'-Amino-4'-sulfo-phenyl)-3-carbethoxy-pyrazolon(5), 1-(4'-$\beta$-Sulfatoethylsulfonyl-phenyl)-3-carbethoxy-pyrazolon(5), 1-(3'-Amino-6'methyl-phenyl)-3-carboxy-pyrazolon-(5), 2-N-Methylamino-8-naphthol-6-sulfonsäure, 3-Carboxy-pyrazolon(5),1-Phenyl-3-carboxy-pyrazolon(5), 1-(4'-Nitrophenyl)-3-carboxypyrazolon(5), 1-(3'-Acetylaminophenyl)-3-carboxy-pyrazolon(5), 1-(3'-Carboxyphenyl)-3-methyl-pyrazolon(5), 2-Hydroxy-3-carboxy-naphthalin, 2-Hydroxy-6-carboxy-naphthalin, 8-Hydroxy-chinolin-5-sulfonsäure, 1,4-Dimethyl-2-hydroxy-6-pyridon-5-sulfonsäure, N-Sulfomethyl-anilin, 3-Acetylamino-5-naphthol-7-sulfonsäure, 2-Methylamino-8-naphthol-6-sulfonsäure, 1-[4'-Chlor-6'-(4''-β-sulfatoethylsulfonyl-phenyl)-amino-1',3',5'-triazin-2'-yl]-amino-8-naphthol-3,6-disulfonsäure, 1-[4'-Chlor-6'-(4''-β-sulfatoethylsulfonyl-phenyl)-amino-1',3',5'-triazin-2'-yl]-amino-8-naphthol-4,6-disulfonsäure, 2-[4'-Chlor-6'-(4''-β-sulfatoethylsulfonyl-phenyl)-amino-1',3',5'-triazin-2'-yl]-amino-8-naphthol-6-sulfonsäure, 3-[4'-Chlor-6'-(4''-β-sulfatoethylsulfonyl-phenyl)-amino-1',3',5'-triazin-2'-yl]-amino-8-naphthol-3,6-disulfonsäure, 1-(4'-Chlor-6'-methoxy-1',3',5'-triazin-2'-yl)-amino-8-naphthol-3,6-disulfonsäure, 1-(4'-Chlor-6'-methoxy-1',3',5'-triazin-2'-yl)-amino-8-naphthol-4,6-disulfonsäure, 2-(4'-Chlor-6'-methoxy-1',3',5'-triazin-2'-yl)-amino-8-naphthol-6-sulfonsäure, 3-(4'-Chlor-6'-methoxy-1',3',5'-triazin-2'-yl)-amino-8-naphthol-6-sulfonsäure, 1-[4'-Fluor-6'-(4''-β-Sulfatoethylsulfonyl-phenyl)-amino-1',3',5'-triazin-2'-yl]-amino-8-naphthol-3,6-disulfonsäure, 1-[4'-Fluor-6'-(4''-β-Sulfatoethylsulfonyl-phenyl)-amino-1',3',5'-triazin-2'-yl]-amino-8-naphthol-4,6-disulfonsäure, 2-[4'-Fluor-6'-(4''-β-Sulfatoethylsulfonyl-phenyl)-amino-1',3',5'-triazin-2'-yl]-amino-8-naphthol-6-sulfonsäure, 3-[4'-Fluor-6'-(4''-β-Sulfatoethylsulfonyl-phenyl)-amino-1',3',5'-triazin-2'-yl]-amino-8-naphthol-6-sulfonsäure, 1-(4'-β-Sulfatoethylsulfonyl-benzoyl)-amino-8-naphthol-3,6-disulfonsäure oder -4,6-disulfonsäure, 2- oder 3-(4'-β-Sulfatoethylsulfonyl-benzoyl)-amino-8-naphthol-6-sulfonsäure, 1-{4'-Chlor-6'-[β-(4''-β''-sulfatoethylsulfonyl-phenyl)-ethyl]-1',3',5'-triazin-2'-yl}-amino-8-naphthol-3,6- oder -4,6-disulfonsäure, 1-{4'-Chlor-6'-[β-(3''-β''-sulfatoethylsulfonyl-phenyl)-ethyl]-1',3',5'-triazin-2'-yl}-amino-8-naphthol-3,6- oder -4,6-disulfonsäure, 1-{4'-Chlor-6'-[β-(4''-sulfo-phenyl)-ethyl]-1',3',5'-triazin-2'-yl}-amino-8-naphthol-3,6- oder -4,6-disulfonsäure, 1-{4'-Chlor-6'-[β-(2'',5''-disulfophenyl)-ethyl]-1',3',5'-triazin-2'-yl}-amino-8-naphthol-3,6- oder -4,6-disulfonsäure, 1-{4'-Fluor-6'-[β-(3'',5''-disulfophenyl)-ethyl]-1',3',5'-triazin-2'-yl}-amino-8-naphthol-3,6- oder -4,6-disulfonsäure, 1-(β-Hydroxyethyl)-4-methyl-6-hydroxy-2-pyridon, 1-(β-Hydroxyethyl)-3-cyano-4-methyl-6-hydroxy-2-pyridon, 1-(β-Hydroxyethyl)-3-carbamoyl-4-methyl-6-hydroxy-2-pyridon, 1-(β-Hydroxyethyl)-4-methyl-6-hydroxy-2-pyridon-3-sulfonsäure, 1-(β-Sulfatoethyl)-4-methyl-6-hydroxy-2-pyridon, 1-(β-Sulfatoethyl)-3-cyano-4-methyl-6-hydroxy-2-pyridon, 1-(β-Sulfatoethyl)-3-carbamoyl-4-methyl-6-hydroxy-2-pyridon, 1-(β-Sulfatoethyl)-4-methyl-6-hydroxy-2-pyridon-3-sulfonsäure, 1-(β-Sulfatoethyl)-4-methyl-6-hydroxy-2-pyridon, 1-(β-Sulfatoethyl)-3-carbamoyl-4-methyl-6-hydroxy-2-pyridon, 1-(β-Sulfatoethyl)-4-methyl-6-hydroxy-2-pyridon-3-sulfonsäure, 1-Carboxymethyl-4-methyl-6-hydroxy-2-pyridon, 1-Carboxymethyl-3-cyano-4-methyl-6-hydroxy-2-pyridon, 1-Carboxymethyl-3-carbamoyl-4-methyl-6-hydroxy-2-pyridon, 1-Carboxymethyl-4-methyl-6-hydroxy-2-pyridon-3-sulfonsäure, 1-(β-Carboxyethyl)-4-methyl-6-hydroxy-2-pyridon, 1-(β-Carboxyethyl)-3-cyano-4-methyl-6-hydroxy-2-pyridon, 1-(β-Carboxyethyl)-3-carbamoyl-4-methyl-6-hydroxy-2-pyridon, 1-(β-Carboxyethyl)-4-methyl-6-hydroxy-2-pyridon-2-sulfonsäure, 1-(β-Acetylaminoethyl)-4-methyl-6-hydroxy-2-pyridon, 1-(β-Acetylaminoethyl)-3-cyano-4-methyl-6-hydroxy-2-pyridon, 1-(β-Acetylaminoethyl)-3-carbamoyl-4-methyl-6-hydroxy-2-pyridon, 1-(β-Acetylaminoethyl)-4-methyl-6-hydroxy-2-pyridon-3-sulfonsäure, 1-(β-Acetylaminopropyl)-4-methyl-6-hydroxy-2-pyridon, 1-(β-Acetylaminopropyl)-3-cyano-4-methyl-6-hydroxy-2-pyridon, 1-(β-Acetylamino-propyl)-3-carbamoyl-4-methyl-6-hydroxy-2-pyridon, 1-(β-Acetylamino-propyl)-4-methyl-6-hydroxy-2-pyridon-3-sulfonsäure, 4-Hydroxy-chinolin-(2), 1-Amino-8-hydroxy-2-(phenylazo)-naphthalin-3,6-disulfonsäure, 1-Amino-8-hydroxy-2-(4'-sulfophenylazo)-naphthalin-3,6-disulfonsäure, 1-Amino-8-hydroxy-2-(2',5'-disulfophenylazo)-naphthalin-3,6-disulfonsäure, 1-(β-Aminoethyl)-3-cyano-4-methyl-6-hydroxy-2-pyridon, 1-(γ-Aminopropyl)-3-sulfomethyl-4-methyl-6-hydroxy-2-pyridon, 1,3-Diaminobenzol, 1-Amino-3-(N,N-di-β-hydroxyethylamino)-benzol, 1-Amino-3-(N,N-di-β-sulfatoethylamino)-benzol, 1-Amino-(3-N,N-di-β-hydroxyethylamino)-4-methoxybenzol, 1-Amino-3-(N,N-di-β-sulfatoethylamino)-4-methoxy-benzol, 1-Amino-3-(sulfobenzylamino)-benzol, 1-Amino-3-(sulfobenzylamino)-4-chlorobenzol, 1-Amino-3-(N,N-di-sulfobenzylamino)-benzol, Phenol, 1-Hydroxy-3- oder -4-methyl-benzol, 1-Hydroxybenzol-4-sulfosäure, 1-Hydroxynaphthalin, 2-Hydroxynaphthalin, 2-Hydroxynaphthalin-6- oder -7-sulfonsäure, 1-Hydroxynaphthalin-4,7-disulfonsäure, 1-Amino-3-methyl-benzol, 1-Amino-2-methoxy-5-methyl-benzol, 1-Amino-2,5-dimethyl-benzol, 3-Aminophenylharnstoff, 1-Amino-3-acetylamino-benzol, 1-Amino-3-(hydroxyacetylamino)-benzol, 1,3-Diaminobenzol-4-sulfonsäure, 1-Amino-naphthalin-6- oder -8-sulfonsäure, 1-Amino-2-methoxy-naphthalin-6-sulfonsäure, 2-Aminonaphthalin-5,7-disulfonsäure, 1-Amino-8-hydroxy-naphthalin-6-sulfonsäure, 2-Hydroxy-3-aminonaphthalin-5,7-disulfonsäure, 1-Amino-8-hydroxy-naphthalin-2,4,6-trisulfonsäure, 1-Hydroxy-8-acetylamino-naphthalin-3-sulfonsäure, 1-Benzoylamino-8-hydroxy-naphthalin-3,6- oder -4,6-disulfonsäure, 2-Benzoylamino-5-hydroxy-naphthalin-7-sulfonsäure, 2-Methyl- und 2-Ethylamino-5-hydroxy-naphthalin-7-sulfonsäure, 2-(N-Acetyl-N-methylamino)-5-hydroxy-naphthalin-7-sulfonsäure, 2-Ethylamino-8-hydroxy-naphthalin-6-sulfonsäure, 2-Acetylamino-8-hydroxy-naphthalin-6-sulfonsäure, 1-(4'-Aminobenzoylamino)-8-hydroxynaphthalin-3,6- und -4,6-disulfonsäure, 1-(4'-Nitrobenzoylamino)-8-hydroxy-naphthalin-3,6- und -4,6-disulfonsäure, 1-(3'-Amino-benzoylamino)-6-hydroxy-naphthalin-3,6- und -4,6-disulfonsäure, 1-(3'-Nitrobenzoylamino)-8-hydroxy-naphthalin-3,6- und -4,6-disulfonsäure, 2-(4'-Amino-3'-sulfophenyl)-amino-5-hydroxy-naphthalin-7-sulfonsäure, 3-Methyl-5-pyrazolon, 1-Phenyl-3-methyl-5-pyrazolon, 1-(3'-Aminophenyl)-3-methyl-5-pyrazolon, 1-(2',5'-Disulfophenyl)-3-methyl-5-pyrazolon, 1-(2'-Methyl-4'-sulfophenyl)-5-pyrazolon-3-carbonsäure, 1-(4',8'-Disulfonaphthyl-2'-yl)-3-methyl-5-pyrazolon, 1-(5',7'-Disulfonaphthyl-2-)-3-methyl-5-pyrazolon, 1-(2',5'-Dichlor-4'-sulfophenyl)-3-methyl-5-pyrazolon, 3-Aminocarbonyl-4-methyl-6-hydroxy-2-pyridon, 1-Ethyl-3-cyano- oder -3-chlor-4-methyl-6-hydroxy-2-pyridon, 1-Ethyl-3-sulfomethyl-4-methyl-6-hydroxy-2-pyridon, 2,4,6-Triamino-3-cyano-pyridin, 2-(3'-Sulfophenyl)-amino-4,6-diamino-3-cyano-pyridin, 2-(2'-Hydroxyethylamino)-3-cyano-4-methyl-6-amino-pyridin, 2,6-Bis-(2'-hydroxyethylamino)-3-cyano-4-methyl-pyridin, 1-Ethyl-3-carbamoyl-4-methyl-6-hydroxy-2-pyridon, 1-Ethyl-3-sulfomethyl-4-methyl-5-carbamoyl-6-hydroxy-2-pyridon,N-Acetoacetylamino-benzol.

Die erfindungsgemäß zur Synthese der erfindungsgemäßen Azoverbindungen (1) einsetzbaren Verbindungen entsprechend der allgemeinen Formel (5) sind bisher noch nicht bekannt. Die Erfindung betrifft somit auch diese Verbindungen, Verfahren zu deren Herstellung und ihre Verwendung zur Synthese von Farbstoffen, wie insbesondere zu den erfindungsgemäßen Azoverbindungen (1). Sie lassen sich analog bekannten Verfahrensweisen der Umsetzung von Säurechloriden mit Aminen herstellen, indem man zunächst eine Verbindung der allgemeinen Formel (6)

Cl — X — Arylen — $NO_2$ (6)

in welcher Arylen und X die obengenannten Bedeutungen haben, mit einer Aminoverbindung der allgemeinen Formel

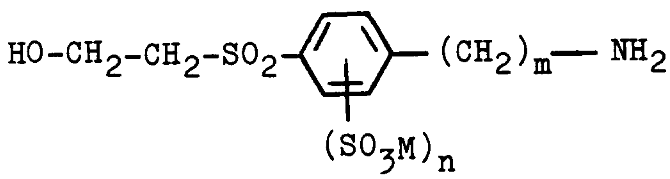

(7)

in welcher M, m und n die obengenannten Bedeutungen haben, umsetzt. Die Umsetzung erfolgt in hierfür üblichen und geeigneten Löse- oder Verdünnungsmitteln und in Gegenwart eines säurebindenden Mittels, in der Regel bei einer Temperatur zwischen 0 und 80 °C, bevorzugt zwischen 5 und 50 °C. Geeignete Lösemittel sind beispielsweise Wasser oder ein organisches Löse- oder Verdünnungsmittel oder ein Gemisch von Wasser und einem Wasser mischbaren organischen Lösemittel. Organische Löse- oder Verdünnungsmittel sind beispielsweise Wasser, Alkanole von 1 bis 6 C-Atomen, bevorzugt 1 bis 4 C-Atomen, wie beispielsweise Methanol, Dioxan, Toluol, die Xylole, Chlorbenzol, o-Dichlorbenzol, m-Dichlorbenzol, Dimethylformamid und N-Methyl-pyrrolidon. Säurebindende Mittel sind beispielsweise Kaliumcarbonat, Magnesiumoxid, Natriumcarbonat, Natriumhydroxid, Triethylamin und Triethanolamin. In wäßrigem Medium wird ein pH-Wert zwischen 6 und 12, bevorzugt zwischen 8 und 10, eingehalten.

Solche Verfahrensweisen sind beispielsweise aus den Verfahren zur Herstellung substituierter Phenyl-(β-hydroxyethyl)-sulfone bekannt (s. bspw. deutsche Offenlegungsschrift Nr. 3 502 991).

Säurechloride der allgemeinen Formel (6) sind beispielsweise 4-Nitro-benzoesäurechlorid, 4-Nitro-benzolsulfonsäurechlorid, 2- oder 3-Nitro-benzolsulfonsäurechlorid, 2- oder 3-Nitro-benzoesäurechlorid, 4-Methyl-3-nitro-benzoesäurechlorid, 3-Methyl-4-nitrobenzoesäurechlorid, 4-Methyl-2-nitrobenzoesäurechlorid,2-Methyl-4-nitro-benzoesäurechlorid, 2-Methyl-5-nitro-benzoesäurechlorid, 4-Chlor-2-nitrobenzoesäurechlorid, 5-Chlor-2-nitro-benzoesäurechlorid, 6-Chlor-2-nitro-benzoesäurechlorid, 2-Chlor-3-nitrobenzoesäurechlorid, 4-Chlor-3-nitro-benzoesäurechlorid, 6-Chlor-3-nitrobenzoesäurechlorid, 1-Sulfo-2-aminonaphthalin-5-sulfonsäurechlorid, 2-Chlor-4-nitro-benzoesäurechlorid, 4-Brom-2-nitro-benzoesäurechlorid, 2-Brom-4-nitro-benzoesäurechlorid, 4-Brom-3-nitrobenzoesäurechlorid, 6-Brom-3-nitro-benzoesäurechlorid,
4-Methyl-3-nitro-benzolsulfonsäurechlorid,
1-Sulfo-2-aminonaphthalin-5-sulfonsäurechlorid,
3-Methyl-4-nitro-benzolsulfonsäurechlorid,
4-Methyl-2-nitro-benzolsulfonsäurechlorid,
2-Methyl-4-nitro-benzolsulfonsäurechlorid,
2-Methyl-5-nitro-benzolsulfonsäurechlorid,
4-Chlor-2-nitro-benzolsulfonsäurechlorid,
5-Chlor-2-nitro-benzolsulfonsäurechlorid,
6-Chlor-2-nitro-benzolsulfonsäurechlorid,
2-Chlor-3-nitro-benzolsulfonsäurechlorid,
4-Chlor-3-nitro-benzolsulfonsäurechlorid,

6-Chlor-3-nitro-benzolsulfonsäurechlorid,
2-Chlor-4-nitro-benzolsulfonsäurechlorid,
4-Brom-2-nitro-benzolsulfonsäurechlorid,
2-Brom-4-nitro-benzolsulfonsäurechlorid,
4-Brom-3-nitro-benzolsulfonsäurechlorid,
6-Brom-3-nitro-benzolsulfonsäurechlorid.

Aminoverbindungen der allgemeinen Formel (7) sind bspw. 4-(β-Hydroxyethylsulfonyl)-1-(β-aminoethyl)-benzol, 5-(β-Hydroxyethylsulfonyl)-2-(β-aminoethyl)-benzolsulfonsäure, 4-(β-Hydroxyethylsulfonyl)-2-sulfobenzylamin und 4-(β-Hydroxyethylsulfonyl)-benzylamin.

Die auf diese Weise erfindungsgemäß erhältlichen und ebenfalls neuen, erfindungsgemäßen Nitroanilin-Verbindungen entsprechend der allgemeinen Formel (8)

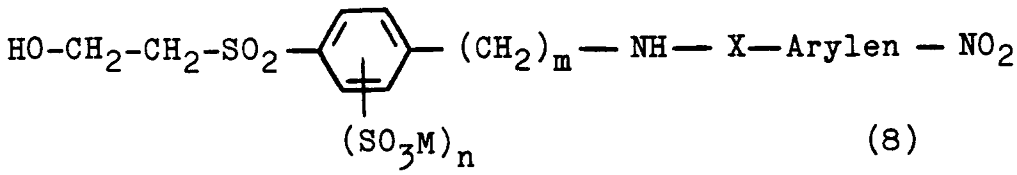

(8)

in welcher Arylen, X, M, m und n die obengenannten Bedeutungen haben, werden sodann analog bekannten Verfahrensweisen, nachdem sie beispielsweise aus dem Reaktionsansatz durch Kristallisation oder durch Abdestillieren des Lösemittels oder durch Ansäuern und Filtration isoliert wurden, zur Aminoverbindung entsprechend der allgemeinen Formel (5) reduziert, so durch katalytische Hydrierung mit Wasserstoff an Palladium, Platin oder Raney-Nickel bei einer Temperatur zwischen 50 und 110 °C und bei erhöhtem Druck oder durch Reduktion nach Béchamp mit Eisen in saurem Medium, beispielsweise mit Eisen in Ethanol/Eisessig. Die Reduktion kann in einem hierfür geeigneten Lösemittel, wie Wasser, Methanol oder Ethanol oder einer Mischung derselben, erfolgen.

Sowohl die erfindungsgemäßen Verbindungen der allgemeinen Formel (1) als auch die der allgemeinen Formel (5), in welchem Y bzw. Y' die β-Hydroxyethyl-Gruppe bedeuten, können in üblicher und bekannter Verfahrensweise in Verbindungen übergeführt werden, in welchen Y bzw. Y' eine andere Bedeutung als die β-Hydroxyethyl-Gruppe besitzt, so beispielsweise in deren Esterderivate, wie beispielsweise von mehrwertigen anorganischen Säuren oder von aliphatischen und aromatischen Carbon- oder Sulfonsäuren, so beispielsweise in Verbindungen, in welchen Z für die Sulfato-, Phosphato-, Thiosulfato-, Acetyloxy- oder Toluylsulfonyloxy-Gruppe oder für ein Chloratom steht. Hierfür geeignete Veresterungs- und Acylierungsmittel sind beispielsweise die entsprechenden anorganischen oder organischen Säuren oder deren Anhydride oder Halogenide oder Amide, wie beispielsweise Schwefelsäure, Schwefeltrioxid enthaltende Schwefelsäure, Chlorsulfonsäure, Amidosulfonsäure, Phosphorsäure, Phosphoroxychlorid, Gemische aus Phosphorsäure und Phosphorpentoxid, Acetanhydrid, Toluolsulfochlorid und Thionylchlorid.

Diejenigen Verbindungen, in welchen Y bzw. Y' für die Vinylgruppe steht, können aus deren analogen Esterderivaten mittels Alkali, so in wäßrigem Medium bei einem pH-Wert von 10 bis 12 und einer Temperatur zwischen 40 und 50 °C während 10 bis 20 Minuten, hergestellt werden. Die Synthese von beispielsweise β-(Dialkylamino)-ethylsulfonyl- und β-Thiosulfatoethylsulfonyl-Derivaten der Verbindungen (1) und (5) erfolgt durch Umsetzung von deren Vinylsulfonyl-Verbindungen mit dem entsprechenden Dialkylamin oder mit einem Alkalisalz der Thioschwefelsäure, wie Natriumthiosulfat.
Alle diese Verfahrensweisen der Überführung von einer Gruppe $-SO_2-Y$ bzw. $-SO_2-Y'$ in eine andere sind dem Fachmann auf diesem faserreaktiven Gebiet geläufig und zahlreich in der Literatur beschrieben.

Die erfindungsgemäßen Verbindungen der allgemeinen Formel (1) - im nachfolgenden als Verbindungen (1) bezeichnet - haben faserreaktive Eigenschaften und besitzen sehr wertvolle Farbstoffeigenschaften. Sie können deshalb zum Färben (einschließlich Bedrucken) von hydroxygruppenhaltigen und/oder carbonamidgruppenhaltigen Materialien verwendet werden. Hierzu können die bei der Synthese der Verbindungen (1) anfallenden Lösungen, gegebenenfalls nach Zusatz einer Puffersubstanz und gegebenenfalls auch nach Konzentrierung, direkt als Flüssigpräparation der färberischen Verwendung zugeführt werden.

Die Abscheidung und Isolierung der Verbindungen (1) aus den wäßrigen Syntheselösungen kann nach allgemein bekannten Methoden für wasserlösliche Verbindungen erfolgen, so beispielsweise durch Ausfällen aus dem Reaktionsmedium mittels eines Elektrolyten, wie beispielsweise Natriumchlorid oder Kaliumchlorid, oder aber durch Eindampfen der Reaktionslösung selbst, beispielsweise durch Sprühtrocknung. Falls die letztgenannte Art der Isolierung gewählt wird, empfiehlt es sich vielfach, vor dem Eindampfen

eventuell in den Lösungen vorhandene Sulfatmengen durch Fällung als Calciumsulfat und Abtrennung durch Filtration zu entfernen.

Gegenstand der vorliegenden Erfindung ist deshalb auch die Verwendung der Verbindungen (1) zum Färben (einschließlich Bedrucken) von hydroxy- und/oder carbonamidgruppenhaltigen Materialien bzw. Verfahren zu deren Anwendung auf diesen Substraten. Bevorzugt kommen die Materialien in Form von Fasermateialien zur Anwendung, insbesondere in Form von Textilfasern, wie Garnen, Wickelkörpern und Geweben. Hierbei kann man analog bekannten Verfahrensweisen vorgehen.

Hydroxygruppenhaltige Materialien sind solche natürlichen oder synthetischen Ursprungs, wie beispielsweise Cellulosefasermaterialien oder deren Regeneratprodukte und Polyvinylalkohole. Cellulosefasermaterialien sind vorzugsweise Baumwolle, aber auch andere Pflanzenfasern, wie Leinen, Hanf, Jute und Ramiefasern; regenerierte Cellulosefasern sind beispielsweise Zellwolle und Viskosekunstseide.

Carbonamidgruppenhaltige Materialien sind beispielsweise synthetische und natürliche Polyamide und Polyurethane, insbesondere in Form von Fasern, beispielsweise Wolle und andere Tierhaare, Seide, Leder, Polyamid-6,6, Polyamid-6, Polyamid-11 und Polyamid-4.

Die Verbindungen (1) lassen sich, gemäß der erfindungsgemäßen Anwendung, auf den genannten Substraten, insbesondere auf den genannten Fasermaterialien, nach den für wasserlösliche, faserreaktive Farbstoffe bekannten Anwendungstechniken applizieren und fixieren, so beispielsweise, indem man die Verbindung (1) in gelöster Form auf das Substrat aufbringt oder sie darin einbringt und sie auf diesem oder in diesem, gegebenenfalls durch Hitzeeinwirkung und/oder gegebenenfalls durch Einwirkung eines alkalisch wirkenden Mittels, fixiert. Solche Färbe- und Fixierweisen sind in der Literatur zahlreich beschrieben (s. bspw. europäische Patentanmeldungs-Veröffentlichung Nr. 0 181 585 A2). Die Verbindungen (1) zeichnen sich durch hohe Auszieh- und Fixiergrade aus.

Die erfindungsgemäßen Färbungen besitzen, insbesondere auf Cellulosefasermaterialien, gute Lichtechtheiten sowohl im trockenen Zustand der Färbung als auch im nassen, beispielsweise mit einer Schweißlösung befeuchteten, Zustand sowie gute Naßechtheiten, wie beispielsweise gute Waschechtheiten bei 60 bis 95 °C, auch in Gegenwart von Perboraten, saure und alkalische Walk-, Überfärbe- und Schweißechtheiten, gute saure und alkalische Schweißechtheiten, eine hohe Dampfbeständigkeit, gute Alkali-, Säure-, Wasser- und Seewasserechtheiten, desweiteren eine gute Plissierechtheit, Bügelechtheit und Reibechtheit. Ebenso besitzen sie eine gute Säurelagerbeständigkeit ("acid fading") beim Lagern von feuchten, noch Essigsäure enthaltendem, gefärbtem Material.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in diesen Beispielen formelmäßig beschriebenen Verbindungen sind in Form der freien Säuren angegeben; im allgemeinen werden sie in Form ihrer Alkalimetallsalze, wie Lithium-, Natrium- oder Kaliumsalze, hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, in die Synthese eingesetzt werden.

Die für die erfindungsgemäßen Verbindungen angegebenen Absorptionsmaxima ($\lambda_{max}$) im sichtbaren Bereich wurden anhand derer Alkalimetallsalze in wäßriger Lösung ermittelt. In den Tabellenbeispielen sind die $\lambda_{max}$-Werte bei der Farbtonangabe in Klammern gesetzt; die Wellenlängenangabe bezieht sich auf nm.

**Beispiel A**

Eine Lösung von 26,55 Teilen β-(4-β'-Hydroxyethylsulfonyl-phenyl)-ethylamin-Hydrochlorid in 100 Teilen Wasser wird bei einer Temperatur von etwa 5 °C mit 50 Teilen einer wäßrigen 2n-Natronlauge und anschließend unter kräftigem Rühren bei Einhaltung einer Temperatur von etwa 5 °C und eines pH-Wertes von 9,5 mit einer Lösung von 18,6 Teilen 4-Nitrobenzoylchlorid in 50 Teilen Aceton versetzt. Man rührt noch 2 Stunden bei 10 °C und anschließend 2 Stunden bei 30 °C nach, filtriert das ausgefallene Produkt ab, wäscht es mit Wasser und trocknet es unter reduziertem Druck bei 60 °C. Nach Umkristallisation aus Ethanol besitzt es einen Schmelzpunkt von 163-166 °C. Die Elementaranalyse lieferte folgende Werte (berechnet auf MG = 378):

| | | | | |
|---|---|---|---|---|
| ber.: | C 54,0% | H 4,7% | N 7,4% | S 8,5% ; |
| gef.: | C 53,8% | H 4,85% | N 7,5% | S 7,8% . |

Es hat die folgende chemische Formel:

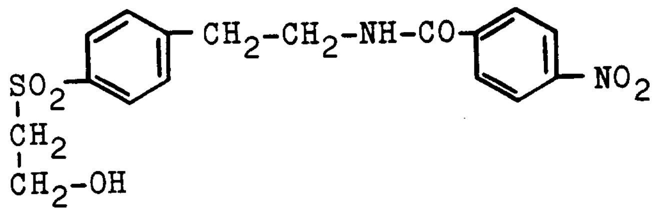

.

**Beispiel B**

37,8 Teile der 4-Nitro-benzamid-Verbindung von Beispiel A bringt man in eine 80 bis 90 ° C heiße Mischung aus 200 Teilen Wasser, 200 Teilen Ethanol, 1 Teil Eisessig und 20 Teilen Eisenspänen ein. Man rührt unter Siedetemperatur noch 1 Stunde nach, saugt das Reaktionsgemisch heiß ab und wäscht den Rückstand mit einer geringen Menge einer heißen Mischung aus gleichen Teilen Ethanol und Wasser nach. Aus dem Filtrat kristallisiert das 4-Amino-N'-[β-(4'-β'-hydroxyethylsulfonyl-phenyl)-ethyl]-benzamid aus. Man filtriert es bei einer Temperatur von 0 bis 5 ° C ab, wäscht es mit wenig Ethanol/Wasser-1:1 nach und kristallisiert es aus einem Gemisch aus gleichen Teilen Ethanol und Wasser um.
Schmelzpunkt: 215-217 ° C;

| Elementaranalyse (MG = 348): | | | | |
|---|---|---|---|---|
| ber.: | C 58,6% | H 5,75% | N 8,05% | S 9,2% ; |
| gef.: | C 58,7% | H 6,0% | N 8,4% | S 9,1% . |

Es hat die folgende chemische Formel

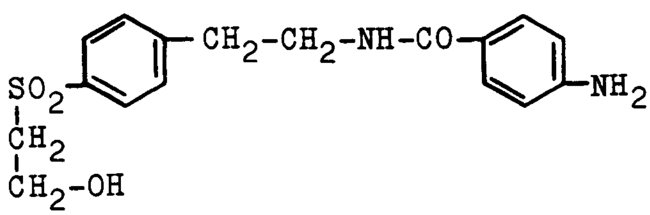

.

**Beispiel C**

Eine Lösung von 26,55 Teilen β-(4-β'-Hydroxyethylsulfonyl-phenyl)-ethylamin-Hydrochlorid in 100 Teilen Wasser wird bei 80 ° C mit 21,6 Teilen Natriumcarbonat versetzt. Anschließend gibt man bei 80 ° C innerhalb von 15 Minuten 18,8 Teile 3-Nitro-benzoylchlorid hinzu, rührt den Ansatz bei 80 ° C noch 15 Minuten nach, läßt ihn langsam abkühlen, filtriert vom ausgefallenen Produkt bei 20 ° C ab, wäscht dieses mit Wasser neutral und trocknet es bei 60 ° C unter reduziertem Druck.
Nach Umkristallisation aus einem Gemisch von Methanol/Wasser-1:2 zeigt das erhaltene 3-Nitro-N-[β-(4'-β'-hydroxyethylsulfonyl-phenyl)-ethyl]-benzamid einen Schmelzpunkt von 148-149 ° C.

| Elementaranlyse (MG = 378) | | | | |
|---|---|---|---|---|
| ber.: | C 54,0% | H 4,76% | N 7,4% | S 8,5% ; |
| gef.: | C 54,0% | H 4,80% | N 7,1% | S 8,7% . |

**Beispiel D**

Unter kräftigem Rühren trägt man 37,8 Teile des Nitrobenzamids von Beispiel C in eine 80 bis 90 °C heiße Mischung von 400 Teilen Wasser, 200 Teilen Ethanol, 1 Teil Eisessig und 20 Teilen Eisenspänen ein. Man führt die Umsetzung noch 1 Stunde unter Siedetemperatur weiter und stellt sodann das Reaktionsgemisch mittels Natriumcarbonat auf einen pH-Wert von 8, entfernt den Rückstand aus der heißen Lösung und wäscht ihn mit 50 Teilen eines heißen Gemisches aus gleichen Teilen von Ethanol und Wasser nach und dampft das Filtrat unter reduziertem Druck ein. Das erhaltene 3-Amino-N'-[β-(4'-β'-hydroxy ethylsulfonyl-phenyl)-ethyl]-benzamid besitzt, nach Umkristallisation aus einem Gemisch von Ethanol/Wasser-1:2, einen Schmelzpunkt von 142-143 °C.

| Elementaranalyse (MG = 348): | | | | |
|---|---|---|---|---|
| ber.: | C 58,6% | H 5,75% | N 8,05% | S 9,2% ; |
| gef.: | C 58,6% | H 5,8 % | N 7,9 % | S 9,0% . |

**Beispiel E**

Eine Lösung von 26,5 Teilen β-(4'-β'-Hydroxyethylsulfonyl-phenyl)-ethylamin-Hydrochlorid in 250 Teilen Wasser versetzt man mit 28 Teilen Natriumhydrogencarbonat und anschließend mit 23,35 Teilen 4-Acetylamino-benzolsulfonsäurechlorid. Man rührt weiter bei Raumtemperatur noch 15 Minuten nach, saugt sodann vom Rückstand ab und wäscht ihn mit wenig Wasser. Zur Hydrolyse der Acetylaminogruppe wird der Rückstand 30 Minuten in 430 Teilen einer 10 %igen wäßrigen Salzsäure unter Rückflußtemperatur behandelt. Nach Abkühlen des Ansatzes auf 20 °C wird ein pH-Wert von 7 eingestellt, das Produkt abgesaugt, mit Wasser gewaschen und unter reduziertem Druck getrocknet.
Nach Umkristallisation aus Wasser zeigt das erhaltene 4-Amino-N'-[β-(4'-β'-hydroxyethylsulfonyl-phenyl)-ethyl]-benzolsulfonamid der Formel

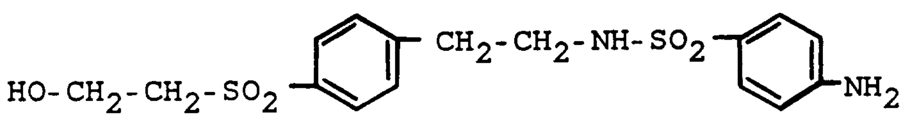

einen Schmelzpunkt von 117-118 °C.

| Elementaranalyse (MG = 384): | | | | |
|---|---|---|---|---|
| ber.: | C 50,0% | H 5,2% | N 7,3% | S 16,7% ; |
| gef.: | C 50,1% | H 5,1% | N 7,4% | S 16,7% . |

**Beispiel 1**

4-Amino-N'-[β-(4'-β'-sulfatoethylsulfonyl-phenyl)-ethyl]-benzolsulfonamid, das in üblicher Weise durch Veresterung der β-Hydroxyethylsulfonyl-Verbindung (Beispiel E) in 100%iger Schwefelsäure bei 5 bis 10 °C und anschließender Ausfällung in Eiswasser erhältlich ist (vgl. auch Beispiel 6), wird diazotiert, indem man eine neutrale Lösung von 111,4 Teilen dieser Verbindung und 16,5 Teilen Natriumnitrit in 700 Teilen Wasser bei einer Temperatur von 5 bis 10 °C in eine Mischung von 43 Teilen einer 30 %igen wäßrigen Salzsäure und 700 Teilen Eis einrührt. Nach einigem Nachrühren wird überschüssiges Nitrit mit Amidosulfonsäure entfernt.
Man gibt 31,9 Teile 1-Amino-8-naphthol-3,6-disulfonsäure hinzu und führt die erste Kupplungsreaktions bei einem pH-Wert von 1,5 und einer Temperatur von 10 °C und die zweite Kupplungsreaktion anschließend bei einem pH-Wert von 5 und einer Temperatur von 10 °C durch.

Nach beendeter Kupplung wird die erfindungsgemäße Disazoverbindung mit Kaliumchlorid ausgefällt, durch Filtration isoliert und getrocknet. Man erhält sie als Alkalimetallsalz, vorwiegend Kaliumsalz, in Form

eines elektrolytsalzhaltigen (vorwiegend kaliumchloridhaltigen) tiefblauen Pulvers. Sie besitzt, in Form der freien Säure geschrieben, die Formel

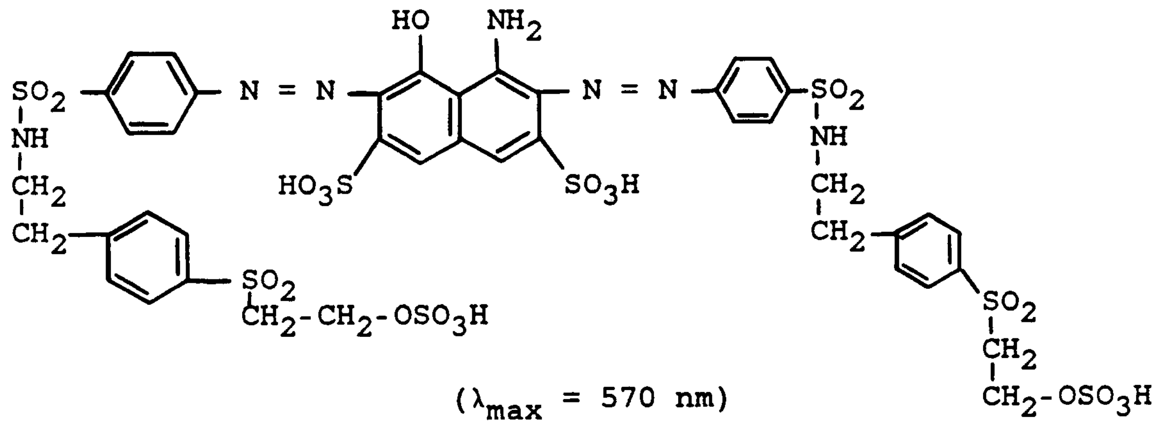

($\lambda_{max}$ = 570 nm)

und zeigt sehr gute faserreaktive Farbstoffeigenschaften. Sie färbt die in der Beschreibung genannten Fasermaterialien wie insbesondere Cellulosefasermaterialien, beispielsweise Baumwolle, nach den in der Technik für faserreaktive Farbstoffe üblichen Anwendungsverfahren in farbstarken blauen, echten Tönen mit hohem Fixiergrad. Von den guten Echtheitseigenschaften der mit der erfindungsgemäßen Disazoverbindung erhältlichen Färbungen und Drucke können insbesondere die Waschechtheit bei 60 °C sowie die Lichtechtheit und Schweißlichtechtheiten hervorgehoben werden. Die erfindungsgemäße Verbindung zeichnet sich weiterhin durch eine hohe Farbstärke aus.

**Beispiel 2**

55,7 Teile des Sulfatoderivates der Verbindung von Beispiel E (s. Beispiel 1) werden analog den Angaben des Beispieles 1 diazotiert und mit 23 Teilen 1-Naphthol-5-sulfonsäure bei einem pH-Wert von 5 und einer Temperatur von 10 bis 15 °C gekuppelt. Anschließend erwärmt man die Syntheselösung auf 60 °C, klärt sie durch Filtration und isoliert die erfindungsgmäße Monoazoverbindung in Form ihres Natriumsalzes durch Aussalzen mit Natriumchlorid; sie besitzt, in Form der freien Säure geschrieben, die Formel

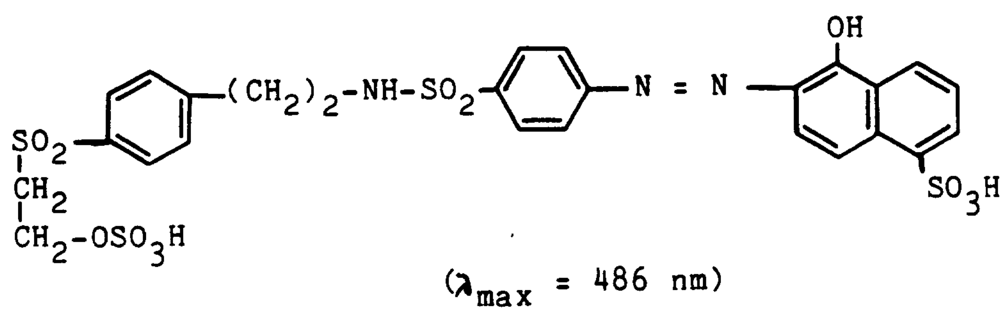

($\lambda_{max}$ = 486 nm)

zeigt sehr gute faserreaktive Farbstoffeigenschaften und färbt die in der Beschreibung genannten Fasermaterialien, wie insbesondere Baumwolle, nach den üblichen Anwendungsverfahren für faserreaktive Farbstoffe in farbstarken roten, echten Tönen mit hohem Fixiergrad. Von den Echtheitseigenschaften können insbesondere die Waschechtheit bei 60 °C sowie die Lichtechtheit und Schweißlichtechtheiten hervorgehoben werden. Die erfindungsgemäße Verbindung zeichnet sich weiterhin durch eine hohe Farbstärke aus.

**Beispiel 3**

34,8 Teile 3-Amino-N'-[(4'-$\beta$'-hydroxyethylsulfonyl-phenyl)-ethyl]-benzamid (s. Beispiel D) werden bei 40 bis 45 °C in 184 Teilen 100 %iger Schwefelsäure gelöst. Man rührt die Lösung noch 1 Stunde bei 50 °C nach, kühlt sie dann auf 5 °C ab und gibt langsam bei etwa 0 °C 43,25 Teile einer 37 %igen Nitrosylschwefelsäure hinzu. Der Ansatz wird noch 15 Minuten bei 5 ° C und danach 30 Minuten bei 20 ° C gerührt und anschließend unter Rühren auf 500 Teile Eis gegeben, wobei die Temperatur nicht 5 ° C übersteigen darf. Nach einigem Nachrühren wird überschüssiges Nitrit mit Amidosulfonsäure zerstört.

Zu dieser Lösung gibt man bei 5 ° C eine neutrale Lösung von 22,4 Teilen 1-Naphthol-4-sulfonsäure in 1500 Teilen Wasser, stellt mittels Calciumcarbonat einen pH-Wert von 6 ein und führt die Kupplungsreaktion bei diesem pH-Wert und einer Temperatur zwischen 10 und 20 ° C durch. Man erwärmt anschließend den Ansatz auf 40 ° C, filtriert vom Calciumsulfat ab und fällt die erfindungsgemäße Azoverbindung aus dem Filtrat mittels 25 % Kaliumchlorid und 5 % Natriumchlorid, bezogen auf das Volumen des Filtrates, aus. Nach Filtration wäscht man den Filterrückstand mit gesättigter wäßriger Natriumchloridlösung und trocknet ihn bei 60 ° C unter reduziertem Druck.

Man erhält ein rotes, salzhaltiges Pulvers des erfindungsgemäßen Alkalimetallsalzes (vorwiegend Kaliumsalzes) der Verbindung der Formel

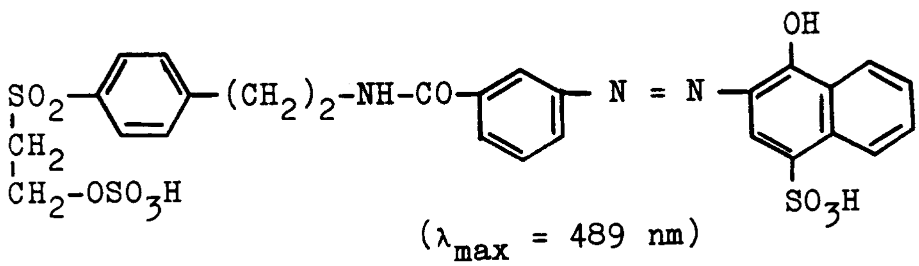

($\lambda_{max}$ = 489 nm)

die sehr gute faserreaktive Farbstoffeigenschaften zeigt und insbesondere Cellulosefasermaterialien nach den üblichen Applikations- und Fixierverfahren in farbstarken, roten Tönen mit guten Echtheitseigenschaften, von denen insbesondere die gute Waschechtheit bei 60 ° C und die guten Licht- und Schweißlichtechtheiten hervorgehoben werden können, färbt.

**Beispiel 4**

Man stellt analog Beispiel 3 die schwefelsaure Lösung des Diazoniumsalzes der 4-Amino-benzamid-Verbindung des Beispieles B her und gibt zu ihr langsam bei 5 ° C eine neutrale Lösung von 30,4 Teilen 1-Naphthol-3,6-disulfonsäure in 500 Teilen Wasser. Man stellt den pH zunächst mit etwa 16 Teilen Natriumcarbonat und anschließend mit Kalziumcarbonat auf einen pH-Wert von 4 bis 4,5, führt innerhalb dieses pH-Bereiches und einer Temperatur von 10 bis 20 ° C die Kupplungsreaktion durch, erwärmt anschließend die Syntheselösung auf 50 ° C, filtriert vom Calciumsulfat ab und isoliert die erfindungsgemäße Azoverbindung durch Aussalzen mit Kaliumchlorid.

Man erhält das Alkalimetallsalz (vorwiegend Kaliumsalz) der Verbindung der Formel

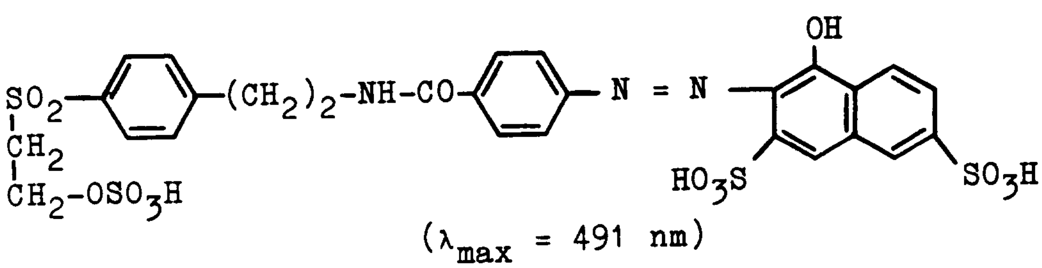

($\lambda_{max}$ = 491 nm)

die sehr gute faserreaktive Farbstoffeigenschaften besitzt und insbesondere Cellulosefasermaterialien nach den üblichen Anwendungstechniken in farbstarken rotstichig orangen Tönen mit guten Echtheitseigenschaften, von denen insbesondere die guten Waschechtheiten bei 60 ° C und 95 ° C sowie die guten Lichtechtheiten sowohl im trockenen Zustand der Färbung als auch im mit Trinkwasser oder mit einer Schweißlösung befeuchteten Zustand hervorgehoben werden können, färbt.

**Beispiel 5**

Man stellt eine schwefelsaure Lösung des Diazoniumsalzes von 38,3 Teilen der im Beispiel B beschriebenen 4-Amino-benzamid-Verbindung gemäß den Angaben des Beispieles 3 her, rührt diese in 600 Teile Eis und und versetzt sie mit 31,9 Teilen 1-Amino-8-naphthol-3,6-disulfonsäure. Mittels etwa 16 Teilen Natriumcarbonat und anschließend mit Calciumcarbonat wird ein pH-Wert von 1 bis 1,5 eingestellt. Man führt die erste Kupplungsreaktion bei einem pH-Wert von 1,5 und einer Temperatur von 10 °C durch.
Für die zweite Kupplungsreaktion gibt man danach die wäßrige, salzsaure Diazoniumsalzlösung aus 28,1 Teilen 3-(β-Sulfatoethylsulfonyl)-anilin in etwa 430 Teilen Wasser hinzu. Mit Calciumcarbonat stellt man einen pH-Wert zwischen 6 und 7 ein und führt innerhalb dieses pH-Bereiches und bei 20 °C die zweite Kupplungsreaktion durch.
Man isoliert die erfindungsgemäße Disazoverbindung in Form ihres Alkalimetallsalzes in üblicher Weise. Sie besitzt, in Form der freien Säure geschrieben, die Formel

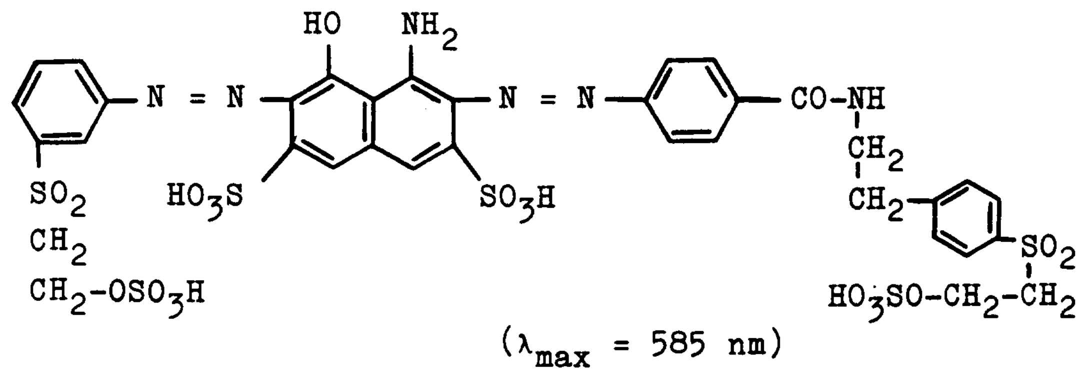


zeigt sehr gute faserreaktive Farbstoffeigenschaften und färbt insbesondere Cellulosefasermaterialien nach den üblichen Anwendungstechniken in farbstarken, blauen Tönen mit guten Echtheitseigenschaften, von denen insbesondere die gute Waschechtheit bei 60 °C und die guten Lichtechtheiten und Schweißlichtechtheiten hervorgehoben werden können.

**Beispiel 6**

51,4 Teile 1-Sulfo-2-aminonaphthalin-5-{N-[-(4'-β'-hydroxyethylsulfonyl-phenyl)-ethyl]}-sulfonamid werden in 100 Teilen konzentrierter Schwefelsäure bei 20 bis 25 °C gelöst und unterhalb 10 °C in eine Mischung aus 300 Teilen Eis und 30 Teilen Natriumchlorid gegeben. Die ausgefallene Sulfatoverbindung wird abgesaugt, mit wenig 20 %iger wäßriger Natriumchloridlösung gewaschen und trockengesaugt, in 1000 Teilen Wasser suspendiert und mit Natriumbicarbonat neutral gelöst. Die Aminonaphthalin-Verbindung wird sodann in üblicher Weise diazotiert und mit 28,4 Teilen 1-(4'-Sulfophenyl)-3-carboxy-5-pyrazolon bei einem pH-Wert zwischen 5 und 6 und einer Temperatur zwischen 10 und 20 °C gekuppelt. Die erfindungsgemäße Azoverbindung der Formel

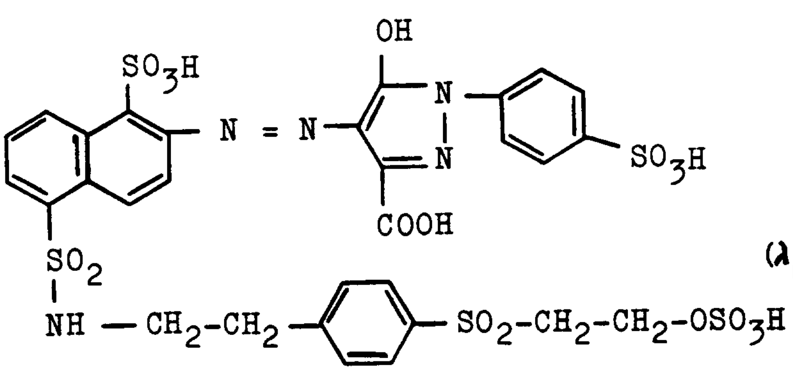

($\lambda_{max}$ = 402 nm)

wird in üblicher Weise als Alkalimetallsalz isoliert, beispielsweise durch Aussalzen mit Kaliumchlorid. Sie besitzt sehr gute faserreaktive Farbstoffeigenschaften und färbt beispielsweise Cellulosefasermaterialien in farbstarken gelben Tönen mit guten Echtheitseigenschaften, von denen insbesondere die gute Waschechtheit bei 60 °C und die guten Licht- und Schweißechtheiten hervorgehoben werden können.

**Beispiele 7 bis 103**

In den nachfolgenden Tabellenbeispielen sind weitere erfindungsgemäße Azoverbindungen entsprechend der in Form der freien Säure geschriebenen allgemeinen Formel (A)

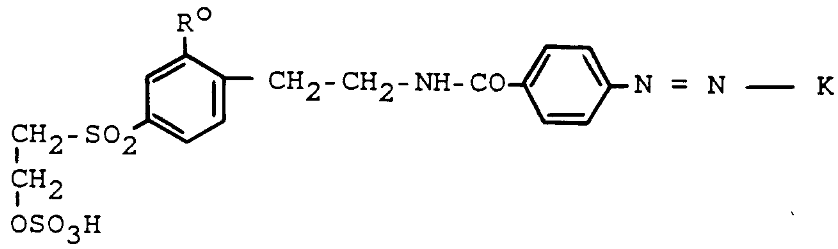

(A)

mit Hilfe ihrer Kupplungskomponente H-K beschrieben. Sie lassen sich in erfindungsgemäßer Weise, beispielsweise gemäß den obigen Ausführungsbeispielen durch Kupplung der angegebenen Kupplungskomponente mit dem Diazoniumsalz der aus Formel (A) ersichtlichen aromatischen Aminoverbindung entsprechend der allgemeinen Formel (5) herstellen und besitzen ebenfalls sehr gute faserreaktive Farbstoffeigenschaften. Sie liefern farbstarke Färbungen und Drucke auf den in der Beschreibung genannten Materialien, wie insbesondere Cellulosefasermaterialien, nach den für faserreaktive Farbstoffe üblichen Applikations- und Fixiermethoden mit den in dem jeweiligen Tabellenbeispiel angegebenen Farbton (hier auf Baumwolle) mit guten Echtheitseigenschaften; die in Klammern angegebenen Werte sind die Absorptionsmaxima ($\lambda_{max}$) in nm.

| Bsp. | Rest $R^o$ | Komponente H-K | Farbton |
|---|---|---|---|
| 7 | Sulfo | N,N-Bis-(ß-sulfatoethyl)-3-chlor-anilin | gelb |
| 8 | Sulfo | 1-(N-ß-Sulfatoethyl)-4-methyl-2-hydroxy-6-pyridon | gelb |
| 9 | Sulfo | 1-Acetylamino-3,6-disulfo-8-naphthol | rot |

| Bsp. | Rest $R^O$ | Komponente H-K | Farbton |
|---|---|---|---|
| 10 | H | 2-Acetylamino-6-sulfo-8-naphthol | rotorange (493) |
| 11 | H | 4-Sulfo-1-naphthol | rotorange (488) |
| 12 | H | 1-Benzoylamino-3,6-disulfo-8-naphthol | rot (520) |
| 13 | H | 1-Benzoylamino-4,6-disulfo-8-naphthol | rot (515) |
| 14 | H | 3-Sulfo-1-naphthol | orange (477) |
| 15 | H | 1-(4'-Sulfophenyl)-3-carboxy-5-pyrazolon | gelb (408) |
| 16 | H | 1-(4'-Sulfophenyl)-3-methyl-5-pyrazolon | gelb (390) |
| 17 | H | 5-Sulfo-1-naphthol | rotorange (487) |
| 18 | H | N-Acetoacetyl-2-methoxy-5-methyl-4-(ß-sulfato-ethylsulfonyl)-anilin | gelb |
| 19 | H | N-Acetoacetyl-2-methoxy-5-(ß-sulfatoethylsulfonyl)-anilin | gelb |
| 20 | H | N-Acetoacetyl-2,5-dimethoxy-4-(ß-sulfatoethylsulfonyl)-anilin | gelb |
| 21 | H | N-Acetoacetyl-3-methoxy-4-(ß-sulfatoethylsulfonyl)-anilin | gelb |
| 22 | H | N-Acetoacetyl-4-methoxy-3-(ß-sulfatoethylsulfonyl)-anilin | gelb |
| 23 | H | 1-(4'-ß-Sulfatoethylsulfonyl-phenyl)-3-methyl-5-pyrazolon | gelb (390) |
| 24 | H | 1-(4'-ß-Sulfatoethylsulfonyl-phenyl)-3-carbethoxy-5-pyrazolon | gelb |
| 25 | H | 3-Acetylamino-6-sulfo-8-naphthol | orange (472) |
| 26 | H | N,N-Bis-(ß-sulfatoethyl)-anilin | gelb |
| 27 | H | N-Ethyl-N-(ß-sulfatoethyl)-anilin | gelb |
| 28 | H | N,N-Bis-(ß-sulfatoethyl)-3-chlor-anilin | gelb |
| 29 | H | 1-(N-ß-Sulfatoethyl)-4-methyl-2-hydroxy-6-pyridon | gelb |
| 30 | H | 1-Acetylamino-3,6-disulfo-8-naphthol | rot (511) |

| Bsp. | Rest $R^O$ | Komponente H-K | Farbton |
|---|---|---|---|
| 31 | H | 1-Acetylamino-4,6-disulfo-8-naphthol | rot |
| 32 | Sulfo | 1-[4'-Chlor-6'-(4"-ß-sulfato-ethylsulfonyl-phenyl)-amino-s-triazin-2'-yl]-amino-3,6-disulfo-8-naphthol | rot |
| 33 | Sulfo | 1-[4'-Chlor-6'-(4"-ß-sulfato-ethylsulfonyl-phenyl)-amino-s-triazin-2'-yl]-amino-4,6-disulfo-8-naphthol | rot |
| 34 | Sulfo | 2-[4'-Chlor-6'-(4"-ß-sulfato-ethylsulfonyl-phenyl)-amino-s-triazin-2'-yl]-amino-6-sulfo-8-naphthol | rot |
| 35 | H | 3-[4'-Chlor-6'-(4"-ß-sulfato-ethylsulfonyl-phenyl)-amino-s-triazin-2'-yl]-amino-6-sulfo-8-naphthol | rot |
| 36 | H | 1-(4'-Chlor-6'-methoxy-s-triazin-2'-yl)-amino-3,6-disulfo-8-naphthol | rot |
| 37 | Sulfo | 1-(4'-Chlor-6'-methoxy-s-triazin-2'-yl)-amino-4,6-disulfo-8-naphthol | rot |
| 38 | Sulfo | 2-Naphthol-6-carbonsäure | orange |
| 39 | Sulfo | 2-(4'-Chlor-6'-methoxy-s-triazin-2'-yl)-amino-6-sulfo-8-naphthol | rot |
| 40 | Sulfo | 3-(4'-Chlor-6'-methoxy-s-triazin-2'-yl)-amino-6-sulfo-8-naphthol | rot |
| 41 | Sulfo | 1-[4'-Fluor-6'-(4"-ß-sulfato-ethylsulfonyl-phenyl)-amino-s-triazin-2'-yl]-amino-3,6-disulfo-8-naphthol | rot |
| 42 | Sulfo | 1-[4'-Fluor-6'-(4"-ß-sulfato-ethylsulfonyl-phenyl)-amino-s-triazin-2'-yl]-amino-4,6-disulfo-8-naphthol | rot |
| 43 | H | 2-[4'-Fluor-6'-(4"-ß-sulfato-ethylsulfonyl-phenyl)-amino-s-triazin-2'-yl]-amino-6-sulfo-8-naphthol | rot |

| Bsp. | Rest $R^o$ | Komponente H-K | Farbton |
|---|---|---|---|
| 44 | H | 3-[4'-Fluor-6'-(4"-ß-sulfatoethylsulfonyl-phenyl)-amino-s-triazin-2'-yl]-amino-6-sulfo-8-naphthol | rot |
| 45 | H | 1-(4'-ß-Sulfatoethylsulfonyl-benzoyl)-amino-3,6-disulfo-8-naphthol | rot |
| 46 | H | 1-(4'-ß-Sulfatoethylsulfonyl-benzoyl)-amino-4,6-disulfo-8-naphthol | rot |
| 47 | H | 2-(4'-ß-Sulfatoethylsulfonyl-benzoyl)-amino-6-sulfo-8-naphthol | rot |
| 48 | H | 3-(4'-ß-Sulfatoethylsulfonyl-benzoyl)-amino-6-sulfo-8-naphthol | rot |
| 49 | Sulfo | 1-{ß-[4'-Chlor-6'-(4"-ß'-sufatoethylsulfonyl-phenyl)-ethyl]-amino-s-triazin-2'-yl}-amino-3,6-disulfo-8-naphthol | rot |
| 50 | Sulfo | 1-{ß-[4'-Chlor-6'-(4"-ß'-sufatoethylsulfonyl-phenyl)-ethyl]-amino-s-triazin-2'-yl}-amino-4,6-disulfo-8-naphthol | rot |
| 51 | Sulfo | 1-{ß-[4'-Chlor-6'-(3"-ß'-sufatoethylsulfonyl-phenyl)-ethyl]-amino-s-triazin-2'-yl}-amino-3,6-disulfo-8-naphthol | rot |
| 52 | Sulfo | 1-{ß-[4'-Chlor-6'-(3"-ß'-sufatoethylsulfonyl-phenyl)-ethyl]-amino-s-triazin-2'-yl}-amino-4,6-disulfo-8-naphthol | rot |
| 53 | H | 1-[4'-Chlor-6'-(4"-sulfophenyl)-amino-s-triazin-2'-yl]-amino-3,6-disulfo-8-naphthol | rot |
| 54 | H | 1-[4'-Chlor-6'-(4"-sulfophenyl)-amino-s-triazin-2'-yl]-amino-4,6-disulfo-8-naphthol | rot |
| 55 | Sulfo | 1-[4'-Chlor-6'-(2",5"-disulfo-amino-s-triazin-2'-yl]-amino-4,6-disulfo-8-naphthol | rot |
| 56 | Sulfo | 1-[4'-Fluor-6'-(3",5"-disulfo-amino-s-triazin-2'-yl]-amino-3,6-disulfo-8-naphthol | rot |

| Bsp. | Rest $R^O$ | Komponente H-K | Farbton |
|---|---|---|---|
| 57 | Sulfo | 1-[4'-Fluor-6'-(3",5"-disulfo-amino-s-triazin-2'-yl]-amino-4,6-disulfo-8-naphthol | rot |
| 58 | Sulfo | 6-Sulfo-2-naphthol | rot |
| 58 | Sulfo | 8-Sulfo-2-naphthol | rot |
| 59 | Sulfo | 1-Acetylamino-3,6-disulfo-8-naphthol | blaustichig rot |
| 60 | Sulfo | 1-Phenylureido-4,6-disulfo-8-naphthol | blaustichig rot |
| 61 | H | 3,6,8-Trisulfo-1-naphthol | rot |
| 62 | H | 3,6-Disulfo-2-naphthol | rot |
| 63 | H | 6,8-Disulfo-2-naphthol | orange |
| 64 | H | N-Acetoacetyl-3-sulfo-anilin | gelb |
| 65 | H | 1-(4'-Sulfophenyl)-3-carbethoxy-5-pyrazolon | gelb |
| 66 | Sulfo | 5-Sulfo-1,4-dimethyl-2-hydroxy-6-pyridon | gelb |
| 67 | Sulfo | 5-Sulfo-8-hydroxy-chinolin | orange |
| 68 | Sulfo | N-Acetoacetyl-2-carboxy-anilin | gelb |
| 69 | H | N-Ethyl-N-(3'-sulfobenzyl)-anilin | orange |
| 70 | H | N,N-Bis-(ß-hydroxyethyl)-anilin | orange |
| 71 | H | 2-Sulfo-5-acetylamino-anilin | goldgelb |
| 72 | Sulfo | 3-Acetylamino-1-N-[4'-chlor-6'-(3"-ß-sulfatoethylsulfonyl-phenyl)-amino-s-triazin-2'-yl]-anilin | gelb |
| 73 | Sulfo | 1-(2'-Chlor-5'-sulfo-phenyl)-3-methyl-5-pyrazolon | gelb |
| 74 | Sulfo | 1-(2',5'-Disulfo-phenyl)-3-methyl-5-pyrazolon | gelb |
| 75 | H | 1-(4',8'-Disulfo-naphth-2'-yl)-3-methyl-5-pyrazolon | gelb |
| 76 | H | 1-(4'-Sulfophenyl)-3-methyl-5-amino-pyrazol | gelb |
| 77 | Sulfo | 3,6-Disulfo-1-naphthol | rotorange |
| 78 | Sulfo | 2-Acetylamino-6-sulfo-8-naphthol | rotorange |
| 79 | Sulfo | 4-Sulfo-1-naphthol | rotorange |
| 80 | Sulfo | 1-Benzoylamino-3,6-disulfo-8-naphthol | rot |

| Bsp. | Rest $R^o$ | Komponente H-K | Farbton |
|---|---|---|---|
| 81 | Sulfo | 1-Benzoylamino-4,6-disulfo-8-naphthol | rot |
| 82 | Sulfo | 3-Sulfo-1-naphthol | rotorange |
| 83 | Sulfo | 1-(4'-Sulfophenyl)-3-carboxy-5-pyrazolon | gelb |
| 84 | Sulfo | 1-(4'-Sulfophenyl)-3-methyl-5-pyrazolon | gelb |
| 85 | Sulfo | 5-Sulfo-1-naphthol | rotorange |
| 86 | Sulfo | N-Acetoacetyl-2-methoxy-5-methyl-4-(ß-sulfato-ethylsulfonyl)-anilin | gelb |
| 87 | Sulfo | N-Acetoacetyl-2-methoxy-5-(ß-sulfatoethylsulfonyl)-anilin | gelb |
| 88 | Sulfo | N-Acetoacetyl-2,5-dimethoxy-4-(ß-sulfatoethylsulfonyl)-anilin | gelb |
| 89 | Sulfo | N-Acetoacetyl-3-methoxy-4-(ß-sulfatoethylsulfonyl)-anilin | gelb |
| 90 | Sulfo | N-Acetoacetyl-4-methoxy-3-(ß-sulfatoethylsulfonyl)-anilin | gelb |
| 91 | Sulfo | 1-(4'-ß-Sulfatoethylsulfonyl-phenyl)-3-methyl-5-pyrazolon | gelb |
| 92 | Sulfo | 1-(4'-ß-Sulfatoethylsulfonyl-phenyl)-3-carbethoxy-5-pyrazolon | gelb |
| 93 | Sulfo | 3-Acetylamino-6-sulfo-8-naphthol | rotorange |
| 94 | Sulfo | N,N-Bis-(ß-sulfatoethyl)-anilin | gelb |
| 95 | Sulfo | N-Ethyl-N-(ß-sulfatoethyl)-anilin | gelb |
| 96 | Sulfo | 1-Acetylamino-4,6-disulfo-8-naphthol | rot |
| 97 | Sulfo | 1-[4'-Chlor-6'-(4"-ß-sulfato-ethylsulfonyl-phenyl)-amino-s-triazin-2'-yl]-amino-3,6-disulfo-8-naphthol | rot |
| 98 | Sulfo | 1-(4'-Chlor-6'-methoxy-s-triazin-2'-yl)-amino-3,6-disulfo-8-naphthol | rot |

| Bsp. | Rest $R^o$ | Komponente H-K | Farbton |
|---|---|---|---|
| 99 | Sulfo | 1-[4'-Chlor-6'-(4"-ß-sulfatoethylsulfonyl-phenyl)-amino-s-triazin-2'-yl]-amino-4,6-disulfo-8-naphthol | rot |
| 100 | Sulfo | 2-[4'-Chlor-6'-(4"-ß-sulfatoethylsulfonyl-phenyl)-amino-s-triazin-2'-yl]-amino-6-sulfo-8-naphthol | rot |
| 101 | Sulfo | 3-[4'-Chlor-6'-(4"-ß-sulfatoethylsulfonyl-phenyl)-amino-s-triazin-2'-yl]-amino-6-sulfo-8-naphthol | rot |
| 102 | Sulfo | 1-(4'-Chlor-6'-methoxy-s-triazin-2'-yl)-amino-4,6-disulfo-8-naphthol | rot |
| 103 | Sulfo | 6-Carboxy-2-naphthol | orange |

**Beispiele 104 bis 200**

In den nachfolgenden Tabellenbeispielen sind weitere erfindungsgemäße Azoverbindungen entsprechend der, in Form der freien Säure geschriebenen, allgemeinen Formel (B)

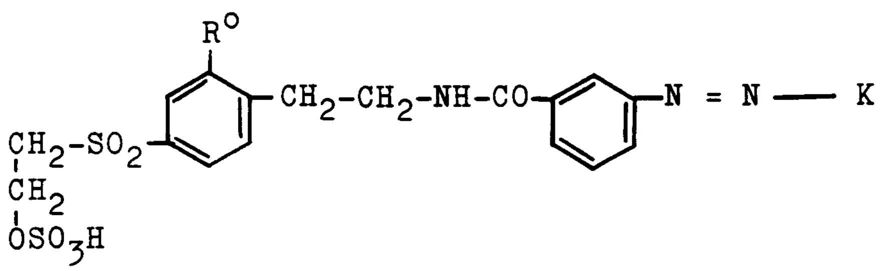

(B)

mit Hilfe ihrer Kupplungskomponente H-K beschrieben. Sie lassen sich in erfindungsgemäßer Weise, beispielsweise gemäß den obigen Ausführungsbeispielen durch Kupplung der angegebenen Kupplungskomponente mit dem Diazoniumsalz der aus Formel (B) ersichtlichen aromatischen Aminoverbindung entsprechend der allgemeinen Formel (5) herstellen und besitzen ebenfalls sehr gute faserreaktive Farbstoffeigenschaften. Sie liefern farbstarke Färbungen und Drucke auf den in der Beschreibung genannten Materialien, wie insbesondere Cellulosefasermaterialien, nach den für faserreaktive Farbstoffe üblichen Applikations- und Fixiermethoden mit den in dem jeweiligen Tabellenbeispiel angegebenen Farbton (hier auf Baumwolle) mit guten Echtheitseigenschaften; die in Klammern angegebenen Werte sind die Absorptionsmaxima ($\lambda_{max}$) in nm.

| Bsp. | Rest $R^o$ | Komponente H-K | Farbton |
|---|---|---|---|
| 104 | H | 2-Acetylamino-6-sulfo-8-naphthol | rotorange (493) |
| 105 | H | 1-Benzoylamino-3,6-disulfo-8-naphthol | rot (512) |
| 106 | H | 1-Benzoylamino-4,6-disulfo-8-naphthol | rot (510) |
| 107 | H | 1-(4'-Sulfophenyl)-3-carboxy-5-pyrazolon | gelb (430) |
| 108 | H | 1-(4'-Sulfophenyl)-3-methyl-5-pyrazolon | gelb |
| 109 | H | 5-Sulfo-1-naphthol | rotorange (490) |
| 110 | H | N-Acetoacetyl-2-methoxy-5-methyl-4-(ß-sulfatoethylsulfonyl)-anilin | gelb |
| 111 | H | N-Acetoacetyl-2-methoxy-5-(ß-sulfatoethylsulfonyl)-anilin | gelb |
| 112 | H | N-Acetoacetyl-2,5-dimethoxy-4-(ß-sulfatoethylsulfonyl)-anilin | gelb |
| 113 | H | N-Acetoacetyl-3-methoxy-4-(ß-sulfatoethylsulfonyl)-anilin | gelb |
| 114 | H | N-Acetoacetyl-4-methoxy-3-(ß-sulfatoethylsulfonyl)-anilin | gelb |
| 115 | H | 1-(4'-ß-Sulfatoethylsulfonyl-phenyl)-3-methyl-5-pyrazolon | gelb |
| 116 | H | 1-(4'-ß-Sulfatoethylsulfonyl-phenyl)-3-carbethoxy-5-pyrazolon | gelb |
| 117 | H | 3-Acetylamino-6-sulfo-8-naphthol | rotorange |
| 118 | H | N,N-Bis-(ß-sulfatoethyl)-anilin | gelb |
| 119 | H | N-Ethyl-N-(ß-sulfatoethyl)-anilin | gelb |
| 120 | H | N,N-Bis-(ß-sulfatoethyl)-3-chlor-anilin | gelb |
| 121 | H | 1-(N-ß-Sulfatoethyl)-4-methyl-2-hydroxy-6-pyridon | gelb |

| Bsp. | Rest $R^o$ | Komponente H-K | Farbton |
|---|---|---|---|
| 122 | H | 1-Acetylamino-3,6-disulfo-8-naphthol | rot |
| 123 | H | 1-Acetylamino-4,6-disulfo-8-naphthol | rot |
| 124 | H | 1-[4'-Chlor-6'-(4"-ß-sulfatoethylsulfonyl-phenyl)-amino-s-triazin-2'-yl]-amino-3,6-disulfo-8-naphthol | rot |
| 125 | H | 1-[4'-Chlor-6'-(4"-ß-sulfatoethylsulfonyl-phenyl)-amino-s-triazin-2'-yl]-amino-4,6-disulfo-8-naphthol | rot |
| 126 | H | 2-[4'-Chlor-6'-(4"-ß-sulfatoethylsulfonyl-phenyl)-amino-s-triazin-2'-yl]-amino-6-sulfo-8-naphthol | rot |
| 127 | H | 3-[4'-Chlor-6'-(4"-ß-sulfatoethylsulfonyl-phenyl)-amino-s-triazin-2'-yl]-amino-6-sulfo-8-naphthol | rot |
| 128 | H | 1-(4'-Chlor-6'-methoxy-s-triazin-2'-yl)-amino-3,6-disulfo-8-naphthol | rot |
| 129 | H | 1-(4'-Chlor-6'-methoxy-s-triazin-2'-yl)-amino-4,6-disulfo-8-naphthol | rot |
| 130 | Sulfo | 2-(4'-Chlor-6'-methoxy-s-triazin-2'-yl)-amino-6-sulfo-8-naphthol | rot |
| 131 | H | 3-(4'-Chlor-6'-methoxy-s-triazin-2'-yl)-amino-6-sulfo-8-naphthol | rot |
| 132 | Sulfo | 1-[4'-Fluor-6'-(4"-ß-sulfatoethylsulfonyl-phenyl)-amino-s-triazin-2'-yl]-amino-3,6-disulfo-8-naphthol | rot |
| 133 | H | 1-[4'-Fluor-6'-(4"-ß-sulfatoethylsulfonyl-phenyl)-amino-s-triazin-2'-yl]-amino-4,6-disulfo-8-naphthol | rot |
| 134 | H | 2-[4'-Fluor-6'-(4"-ß-sulfatoethylsulfonyl-phenyl)-amino-s-triazin-2'-yl]-amino-6-sulfo-8-naphthol | rot |

| Bsp. | Rest R° | Komponente H-K | Farbton |
|---|---|---|---|
| 135 | Sulfo | 3-[4'-Fluor-6'-(4"-ß-sulfato-ethylsulfonyl-phenyl)-amino-s-triazin-2'-yl]-amino-6-sulfo-8-naphthol | rot |
| 136 | H | 1-(4'-ß-Sulfatoethylsulfonyl-benzoyl)-amino-3,6-disulfo-8-naphthol | rot |
| 137 | H | 1-(4'-ß-Sulfatoethylsulfonyl-benzoyl)-amino-4,6-disulfo-8-naphthol | rot |
| 138 | Sulfo | 2-(4'-ß-Sulfatoethylsulfonyl-benzoyl)-amino-6-sulfo-8-naphthol | rot |
| 139 | Sulfo | 3-(4'-ß-Sulfatoethylsulfonyl-benzoyl)-amino-6-sulfo-8-naphthol | rot |
| 140 | H | 1-{ß-[4'-Chlor-6'-(4"-ß'-sulfatoethylsulfonyl-phenyl)-ethyl]-amino-s-triazin-2'-yl}-amino-3,6-disulfo-8-naphthol | rot |
| 141 | H | 1-{ß-[4'-Chlor-6'-(4"-ß'-sulfatoethylsulfonyl-phenyl)-ethyl]-amino-s-triazin-2'-yl}-amino-4,6-disulfo-8-naphthol | rot |
| 142 | Sulfo | 1-[4'-Chlor-6'-(3"-ß-sulfato-ethylsulfonyl-phenyl)-amino-s-triazin-2'-yl]-amino-3,6-disulfo-8-naphthol | rot |
| 143 | Sulfo | 1-[4'-Chlor-6'-(3"-ß-sulfato-ethylsulfonyl-phenyl)-amino-s-triazin-2'-yl]-amino-4,6-disulfo-8-naphthol | rot |
| 144 | H | 1-[4'-Chlor-6'-(4"-sulfophenyl)-amino-s-triazin-2'-yl]-amino-3,6-disulfo-8-naphthol | rot |
| 145 | H | 1-[4'-Chlor-6'-(4"-sulfophenyl)-amino-s-triazin-2'-yl]-amino-4,6-disulfo-8-naphthol | rot |
| 146 | Sulfo | 1-[4'-Chlor-6'-(2",5"-disulfo-phenyl)-amino-s-triazin-2'-yl]-amino-4,6-disulfo-8-naphthol | rot |

| Bsp. | Rest $R^o$ | Komponente H-K | Farbton |
|---|---|---|---|
| 147 | Sulfo | 1-[4'-Fluor-6'-(3",5"-disulfo-phenyl)-amino-s-triazin-2'-yl]-amino-3,6-disulfo-8-naphthol | rot |
| 148 | H | 1-[4'-Fluor-6'-(3",5"-disulfo-phenyl)-amino-s-triazin-2'-yl]-amino-4,6-disulfo-8-naphthol | rot |
| 149 | Sulfo | 6-Sulfo-2-naphthol | rot |
| 150 | Sulfo | 8-Sulfo-2-naphthol | rot |
| 151 | H | 1-Acetylamino-3,6-disulfo-8-naphthol | blaustichig rot |
| 152 | H | 1-Phenylureido-4,6-disulfo-8-naphthol | blaustichig rot |
| 153 | H | 3,6,8-Trisulfo-1-naphthol | rot |
| 154 | H | 3,6-Disulfo-2-naphthol | rot |
| 155 | H | 6,8-Disulfo-2-naphthol | orange |
| 156 | H | N-Acetoacetyl-3-sulfo-anilin | gelb |
| 157 | H | 1-(4'-Sulfophenyl)-3-carbethoxy-5-pyrazolon | gelb |
| 158 | Sulfo | 5-Sulfo-1,4-dimethyl-2-hydroxy-6-pyridon | gelb |
| 159 | H | 5-Sulfo-8-hydroxy-chinolin | orange |
| 160 | Sulfo | N-Acetoacetyl-2-carboxy-anilin | gelb |
| 161 | Sulfo | N-Ethyl-N-(3'-sulfo-benzyl)-anilin | orange |
| 162 | Sulfo | N,N-Bis-(ß-hydroxyethyl)-anilin | orange |
| 163 | H | 2-Sulfo-5-acetylamino-anilin | goldgelb |
| 164 | Sulfo | 3-Acetylamino-1-N-[4'-chlor-6'-(3"-ß-sulfatoethylsulfonyl-phenyl)-amino-s-triazin-2'-yl]-anilin | gelb |
| 165 | H | 1-(2'-Chlor-5'-sulfo-phenyl)-3-methyl-5-pyrazolon | gelb |
| 166 | H | 1-(2',5'-Disulfo-phenyl)-3-methyl-5-pyrazolon | gelb |
| 167 | Sulfo | 1-(4',8'-Disulfo-naphth-2'-yl)-3-methyl-5-pyrazolon | gelb |
| 168 | Sulfo | 1-(4'-Sulfophenyl)-3-methyl-5-amino-pyrazol | gelb |

| Bsp. | Rest R° | Komponente H-K | Farbton |
|---|---|---|---|
| 169 | H | 2-Acetylamino-6-sulfo-8-naphthol | rotorange (493) |
| 170 | Sulfo | 4-Sulfo-1-naphthol | rotorange (489) |
| 171 | Sulfo | 3,6-Disulfo-1-naphthol | rotorange (490) |
| 172 | Sulfo | 1-Benzoylamino-3,6-disulfo-8-naphthol | rot (512) |
| 173 | Sulfo | 1-Benzoylamino-4,6-disulfo-8-naphthol | rot (510) |
| 174 | Sulfo | 3-Sulfo-1-naphthol | rotorange (490) |
| 175 | Sulfo | 1-(4'-Sulfophenyl)-3-carboxy-5-pyrazolon | gelb (430) |
| 176 | Sulfo | 1-(4'-Sulfophenyl)-3-methyl-5-pyrazolon | gelb |
| 177 | Sulfo | 5-Sulfo-1-naphthol | rotorange (490) |
| 178 | Sulfo | N-Acetoacetyl-2-methoxy-5-methyl-4-(ß-sulfato-ethylsulfonyl)-anilin | gelb |
| 179 | Sulfo | N-Acetoacetyl-2-methoxy-5-(ß-sulfatoethylsulfonyl)-anilin | gelb |
| 180 | Sulfo | N-Acetoacetyl-2,5-dimethoxy-4-(ß-sulfatoethylsulfonyl)-anilin | gelb |
| 181 | Sulfo | N-Acetoacetyl-3-methoxy-4-(ß-sulfatoethylsulfonyl)-anilin | gelb |
| 182 | Sulfo | N-Acetoacetyl-4-methoxy-3-(ß-sulfatoethylsulfonyl)-anilin | gelb |
| 183 | Sulfo | 1-(4'-ß-Sulfatoethylsulfonyl)-phenyl)-3-methyl-5-pyrazolon | gelb |
| 184 | Sulfo | 1-(4'-ß-Sulfatoethylsulfonyl)-phenyl)-3-carbethoxy-5-pyrazolon | gelb |
| 185 | Sulfo | 3-Acetylamino-6-sulfo-8-naphthol | rotorange |
| 186 | Sulfo | N,N-Bis-(ß-sulfatoethyl)-anilin | gelb |

| Bsp. | Rest $R^o$ | Komponente H-K | Farbton |
|---|---|---|---|
| 187 | Sulfo | N-Ethyl-N-(ß-sulfatoethyl)-anilin | gelb |
| 188 | Sulfo | N,N-Bis-(ß-sulfatoethyl)-3-chlor-anilin | gelb |
| 189 | Sulfo | 1-(N-ß-Sulfatoethyl)-4-methyl-2-hydroxy-6-pyridon | gelb |
| 190 | Sulfo | 1-Acetylamino-3,6-disulfo-8-naphthol | rot |
| 191 | Sulfo | 1-Acetylamino-4,6-disulfo-8-naphthol | rot |
| 192 | Sulfo | 1-[4'-Chlor-6'-(4"-ß-sulfato-ethylsulfonyl-phenyl)-amino-s-triazin-2'-yl]-amino-3,6-disulfo-8-naphthol | rot |
| 193 | Sulfo | 1-[4'-Chlor-6'-(4"-ß-sulfato-ethylsulfonyl-phenyl)-amino-s-triazin-2'-yl]-amino-4,6-disulfo-8-naphthol | rot |
| 194 | Sulfo | 2-[4'-Chlor-6'-(4"-ß-sulfato-ethylsulfonyl-phenyl)-amino-s-triazin-2'-yl]-amino-6-sulfo-8-naphthol | rot |
| 195 | Sulfo | 3-[4'-Chlor-6'-(4"-ß-sulfato-ethylsulfonyl-phenyl)-amino-s-triazin-2'-yl]-amino-6-sulfo-8-naphthol | rot |
| 196 | Sulfo | 1-(4'-Chlor-6'-methoxy-s-triazin-2'-yl)-amino-3,6-disulfo-8-naphthol | rot |
| 197 | Sulfo | 1-(4'-Chlor-6'-methoxy-s-triazin-2'-yl)-amino-4,6-disulfo-8-naphthol | rot |
| 198 | Sulfo | 3-(4'-Chlor-6'-methoxy-s-triazin-2'-yl)-amino-6-sulfo-8-naphthol | rot |
| 199 | H | 3,6-Disulfo-1-naphthol | rotorange (490) |
| 200 | H | 3-Sulfo-1-naphthol | orange (483) |

**Beispiele 201 bis 340**

In den nachfolgenden Tabellenbeispielen sind weitere erfindungsgemäße Azoverbindungen entsprechend der, in Form der freien Säure geschriebenen, allgemeinen Formel (C)

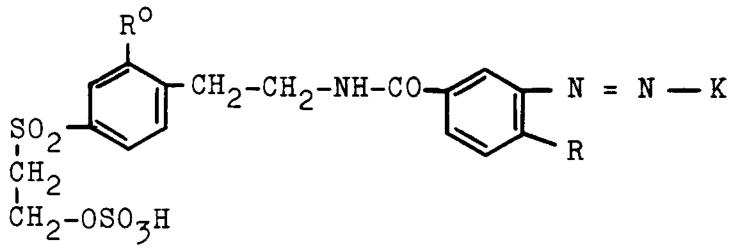

(C)

mit Hilfe der Formelreste und deren Kupplungskomponente H-K beschrieben. Sie lassen sich in erfindungsgemäßer Weise, beispielsweise gemäß den obigen Ausführungsbeispielen durch Kupplung der angegebenen Kupplungskomponente mit dem Diazoniumsalz der aus Formel (C) ersichtlichen aromatischen Aminoverbindung entsprechend der allgemeinen Formel (5) herstellen und besitzen ebenfalls sehr gute faserreaktive Farbstoffeigenschaften. Sie liefern farbstarke Färbungen und Drucke auf den in der Beschreibung genannten Materialien, wie insbesondere Cellulosefasermaterialien, nach den für faserreaktive Farbstoffe üblichen Applikations- und Fixiermethoden mit den in dem jeweiligen Tabellenbeispiel angegebenen Farbton (hier auf Baumwolle) mit guten Echtheitseigenschaften; die in Klammern angegebenen Werte sind die Absorptionsmaxima ($\lambda_{max}$) in nm.

| Bsp. | Rest $R^o$ | Rest R | Komponente H-K | Farbton |
|---|---|---|---|---|
| 201 | Sulfo | Chlor | 3,6-Disulfo-1-naphthol | rotorange (480) |
| 202 | Sulfo | Methyl | 3,6-Disulfo-1-naphthol | rotorange (487) |
| 203 | Sulfo | Chlor | 2-Acetylamino-6-sulfo-8-naphthol | rotorange |
| 204 | Sulfo | Methoxy | 4-Sulfo-1-naphthol | rotorange |
| 205 | Sulfo | Methoxy | 1-Benzoylamino-3,6-disulfo-8-naphthol | rot |
| 206 | Sulfo | Methoxy | 1-Benzoylamino-4,6-disulfo-8-naphthol | rot |
| 207 | Sulfo | Methoxy | 3-Sulfo-1-naphthol | rotorange |

| Bsp. | Rest $R^o$ | Rest R | Komponente H-K | Farbton |
|---|---|---|---|---|
| 208 | Sulfo | Methoxy | 3,6-Disulfo-naphthol | rotorange |
| 209 | Sulfo | Chlor | 1-(4'-Sulfophenyl)-3-carboxy-5-pyrazolon | gelb |
| 210 | Sulfo | Chlor | 1-(4'-Sulfophenyl)-3-methyl-5-pyrazolon | gelb |
| 211 | Sulfo | Chlor | 5-Sulfo-1-naphthol | rotorange |
| 212 | Sulfo | Methyl | N-Acetoacetyl-2-methoxy-5-methyl-4-(ß-sulfato-ethylsulfonyl)-anilin | gelb |
| 213 | Sulfo | Chlor | N-Acetoacetyl-2-methoxy-5-(ß-sulfatoethyl-sulfonyl)-anilin | gelb |
| 214 | Sulfo | Chlor | N-Acetoacetyl-2,5-dimethoxy-4-(ß-sulfato-ethylsulfonyl)-anilin | gelb |
| 215 | Sulfo | Methoxy | N-Acetoacetyl-3-methoxy-4-(ß-sulfatoethyl-sulfonyl)-anilin | gelb |
| 216 | Sulfo | Chlor | N-Acetoacetyl-4-methoxy-3-(ß-sulfatoethyl-sulfonyl)-anilin | gelb |
| 217 | Sulfo | Methoxy | 1-(4'-ß-Sulfatoethyl-sulfonyl-phenyl)-3-methyl-5-pyrazolon | gelb |
| 218 | Sulfo | Methyl | 1-(4'-ß-Sulfatoethyl-sulfonyl-phenyl)-3-carb-ethoxy-5-pyrazolon | gelb |
| 219 | Sulfo | Methoxy | 3-Acetylamino-6-sulfo-8-naphthol | rotorange |
| 220 | Sulfo | Methoxy | N,N-Bis-(ß-sulfatoethyl)-anilin | gelb |
| 221 | Sulfo | Methoxy | N-Ethyl-N-(ß-sulfato-ethyl)-anilin | gelb |
| 222 | Sulfo | Methoxy | N,N-Bis-(ß-sulfatoethyl)-3-chlor-anilin | gelb |
| 223 | Sulfo | Methoxy | 1-(N-ß-Sulfatoethyl)-4-methyl-2-hydroxy-6-pyridon | gelb |
| 224 | Sulfo | Methoxy | 1-Acetylamino-3,6-disulfo-8-naphthol | rot |
| 225 | Sulfo | Methoxy | 1-Acetylamino-4,6-disulfo-8-naphthol | rot |
| 226 | Sulfo | Chlor | 1-[4'-Chlor-6'-(4"-ß-sulfatoethylsulfonyl)-phenyl)-amino-s-triazin-2'-yl]-amino-3,6-disulfo-8-naphthol | rot |

| Bsp. | Rest $R^o$ | Rest R | Komponente H-K | Farbton |
|---|---|---|---|---|
| 227 | Sulfo | Methyl | 1-[4'-Chlor-6'-(4"-ß-sulfatoethylsulfonyl-phenyl)-amino-s-triazin-2'-yl]-amino-4,6-disulfo-8-naphthol | rot |
| 228 | Sulfo | Methyl | 2-[4'-Chlor-6'-(4"-ß-sulfatoethylsulfonyl-phenyl)-amino-s-triazin-2'-yl]-amino-6-sulfo-8-naphthol | rot |
| 229 | Sulfo | Methoxy | 3-[4'-Chlor-6'-(4"-ß-sulfatoethylsulfonyl-phenyl)-amino-s-triazin-2'-yl]-amino-6-sulfo-8-naphthol | rot |
| 230 | Sulfo | Methoxy | 1-(4'-Chlor-6'-methoxy-s-triazin-2'-yl)-amino-3,6-disulfo-8-naphthol | rot |
| 231 | Sulfo | Methoxy | 1-(4'-Chlor-6'-methoxy-s-triazin-2'-yl)-amino-4,6-disulfo-8-naphthol | rot |
| 232 | Sulfo | Methoxy | 2-(4'-Chlor-6'-methoxy-s-triazin-2'-yl)-amino-6-sulfo-8-naphthol | rot |
| 233 | Sulfo | Methoxy | 3-(4'-Chlor-6'-methoxy-s-triazin-2'-yl)-amino-6-sulfo-8-naphthol | rot |
| 234 | Sulfo | Methoxy | 1-[4'-Fluor-6'-(4"-ß-sulfatoethylsulfonyl-phenyl)-amino-s-triazin-2'-yl]-amino-3,6-disulfo-8-naphthol | rot |
| 235 | Sulfo | Methoxy | 1-[4'-Fluor-6'-(4"-ß-sulfatoethylsulfonyl-phenyl)-amino-s-triazin-2'-yl]-amino-4,6-disulfo-8-naphthol | rot |
| 236 | Sulfo | Methoxy | 2-[4'-Fluor-6'-(4"-ß-sulfatoethylsulfonyl-phenyl)-amino-s-triazin-2'-yl]-amino-6-sulfo-8-naphthol | rot |
| 237 | Sulfo | Methoxy | 3-[4'-Fluor-6'-(4"-ß-sulfatoethylsulfonyl-phenyl)-amino-s-triazin-2'-yl]-amino-6-sulfo-8-naphthol | rot |
| 238 | Sulfo | Chlor | 1-(4'-ß-Sulfatoethylsulfonyl-benzoyl)-amino-3,6-disulfo-8-naphthol | rot |

| Bsp. | Rest $R^o$ | Rest R | Komponente H-K | Farbton |
|---|---|---|---|---|
| 239 | Sulfo | Chlor | 1-(4'-ß-Sulfatoethylsulfonyl-benzoyl)-amino-4,6-disulfo-8-naphthol | rot |
| 240 | Sulfo | Methyl | 2-(4'-ß-Sulfatoethylsulfonyl-benzoyl)-amino-6-sulfo-8-naphthol | rot |
| 241 | Sulfo | Methyl | 3-(4'-ß-Sulfatoethylsulfonyl-benzoyl)-amino-6-sulfo-8-naphthol | rot |
| 242 | Sulfo | Methyl | 1-{ß-[4'-Chlor-6'-(4"-ß'-sufatoethylsulfonyl-phenyl)-ethyl]-amino-s-triazin-2'-yl}-amino-3,6-disulfo-8-naphthol | rot |
| 243 | Sulfo | Methyl | 1-{ß-[4'-Chlor-6'-(4"-ß'-sufatoethylsulfonyl-phenyl)-ethyl]-amino-s-triazin-2'-yl}-amino-4,6-disulfo-8-naphthol | rot |
| 244 | Sulfo | Methyl | 1-[4'-Chlor-6'-(3"-ß'-sufatoethylsulfonyl-phenyl)-amino-s-triazin-2'-yl]-amino-3,6-disulfo-8-naphthol | rot |
| 245 | Sulfo | Methyl | 1-[4'-Chlor-6'-(3"-ß'-sufatoethylsulfonyl-phenyl)-amino-s-triazin-2'-yl]-amino-4,6-disulfo-8-naphthol | rot |
| 246 | Sulfo | Methoxy | 1-[4'-Chlor-6'-(4"-sulfo-phenyl)-amino-s-triazin-2'-yl]-amino-3,6-disulfo-8-naphthol | rot |
| 247 | Sulfo | Methoxy | 1-[4'-Chlor-6'-(4"-sulfo-phenyl)-amino-s-triazin-2'-yl]-amino-4,6-disulfo-8-naphthol | rot |
| 248 | Sulfo | Methoxy | 1-[4'-Chlor-6'-(2",5"-disulfo-amino-s-triazin-2'-yl]-amino-4,6-disulfo-8-naphthol | rot |
| 249 | Sulfo | Methoxy | 1-[4'-Fluor-6'-(3",5"-disulfo-amino-s-triazin-2'-yl]-amino-3,6-disulfo-8-naphthol | rot |

| Bsp. | Rest $R^O$ | Rest R | Komponente H-K | Farbton |
|---|---|---|---|---|
| 250 | Sulfo | Methoxy | 1-[4'-Fluor-6'-(3",5"-disulfo-amino-s-triazin-2'-yl]-amino-4,6-disulfo-8-naphthol | rot |
| 251 | Sulfo | Methoxy | 6-Sulfo-2-naphthol | rot |
| 252 | Sulfo | Methoxy | 8-Sulfo-2-naphthol | rot |
| 253 | Sulfo | Methoxy | 1-Acetylamino-3,6-disulfo-8-naphthol | blaustichig rot |
| 254 | Sulfo | Methoxy | 1-Phenylureido-4,6-disulfo-8-naphthol | blaustichig rot |
| 255 | Sulfo | Methoxy | 3,6,8-Trisulfo-1-naphthol | rot |
| 256 | Sulfo | Methoxy | 3,6-Disulfo-2-naphthol | rot |
| 257 | Sulfo | Methoxy | 6,8-Disulfo-2-naphthol | orange |
| 258 | Sulfo | Methoxy | N-Acetoacetyl-3-sulfo-anilin | gelb |
| 259 | Sulfo | Methoxy | 1-(4'-Sulfophenyl)-3-carbethoxy-5-pyrazolon | gelb |
| 260 | Sulfo | Methoxy | 5-Sulfo-1,4-dimethyl-2-hydroxy-6-pyridon | gelb |
| 261 | Sulfo | Methoxy | 5-Sulfo-8-hydroxy-chinolin | orange |
| 262 | Sulfo | Methoxy | N-Acetoacetyl-2-carboxy-anilin | gelb |
| 263 | Sulfo | Chlor | N-Ethyl-N-(3'-sulfo-benzyl)-anilin | orange |
| 264 | Sulfo | Methyl | N,N-Bis-(ß-hydroxyethyl)-anilin | orange |
| 265 | Sulfo | Methyl | 2-Sulfo-5-acetylamino-anilin | goldgelb |
| 266 | Sulfo | Methyl | 3-Acetylamino-1-N-[4'-chlor-6'-(3"-ß-sulfato-ethylsulfonyl-phenyl)-amino-s-triazin-2'-yl]-anilin | gelb |
| 267 | Sulfo | Methyl | 1-(2'-Chlor-5'-sulfo-phenyl)-3-methyl-5-pyrazolon | gelb |
| 268 | Sulfo | Methyl | 1-(2',5'-Disulfo-phenyl)-3-methyl-5-pyrazolon | gelb |
| 269 | Sulfo | Methyl | 1-(4',8'-Disulfo-naphth-2'-yl)-3-methyl-5-pyrazolon | gelb |
| 270 | Sulfo | Methyl | 1-(4'-Sulfophenyl)-3-methyl-5-amino-pyrazol | gelb |

| Bsp. | Rest $R^o$ | Rest R | Komponente H-K | Farbton |
|---|---|---|---|---|
| 271 | H | Chlor | 3,6-Disulfo-1-naphthol | rotorange (480) |
| 272 | H | Methyl | 3,6-Disulfo-1-naphthol | rotorange (487) |
| 273 | H | Chlor | 2-Acetylamino-6-sulfo-8-naphthol | rotorange |
| 274 | H | Methoxy | 4-Sulfo-1-naphthol | rotorange |
| 275 | H | Methoxy | 1-Benzoylamino-3,6-disulfo-8-naphthol | rot |
| 276 | H | Methoxy | 1-Benzoylamino-4,6-disulfo-8-naphthol | rot |
| 277 | H | Methoxy | 3-Sulfo-1-naphthol | rotorange |
| 278 | H | Methoxy | 3,6-Disulfo-naphthol | rotorange (500) |
| 279 | H | Chlor | 1-(4'-Sulfophenyl)-3-carboxy-5-pyrazolon | gelb |
| 280 | H | Chlor | 1-(4'-Sulfophenyl)-3-methyl-5-pyrazolon | gelb |
| 281 | H | Chlor | 5-Sulfo-1-naphthol | rotorange |
| 282 | H | Methyl | N-Acetoacetyl-2-methoxy-5-methyl-4-(ß-sulfato-ethylsulfonyl)-anilin | gelb |
| 283 | H | Chlor | N-Acetoacetyl-2-methoxy-5-(ß-sulfatoethyl-sulfonyl)-anilin | gelb |
| 284 | H | Chlor | N-Acetoacetyl-2,5-dimethoxy-4-(ß-sulfato-ethylsulfonyl)-anilin | gelb |
| 285 | H | Methoxy | N-Acetoacetyl-3-methoxy-4-(ß-sulfatoethyl-sulfonyl)-anilin | gelb |
| 286 | H | Chlor | N-Acetoacetyl-4-methoxy-3-(ß-sulfatoethyl-sulfonyl)-anilin | gelb |
| 287 | H | Methoxy | 1-(4'-ß-Sulfatoethyl-sulfonyl-phenyl)-3-methyl-5-pyrazolon | gelb |
| 288 | H | Methyl | 1-(4'-ß-Sulfatoethyl-sulfonyl-phenyl)-3-carb-ethoxy-5-pyrazolon | gelb |
| 289 | H | Methoxy | 3-Acetylamino-6-sulfo-8-naphthol | rotorange |

| Bsp. | Rest $R^o$ | Rest R | Komponente H-K | Farbton |
|---|---|---|---|---|
| 290 | H | Methoxy | N,N-Bis-(ß-sulfatoethyl)-anilin | gelb |
| 291 | H | Methoxy | N-Ethyl-N-(ß-sulfato-ethyl)-anilin | gelb |
| 292 | H | Methyl | N,N-Bis-(ß-sulfatoethyl)-3-chlor-anilin | gelb |
| 293 | H | Methyl | 1-(N-ß-Sulfatoethyl)-4-methyl-2-hydroxy-6-pyridon | gelb |
| 294 | H | Methyl | 1-Acetylamino-3,6-disulfo-8-naphthol | rot |
| 295 | H | Methoxy | 1-Acetylamino-4,6-disulfo-8-naphthol | rot |
| 296 | H | Chlor | 1-[4'-Chlor-6'-(4"-ß-sulfatoethylsulfonyl)-phenyl)-amino-s-triazin-2'-yl]-amino-3,6-disulfo-8-naphthol | rot |
| 297 | H | Methyl | 1-[4'-Chlor-6'-(4"-ß-sulfatoethylsulfonyl-phenyl)-amino-s-triazin-2'-yl]-amino-4,6-disulfo-8-naphthol | rot |
| 298 | H | Methyl | 1-[4'-Chlor-6'-(4"-ß-sulfatoethylsulfonyl-phenyl)-amino-s-triazin-2'-yl]-amino-6-sulfo-8-naphthol | rot |
| 299 | H | Methoxy | 1-[4'-Chlor-6'-(4"-ß-sulfatoethylsulfonyl-phenyl)-amino-s-triazin-2'-yl]-amino-6-sulfo-8-naphthol | rot |
| 300 | H | Methoxy | 1-(4'-Chlor-6'-methoxy-s-triazin-2'-yl)-amino-3,6-disulfo-8-naphthol | rot |
| 301 | H | Chlor | 1-(4'-Chlor-6'-methoxy-s-triazin-2'-yl)-amino-4,6-disulfo-8-naphthol | rot |
| 302 | H | Methoxy | 2-(4'-Chlor-6'-methoxy-s-triazin-2'-yl)-amino-6-sulfo-8-naphthol | rot |

| Bsp. | Rest $R^o$ | Rest R | Komponente H-K | Farbton |
|---|---|---|---|---|
| 303 | H | Methoxy | 3-(4'-Chlor-6'-methoxy-s-triazin-2'-yl)-amino-6-sulfo-8-naphthol | rot |
| 304 | H | Methoxy | 1-[4'-Fluor-6'-(4"-ß-sulfatoethylsulfonyl-phenyl)-amino-s-triazin-2'-yl]-amino-3,6-disulfo-8-naphthol | rot |
| 305 | H | Methoxy | 1-[4'-Fluor-6'-(4"-ß-sulfatoethylsulfonyl-phenyl)-amino-s-triazin-2'-yl]-amino-4,6-disulfo-8-naphthol | rot |
| 306 | H | Chlor | 2-[4'-Fluor-6'-(4"-ß-sulfatoethylsulfonyl-phenyl)-amino-s-triazin-2'-yl]-amino-6-sulfo-8-naphthol | rot |
| 307 | H | Methyl | 3-[4'-Fluor-6'-(4"-ß-sulfatoethylsulfonyl-phenyl)-amino-s-triazin-2'-yl]-amino-6-sulfo-8-naphthol | rot |
| 308 | H | Methyl | 1-(4'-ß-Sulfatoethylsulfonyl-benzoyl)-amino-3,6-disulfo-8-naphthol | rot |
| 309 | H | Methyl | 1-(4'-ß-Sulfatoethylsulfonyl-benzoyl)-amino-4,6-disulfo-8-naphthol | rot |
| 310 | H | Methoxy | 2-(4'-ß-Sulfatoethylsulfonyl-benzoyl)-amino-6-sulfo-8-naphthol | rot |
| 311 | H | Chlor | 3-(4'-ß-Sulfatoethylsulfonyl-benzoyl)-amino-6-sulfo-8-naphthol | rot |
| 312 | H | Chlor | 1-{ß-[4'-Chlor-6'-(4"-ß'-sufatoethylsulfonyl-phenyl)-ethyl]-amino-s-triazin-2'-yl}-amino-3,6-disulfo-8-naphthol | rot |

| Bsp. | Rest $R^O$ | Rest R | Komponente H-K | Farbton |
|---|---|---|---|---|
| 313 | H | Methoxy | 1-{ß-[4'-Chlor-6'-(4"-ß'-sufatoethylsulfonyl-phenyl)-ethyl]-amino-s-triazin-2'-yl}-amino-4,6-disulfo-8-naphthol | rot |
| 314 | H | Methyl | 1-[4'-Chlor-6'-(3"-ß'-sufatoethylsulfonyl-phenyl)-amino-s-triazin-2'-yl]-amino-3,6-disulfo-8-naphthol | rot |
| 315 | H | Methyl | 1-[4'-Chlor-6'-(3"-ß'-sufatoethylsulfonyl-phenyl)-amino-s-triazin-2'-yl]-amino-4,6-disulfo-8-naphthol | rot |
| 316 | H | Methoxy | 1-[4'-Chlor-6'-(4"-sulfo-phenyl)-amino-s-triazin-2'-yl]-amino-3,6-disulfo-8-naphthol | rot |
| 317 | H | Chlor | 1-[4'-Chlor-6'-(4"-sulfo-phenyl)-amino-s-triazin-2'-yl]-amino-4,6-disulfo-8-naphthol | rot |
| 318 | H | Methoxy | 1-[4'-Chlor-6'-(2",5"-disulfo-amino-s-triazin-2'-yl]-amino-4,6-disulfo-8-naphthol | rot |
| 319 | H | Methoxy | 1-[4'-Fluor-6'-(3",5"-disulfo-amino-s-triazin-2'-yl]-amino-3,6-disulfo-8-naphthol | rot |
| 320 | H | Methyl | 1-[4'-Fluor-6'-(3",5"-disulfo-amino-s-triazin-2'-yl]-amino-4,6-disulfo-8-naphthol | rot |
| 321 | H | Methoxy | 6-Sulfo-2-naphthol | rot |
| 322 | H | Methoxy | 8-Sulfo-2-naphthol | rot |
| 323 | H | Methoxy | 1-Acetylamino-3,6-disulfo-8-naphthol | blaustichig rot |
| 324 | H | Methoxy | 1-Phenylureido-4,6-disulfo-8-naphthol | blaustichig rot |

| Bsp. | Rest $R^o$ | Rest R | Komponente H-K | Farbton |
|---|---|---|---|---|
| 325 | H | Methoxy | 3,6,8-Trisulfo-1-naphthol | rot |
| 326 | H | Methoxy | 3,6-Disulfo-2-naphthol | rot |
| 327 | H | Methyl | 6,8-Disulfo-2-naphthol | orange |
| 328 | H | Methyl | N-Acetoacetyl-3-sulfo-anilin | gelb |
| 329 | H | Methyl | 1-(4'-Sulfophenyl)-3-carbethoxy-5-pyrazolon | gelb |
| 330 | H | Methoxy | 5-Sulfo-1,4-dimethyl-2-hydroxy-6-pyridon | gelb |
| 331 | H | Methoxy | 5-Sulfo-8-hydroxy-chinolin | orange |
| 332 | H | Methoxy | N-Acetoacetyl-2-carboxy-anilin | gelb |
| 333 | H | Methoxy | N-Ethyl-N-(3'-sulfo-benzyl)-anilin | orange |
| 334 | H | Methoxy | N,N-Bis-(ß-hydroxyethyl)-anilin | orange |
| 335 | H | Methoxy | 2-Sulfo-5-acetylamino-anilin | goldgelb |
| 336 | H | Methyl | 3-Acetylamino-1-N-[4'-chlor-6'-(3"-ß-sulfato-ethylsulfonyl-phenyl)-amino-s-triazin-2'-yl]-anilin | gelb |
| 337 | H | Chlor | 1-(2'-Chlor-5'-sulfo-phenyl)-3-methyl-5-pyrazolon | gelb |
| 338 | H | Methyl | 1-(2',5'-Disulfo-phenyl)-3-methyl-5-pyrazolon | gelb |
| 339 | H | Methoxy | 1-(4',8'-Disulfo-naphth-2'-yl)-3-methyl-5-pyrazolon | gelb |
| 340 | H | Methoxy | 1-(4'-Sulfophenyl)-3-methyl-5-amino-pyrazol | gelb |

**Beispiele 341 bis 407**

In den nachfolgenden Tabellenbeispielen sind weitere erfindungsgemäße Azoverbindungen entsprechend der, in Form der freien Säure geschriebenen, allgemeinen Formel (D)

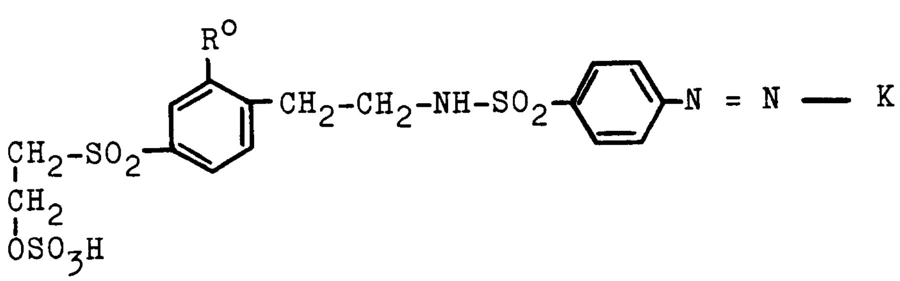

(D)

mit Hilfe ihrer Kupplungskomponente H-K beschrieben. Sie lassen sich in erfindungsgemäßer Weise, beispielsweise gemäß den obigen Ausführungsbeispielen durch Kupplung der angegebenen Kupplungskomponente mit dem Diazoniumsalz der aus Formel (D) ersichtlichen aromatischen Aminoverbindung entsprechend der allgemeinen Formel (5) herstellen und besitzen ebenfalls sehr gute faserreaktive Farbstoffeigenschaften. Sie liefern farbstarke Färbungen und Drucke auf den in der Beschreibung genannten Materialien, wie insbesondere Cellulosefasermaterialien, nach den für faserreaktive Farbstoffe üblichen Applikations- und Fixiermethoden mit den in dem jeweiligen Tabellenbeispiel angegebenen Farbton (hier auf Baumwolle) mit guten Echtheitseigenschaften; die in Klammern angegebenen Werte sind die Absorptionsmaxima ($\lambda_{max}$) in nm.

| Bsp. | Rest $R^o$ | Komponente H-K | Farbton |
|---|---|---|---|
| 341 | H | 2-Acetylamino-6-sulfo-8-naphthol | rotorange |
| 342 | H | 4-Sulfo-1-naphthol | rotorange (480) |
| 343 | H | 1-Benzoylamino-3,6-disulfo-8-naphthol | rot |
| 344 | H | 1-Benzoylamino-4,6-disulfo-8-naphthol | rot (514) |
| 345 | H | 3-Sulfo-1-naphthol | rotorange |
| 346 | H | 3,6-Disulfo-1-naphthol | rotorange (489) |
| 347 | H | 1-(4'-Sulfophenyl)-3-carboxy-5-pyrazolon | gelb |
| 348 | H | 1-(4'-Sulfophenyl)-3-methyl-5-pyrazolon | gelb |
| 349 | H | N-Acetoacetyl-2-methoxy-5-methyl-4-(ß-sulfato-ethylsulfonyl)-anilin | gelb |
| 350 | Sulfo | N-Acetoacetyl-2-methoxy-5-(ß-sulfatoethylsulfonyl)-anilin | gelb |
| 351 | Sulfo | N-Acetoacetyl-2,5-dimethoxy-4-(ß-sulfatoethylsulfonyl)-anilin | gelb |
| 352 | Sulfo | N-Acetoacetyl-3-methoxy-4-(ß-sulfatoethylsulfonyl)-anilin | gelb |
| 353 | Sulfo | N-Acetoacetyl-4-methoxy-3-(ß-sulfatoethylsulfonyl)-anilin | gelb |
| 354 | Sulfo | 1-(4'-ß-Sulfatoethylsulfonyl-phenyl)-3-methyl-5-pyrazolon | gelb |
| 355 | H | 1-(4'-ß-Sulfatoethylsulfonyl-phenyl)-3-carbethoxy-5-pyrazolon | gelb |
| 356 | H | 3-Acetylamino-6-sulfo-8-naphthol | rotorange |
| 357 | H | N,N-Bis-(ß-sulfatoethyl)-anilin | gelb |
| 358 | Sulfo | N-Ethyl-N-(ß-sulfatoethyl)-anilin | gelb |
| 359 | Sulfo | N,N-Bis-(ß-sulfatoethyl)-3-chlor-anilin | gelb |
| 360 | H | 1-(N-ß-Sulfatoethyl)-4-methyl-2-hydroxy-6-pyridon | gelb |

| Bsp. | Rest $R^o$ | Komponente H-K | Farbton |
|---|---|---|---|
| 361 | H | 1-Acetylamino-3,6-disulfo-8-naphthol | rot (505) |
| 362 | H | 1-Acetylamino-4,6-disulfo-8-naphthol | rot |
| 363 | Sulfo | 1-[4'-Chlor-6'-(4"-ß-sulfato-ethylsulfonyl-phenyl)-amino-s-triazin-2'-yl]-amino-3,6-disulfo-8-naphthol | rot |
| 364 | H | 1-[4'-Chlor-6'-(4"-ß-sulfato-ethylsulfonyl-phenyl)-amino-s-triazin-2'-yl]-amino-4,6-disulfo-8-naphthol | rot |
| 365 | Sulfo | 2-[4'-Chlor-6'-(4"-ß-sulfato-ethylsulfonyl-phenyl)-amino-s-triazin-2'-yl]-amino-6-sulfo-8-naphthol | rot |
| 366 | H | 3-[4'-Chlor-6'-(4"-ß-sulfato-ethylsulfonyl-phenyl)-amino-s-triazin-2'-yl]-amino-6-sulfo-8-naphthol | rot |
| 367 | Sulfo | 1-(4'-Chlor-6'-methoxy-s-triazin-2'-yl)-amino-3,6-disulfo-8-naphthol | rot |
| 368 | Sulfo | 1-(4'-Chlor-6'-methoxy-s-triazin-2'-yl)-amino-4,6-disulfo-8-naphthol | rot |
| 369 | Sulfo | 6-Carboxy-2-naphthol | orange |
| 370 | Sulfo | 2-(4'-Chlor-6'-methoxy-s-triazin-2'-yl)-amino-6-sulfo-8-naphthol | rot |
| 371 | Sulfo | 3-(4'-Chlor-6'-methoxy-s-triazin-2'-yl)-amino-6-sulfo-8-naphthol | rot |
| 372 | Sulfo | 1-[4'-Fluor-6'-(4"-ß-sulfato-ethylsulfonyl-phenyl)-amino-s-triazin-2'-yl]-amino-3,6-disulfo-8-naphthol | rot |
| 373 | Sulfo | 1-[4'-Fluor-6'-(4"-ß-sulfato-ethylsulfonyl-phenyl)-amino-s-triazin-2'-yl]-amino-4,6-disulfo-8-naphthol | rot |

| Bsp. | Rest R° | Komponente H-K | Farbton |
|---|---|---|---|
| 374 | H | 2-[4'-Fluor-6'-(4"-ß-sulfato-ethylsulfonyl-phenyl)-amino-s-triazin-2'-yl]-amino-6-sulfo-8-naphthol | rot |
| 375 | H | 3-[4'-Fluor-6'-(4"-ß-sulfato-ethylsulfonyl-phenyl)-amino-s-triazin-2'-yl]-amino-6-sulfo-8-naphthol | rot |
| 376 | H | 1-(4'-ß-Sulfatoethylsulfonyl-benzoyl)-amino-3,6-disulfo-8-naphthol | rot |
| 377 | H | 1-(4'-ß-Sulfatoethylsulfonyl-benzoyl)-amino-4,6-disulfo-8-naphthol | rot |
| 378 | H | 2-(4'-ß-Sulfatoethylsulfonyl-benzoyl)-amino-6-sulfo-8-naphthol | rot |
| 379 | H | 3-(4'-ß-Sulfatoethylsulfonyl-benzoyl)-amino-6-sulfo-8-naphthol | rot |
| 380 | Sulfo | 1-{ß-[4'-Chlor-6'-(4"-ß'-sulfatoethylsulfonyl-phenyl)-ethyl]-amino-s-triazin-2'-yl}-amino-3,6-disulfo-8-naphthol | rot |
| 381 | Sulfo | 1-{ß-[4'-Chlor-6'-(4"-ß'-sulfatoethylsulfonyl-phenyl)-ethyl]-amino-s-triazin-2'-yl}-amino-4,6-disulfo-8-naphthol | rot |
| 382 | Sulfo | 1-[4'-Chlor-6'-(3"-ß-sulfato-ethylsulfonyl-phenyl)-amino-s-triazin-2'-yl]-amino-3,6-disulfo-8-naphthol | rot |
| 383 | Sulfo | 1-[4'-Chlor-6'-(3"-ß-sulfato-ethylsulfonyl-phenyl)-amino-s-triazin-2'-yl]-amino-4,6-disulfo-8-naphthol | rot |
| 384 | H | 1-[4'-Chlor-6'-(4"-sulfophenyl)-amino-s-triazin-2'-yl]-amino-3,6-disulfo-8-naphthol | rot |
| 385 | H | 1-[4'-Chlor-6'-(4"-sulfophenyl)-amino-s-triazin-2'-yl]-amino-4,6-disulfo-8-naphthol | rot |

| Bsp. | Rest $R^o$ | Komponente H-K | Farbton |
|---|---|---|---|
| 386 | Sulfo | 1-[4'-Chlor-6'-(2",5"-disulfo-phenyl)-amino-s-triazin-2'-yl]-amino-4,6-disulfo-8-naphthol | rot |
| 387 | Sulfo | 1-[4'-Fluor-6'-(3",5"-disulfo-phenyl)-amino-s-triazin-2'-yl]-amino-3,6-disulfo-8-naphthol | rot |
| 388 | Sulfo | 1-[4'-Fluor-6'-(3",5"-disulfo-phenyl)-amino-s-triazin-2'-yl]-amino-4,6-disulfo-8-naphthol | rot |
| 389 | Sulfo | 6-Sulfo-2-naphthol | rot |
| 390 | Sulfo | 8-Sulfo-2-naphthol | rot |
| 391 | Sulfo | 1-Acetylamino-3,6-disulfo-8-naphthol | blaustichig rot |
| 392 | Sulfo | 1-Phenylureido-4,6-disulfo-8-naphthol | blaustichig rot |
| 393 | H | 3,6,8-Trisulfo-1-naphthol | rot |
| 394 | H | 3,6-Disulfo-2-naphthol | rot |
| 395 | H | 6,8-Disulfo-2-naphthol | orange |
| 396 | H | N-Acetoacetyl-3-sulfo-anilin | gelb |
| 397 | H | 1-(4'-Sulfophenyl)-3-carbethoxy-5-pyrazolon | gelb |
| 398 | Sulfo | 5-Sulfo-1,4-dimethyl-2-hydroxy-6-pyridon | gelb |
| 399 | Sulfo | 5-Sulfo-8-hydroxy-chinolin | orange |
| 400 | H | N-Acetoacetyl-2-carboxy-anilin | gelb |
| 401 | H | N-Ethyl-N-(3'-sulfo-benzyl)-anilin | orange |
| 402 | Sulfo | N,N-Bis-(ß-hydroxyethyl)-anilin | orange |
| 403 | Sulfo | 2-Sulfo-5-acetylamino-anilin | goldgelb |
| 404 | H | 1-(2'-Chlor-5'-sulfo-phenyl)-3-methyl-5-pyrazolon | gelb |
| 405 | H | 1-(2',5'-Disulfo-phenyl)-3-methyl-5-pyrazolon | gelb |
| 406 | Sulfo | 1-(4',8'-Disulfo-naphth-2'-yl)-3-methyl-5-pyrazolon | gelb |
| 407 | Sulfo | 1-(4'-Sulfophenyl)-3-methyl-5-amino-pyrazol | gelb |

**Beispiele 408 bis 475**

In den nachfolgenden Tabellenbeispielen sind weitere erfindungsgemäße Azoverbindungen entsprechend der, in Form der freien Säure geschriebenen, allgemeinen Formel (E)

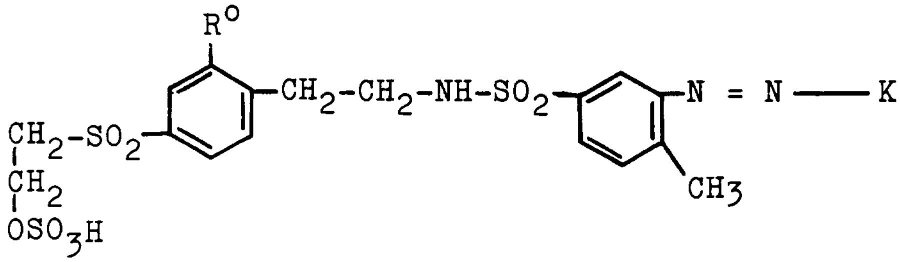

(E)

mit Hilfe ihrer Kupplungskomponente H-K beschrieben. Sie lassen sich in erfindungsgemäßer Weise, beispielsweise gemäß den obigen Ausführungsbeispielen durch Kupplung der angegebenen Kupplungskomponente mit dem Diazoniumsalz der aus Formel (E) ersichtlichen aromatischen Aminoverbindung entsprechend der allgemeinen Formel (5) herstellen und besitzen ebenfalls sehr gute faserreaktive Farbstoffeigenschaften. Sie liefern farbstarke Färbungen und Drucke auf den in der Beschreibung genannten Materialien, wie insbesondere Cellulosefasermaterialien, nach den für faserreaktive Farbstoffe üblichen Applikations- und Fixiermethoden mit den in dem jeweiligen Tabellenbeispiel angegebenen Farbton (hier auf Baumwolle) mit guten Echtheitseigenschaften; die in Klammern angegebenen Werte sind die Absorptionsmaxima ($\lambda_{max}$) in nm.

| Bsp. | Rest R° | Komponente H-K | Farbton |
|---|---|---|---|
| 408 | H | 2-Acetylamino-6-sulfo-8-naphthol | rotorange |
| 409 | H | 4-Sulfo-1-naphthol | rotorange |
| 410 | Sulfo | 1-Benzoylamino-3,6-disulfo-8-naphthol | rot |
| 411 | Sulfo | 1-Benzoylamino-4,6-disulfo-8-naphthol | rot |
| 412 | Sulfo | 3-Sulfo-1-naphthol | rotorange |
| 413 | H | 3,6-Disulfo-1-naphthol | rotorange |
| 414 | H | 1-(4'-Sulfophenyl)-3-carboxy-5-pyrazolon | gelb |
| 415 | Sulfo | 1-(4'-Sulfophenyl)-3-methyl-5-pyrazolon | gelb |
| 416 | Sulfo | 5-Sulfo-1-naphthol | rotorange |
| 417 | H | N-Acetoacetyl-2-methoxy-5-methyl-4-(ß-sulfato-ethylsulfonyl)-anilin | gelb |
| 418 | Sulfo | N-Acetoacetyl-2-methoxy-5-(ß-sulfatoethylsulfonyl)-anilin | gelb |
| 419 | H | N-Acetoacetyl-2,5-dimethoxy-4-(ß-sulfatoethylsulfonyl)-anilin | gelb |
| 420 | H | N-Acetoacetyl-3-methoxy-4-(ß-sulfatoethylsulfonyl)-anilin | gelb |
| 421 | H | N-Acetoacetyl-4-methoxy-3-(ß-sulfatoethylsulfonyl)-anilin | gelb |
| 422 | Sulfo | 1-(4'-ß-Sulfatoethylsulfonyl-phenyl)-3-methyl-5-pyrazolon | gelb (390) |
| 423 | Sulfo | 1-(4'-ß-Sulfatoethylsulfonyl-phenyl)-3-carbethoxy-5-pyrazolon | gelb |
| 424 | H | 3-Acetylamino-6-sulfo-8-naphthol | rotorange |
| 425 | H | N,N-Bis-(ß-sulfatoethyl)-anilin | gelb |
| 426 | H | N-Ethyl-N-(ß-sulfatoethyl)-anilin | gelb |
| 427 | H | N,N-Bis-(ß-sulfatoethyl)-3-chlor-anilin | gelb |
| 428 | H | 1-(N-ß-Sulfatoethyl)-4-methyl-2-hydroxy-6-pyridon | gelb |

| Bsp. | Rest $R^o$ | Komponente H-K | Farbton |
|---|---|---|---|
| 429 | H | 1-Acetylamino-3,6-disulfo-8-naphthol | rot |
| 430 | H | 1-Acetylamino-4,6-disulfo-8-naphthol | rot |
| 431 | H | 1-[4'-Chlor-6'-(4"-ß-sulfatoethylsulfonyl-phenyl)-amino-s-triazin-2'-yl]-amino-3,6-disulfo-8-naphthol | rot |
| 432 | H | 1-[4'-Chlor-6'-(4"-ß-sulfatoethylsulfonyl-phenyl)-amino-s-triazin-2'-yl]-amino-4,6-disulfo-8-naphthol | rot |
| 433 | H | 2-[4'-Chlor-6'-(4"-ß-sulfatoethylsulfonyl-phenyl)-amino-s-triazin-2'-yl]-amino-6-sulfo-8-naphthol | rot |
| 434 | H | 3-[4'-Chlor-6'-(4"-ß-sulfatoethylsulfonyl-phenyl)-amino-s-triazin-2'-yl]-amino-6-sulfo-8-naphthol | rot |
| 435 | H | 1-(4'-Chlor-6'-methoxy-s-triazin-2'-yl)-amino-3,6-disulfo-8-naphthol | rot |
| 436 | H | 1-(4'-Chlor-6'-methoxy-s-triazin-2'-yl)-amino-4,6-disulfo-8-naphthol | rot |
| 437 | Sulfo | 2-(4'-Chlor-6'-methoxy-s-triazin-2'-yl)-amino-6-sulfo-8-naphthol | rot |
| 438 | Sulfo | 3-(4'-Chlor-6'-methoxy-s-triazin-2'-yl)-amino-6-sulfo-8-naphthol | rot |
| 439 | Sulfo | 1-[4'-Fluor-6'-(4"-ß-sulfatoethylsulfonyl-phenyl)-amino-s-triazin-2'-yl]-amino-3,6-disulfo-8-naphthol | rot |
| 440 | Sulfo | 1-[4'-Fluor-6'-(4"-ß-sulfatoethylsulfonyl-phenyl)-amino-s-triazin-2'-yl]-amino-4,6-disulfo-8-naphthol | rot |
| 441 | Sulfo | 2-[4'-Fluor-6'-(4"-ß-sulfatoethylsulfonyl-phenyl)-amino-s-triazin-2'-yl]-amino-6-sulfo-8-naphthol | rot |

| Bsp. | Rest R° | Komponente H-K | Farbton |
| --- | --- | --- | --- |
| 442 | H | 3-[4'-Fluor-6'-(4"-ß-sulfato-ethylsulfonyl-phenyl)-amino-s-triazin-2'-yl]-amino-6-sulfo-8-naphthol | rot |
| 443 | Sulfo | 1-(4'-ß-Sulfatoethylsulfonyl-benzoyl)-amino-3,6-disulfo-8-naphthol | rot |
| 444 | Sulfo | 1-(4'-ß-Sulfatoethylsulfonyl-benzoyl)-amino-4,6-disulfo-8-naphthol | rot |
| 445 | Sulfo | 2-(4'-ß-Sulfatoethylsulfonyl-benzoyl)-amino-6-sulfo-8-naphthol | rot |
| 446 | Sulfo | 3-(4'-ß-Sulfatoethylsulfonyl-benzoyl)-amino-6-sulfo-8-naphthol | rot |
| 447 | H | 1-{ß-[4'-Chlor-6'-(4"-ß'-sulfatoethylsulfonyl-phenyl)-ethyl]-amino-s-triazin-2'-yl}-amino-3,6-disulfo-8-naphthol | rot |
| 448 | H | 1-{ß-[4'-Chlor-6'-(4"-ß'-sulfatoethylsulfonyl-phenyl)-ethyl]-amino-s-triazin-2'-yl}-amino-4,6-disulfo-8-naphthol | rot |
| 449 | H | 1-[4'-Chlor-6'-(3"-ß-sulfato-ethylsulfonyl-phenyl)-amino-s-triazin-2'-yl]-amino-3,6-disulfo-8-naphthol | rot |
| 450 | H | 1-[4'-Chlor-6'-(3"-ß-sulfato-ethylsulfonyl-phenyl)-amino-s-triazin-2'-yl]-amino-4,6-disulfo-8-naphthol | rot |
| 451 | H | 1-[4'-Chlor-6'-(4"-sulfophenyl)-amino-s-triazin-2'-yl]-amino-3,6-disulfo-8-naphthol | rot |
| 452 | H | 1-[4'-Chlor-6'-(4"-sulfophenyl)-amino-s-triazin-2'-yl]-amino-4,6-disulfo-8-naphthol | rot |
| 453 | Sulfo | 1-[4'-Chlor-6'-(2",5"-disulfo-phenyl)-amino-s-triazin-2'-yl]-amino-4,6-disulfo-8-naphthol | rot |

| Bsp. | Rest $R^o$ | Komponente H-K | Farbton |
|---|---|---|---|
| 454 | Sulfo | 1-[4'-Fluor-6'-(3",5"-disulfo-phenyl)-amino-s-triazin-2'-yl]-amino-3,6-disulfo-8-naphthol | rot |
| 455 | Sulfo | 1-[4'-Fluor-6'-(3",5"-disulfo-phenyl)-amino-s-triazin-2'-yl]-amino-4,6-disulfo-8-naphthol | rot |
| 456 | Sulfo | 6-Sulfo-2-naphthol | rot |
| 457 | Sulfo | 8-Sulfo-2-naphthol | rot |
| 458 | H | 1-Acetylamino-3,6-disulfo-8-naphthol | blaustichig rot |
| 459 | H | 1-Phenylureido-4,6-disulfo-8-naphthol | blaustichig rot |
| 460 | H | 3,6,8-Trisulfo-1-naphthol | rot |
| 461 | H | 3,6-Disulfo-2-naphthol | rot |
| 462 | H | 6,8-Disulfo-2-naphthol | orange |
| 463 | H | N-Acetoacetyl-3-sulfo-anilin | gelb |
| 464 | H | 1-(4'-Sulfophenyl)-3-carbethoxy-5-pyrazolon | gelb |
| 465 | H | 5-Sulfo-1,4-dimethyl-2-hydroxy-6-pyridon | gelb |
| 466 | H | 5-Sulfo-8-hydroxy-chinolin | orange |
| 467 | H | N-Acetoacetyl-2-carboxy-anilin | gelb |
| 468 | Sulfo | N-Ethyl-N-(3'-sulfo-benzyl)-anilin | orange |
| 469 | H | N,N-Bis-(ß-hydroxyethyl)-anilin | orange |
| 470 | H | 2-Sulfo-5-acetylamino-anilin | goldgelb |
| 471 | H | 3-Acetylamino-1-N-[4'-chlor-6'-(3"-ß-sulfatoethylsulfonyl-phenyl)-amino-s-triazin-2'-yl]-anilin | gelb |
| 472 | Sulfo | 1-(2'-Chlor-5'-sulfo-phenyl)-3-methyl-5-pyrazolon | gelb |
| 473 | Sulfo | 1-(2',5'-Disulfo-phenyl)-3-methyl-5-pyrazolon | gelb |
| 474 | Sulfo | 1-(4',8'-Disulfo-naphth-2'-yl)-3-methyl-5-pyrazolon | gelb |
| 475 | Sulfo | 1-(4'-Sulfophenyl)-3-methyl-5-amino-pyrazol | gelb |

**Beispiele 476 bis 542**

In den nachfolgenden Tabellenbeispielen sind weitere erfindungsgemäße Azoverbindungen entsprechend der, in Form der freien Säure geschriebenen, allgemeinen Formel (F)

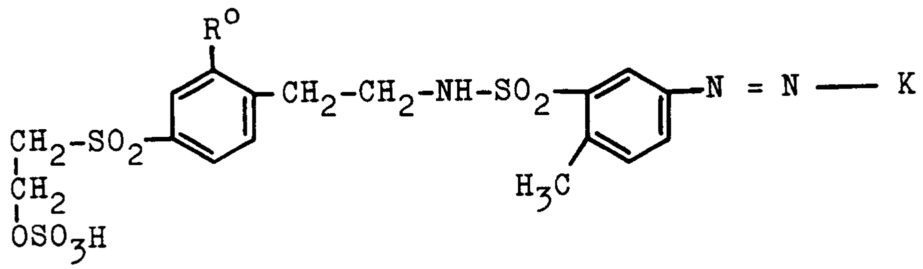

(F)

mit Hilfe ihrer Kupplungskomponente H-K beschrieben. Sie lassen sich in erfindungsgemäßer Weise, beispielsweise gemäß den obigen Ausführungsbeispielen durch Kupplung der angegebenen Kupplungskomponente mit dem Diazoniumsalz der aus Formel (F) ersichtlichen aromatischen Aminoverbindung entsprechend der allgemeinen Formel (5) herstellen und besitzen ebenfalls sehr gute faserreaktive Farbstoffeigenschaften. Sie liefern farbstarke Färbungen und Drucke auf den in der Beschreibung genannten Materialien, wie insbesondere Cellulosefasermaterialien, nach den für faserreaktive Farbstoffe üblichen Applikations- und Fixiermethoden mit den in dem jeweiligen Tabellenbeispiel angegebenen Farbton (hier auf Baumwolle) mit guten Echtheitseigenschaften; die in Klammern angegebenen Werte sind die Absorptionsmaxima ($\lambda_{max}$) in nm.

| Bsp. | Rest $R^o$ | Komponente H-K | Farbton |
|---|---|---|---|
| 476 | H | 2-Acetylamino-6-sulfo-8-naphthol | rotorange |
| 477 | H | 4-Sulfo-1-naphthol | rotorange |
| 478 | H | 1-Benzoylamino-3,6-disulfo-8-naphthol | rot |
| 479 | H | 1-Benzoylamino-4,6-disulfo-8-naphthol | rot |
| 480 | H | 3-Sulfo-1-naphthol | rotorange |
| 481 | H | 3,6-Disulfo-1-naphthol | rotorange (490) |
| 482 | H | 1-(4'-Sulfophenyl)-3-carboxy-5-pyrazolon | gelb |
| 483 | H | 1-(4'-Sulfophenyl)-3-methyl-5-pyrazolon | gelb |
| 484 | H | 5-Sulfo-1-naphthol | rotorange |
| 485 | H | N-Acetoacetyl-2-methoxy-5-methyl-4-(ß-sulfato-ethylsulfonyl)-anilin | gelb |
| 486 | H | N-Acetoacetyl-2-methoxy-5-(ß-sulfatoethylsulfonyl)-anilin | gelb |
| 487 | Sulfo | N-Acetoacetyl-2,5-dimethoxy-4-(ß-sulfatoethylsulfonyl)-anilin | gelb |
| 488 | Sulfo | N-Acetoacetyl-3-methoxy-4-(ß-sulfatoethylsulfonyl)-anilin | gelb |
| 489 | Sulfo | N-Acetoacetyl-4-methoxy-3-(ß-sulfatoethylsulfonyl)-anilin | gelb |
| 490 | H | 1-(4'-ß-Sulfatoethylsulfonyl-phenyl)-3-methyl-5-pyrazolon | gelb |
| 491 | H | 1-(4'-ß-Sulfatoethylsulfonyl-phenyl)-3-carbethoxy-5-pyrazolon | gelb |
| 492 | Sulfo | 3-Acetylamino-6-sulfo-8-naphthol | rotorange |
| 493 | Sulfo | N,N-Bis-(ß-sulfatoethyl)-anilin | gelb |
| 494 | Sulfo | N-Ethyl-N-(ß-sulfatoethyl)-anilin | gelb |
| 495 | Sulfo | N,N-Bis-(ß-sulfatoethyl)-3-chlor-anilin | gelb |
| 496 | Sulfo | 1-(N-ß-Sulfatoethyl)-4-methyl-2-hydroxy-6-pyridon | gelb |

| Bsp. | Rest R° | Komponente H-K | Farbton |
|---|---|---|---|
| 497 | Sulfo | 1-Acetylamino-3,6-disulfo-8-naphthol | rot |
| 498 | Sulfo | 1-Acetylamino-4,6-disulfo-8-naphthol | rot |
| 499 | Sulfo | 1-[4'-Chlor-6'-(4"-ß-sulfato-ethylsulfonyl-phenyl)-amino-s-triazin-2'-yl]-amino-3,6-disulfo-8-naphthol | rot |
| 500 | Sulfo | 1-[4'-Chlor-6'-(4"-ß-sulfato-ethylsulfonyl-phenyl)-amino-s-triazin-2'-yl]-amino-4,6-disulfo-8-naphthol | rot |
| 501 | Sulfo | 2-[4'-Chlor-6'-(4"-ß-sulfato-ethylsulfonyl-phenyl)-amino-s-triazin-2'-yl]-amino-6-sulfo-8-naphthol | rot |
| 502 | Sulfo | 3-[4'-Chlor-6'-(4"-ß-sulfato-ethylsulfonyl-phenyl)-amino-s-triazin-2'-yl]-amino-6-sulfo-8-naphthol | rot |
| 503 | Sulfo | 1-(4'-Chlor-6'-methoxy-s-triazin-2'-yl)-amino-3,6-disulfo-8-naphthol | rot |
| 504 | Sulfo | 1-(4'-Chlor-6'-methoxy-s-triazin-2'-yl)-amino-4,6-disulfo-8-naphthol | rot |
| 505 | Sulfo | 2-(4'-Chlor-6'-methoxy-s-triazin-2'-yl)-amino-6-sulfo-8-naphthol | rot |
| 506 | Sulfo | 3-(4'-Chlor-6'-methoxy-s-triazin-2'-yl)-amino-6-sulfo-8-naphthol | rot |
| 507 | Sulfo | 1-[4'-Fluor-6'-(4"-ß-sulfato-ethylsulfonyl-phenyl)-amino-s-triazin-2'-yl]-amino-3,6-disulfo-8-naphthol | rot |
| 508 | Sulfo | 1-[4'-Fluor-6'-(4"-ß-sulfato-ethylsulfonyl-phenyl)-amino-s-triazin-2'-yl]-amino-4,6-disulfo-8-naphthol | rot |
| 509 | Sulfo | 2-[4'-Fluor-6'-(4"-ß-sulfato-ethylsulfonyl-phenyl)-amino-s-triazin-2'-yl]-amino-6-sulfo-8-naphthol | rot |

| Bsp. | Rest R° | Komponente H-K | Farbton |
|---|---|---|---|
| 510 | Sulfo | 3-[4'-Fluor-6'-(4"-ß-sulfato-ethylsulfonyl-phenyl)-amino-s-triazin-2'-yl]-amino-6-sulfo-8-naphthol | rot |
| 511 | Sulfo | 1-(4'-ß-Sulfatoethylsulfonyl-benzoyl)-amino-3,6-disulfo-8-naphthol | rot |
| 512 | Sulfo | 1-(4'-ß-Sulfatoethylsulfonyl-benzoyl)-amino-4,6-disulfo-8-naphthol | rot |
| 513 | Sulfo | 2-(4'-ß-Sulfatoethylsulfonyl-benzoyl)-amino-6-sulfo-8-naphthol | rot |
| 514 | Sulfo | 3-(4'-ß-Sulfatoethylsulfonyl-benzoyl)-amino-6-sulfo-8-naphthol | rot |
| 515 | Sulfo | 1-{ß-[4'-Chlor-6'-(4"-ß'-sulfatoethylsulfonyl-phenyl)-ethyl]-amino-s-triazin-2'-yl}-amino-3,6-disulfo-8-naphthol | rot |
| 516 | Sulfo | 1-{ß-[4'-Chlor-6'-(4"-ß'-sulfatoethylsulfonyl-phenyl)-ethyl]-amino-s-triazin-2'-yl}-amino-4,6-disulfo-8-naphthol | rot |
| 517 | Sulfo | 1-[4'-Chlor-6'-(3"-ß-sulfato-ethylsulfonyl-phenyl)-amino-s-triazin-2'-yl]-amino-3,6-disulfo-8-naphthol | rot |
| 518 | Sulfo | 1-[4'-Chlor-6'-(3"-ß-sulfato-ethylsulfonyl-phenyl)-amino-s-triazin-2'-yl]-amino-4,6-disulfo-8-naphthol | rot |
| 519 | Sulfo | 1-[4'-Chlor-6'-(4"-sulfophenyl)-amino-s-triazin-2'-yl]-amino-3,6-disulfo-8-naphthol | rot |
| 520 | Sulfo | 1-[4'-Chlor-6'-(4"-sulfophenyl)-amino-s-triazin-2'-yl]-amino-4,6-disulfo-8-naphthol | rot |
| 521 | Sulfo | 1-[4'-Chlor-6'-(2",5"-disulfo-phenyl)-amino-s-triazin-2'-yl]-amino-4,6-disulfo-8-naphthol | rot |

| Bsp. | Rest $R^O$ | Komponente H-K | Farbton |
|---|---|---|---|
| 522 | Sulfo | 1-[4'-Fluor-6'-(3",5"-disulfo-phenyl)-amino-s-triazin-2'-yl]-amino-3,6-disulfo-8-naphthol | rot |
| 523 | Sulfo | 1-[4'-Fluor-6'-(3",5"-disulfo-phenyl)-amino-s-triazin-2'-yl]-amino-4,6-disulfo-8-naphthol | rot |
| 524 | Sulfo | 6-Sulfo-2-naphthol | rot |
| 525 | Sulfo | 8-Sulfo-2-naphthol | rot |
| 526 | H | 1-Acetylamino-3,6-disulfo-8-naphthol | blaustichig rot |
| 527 | H | 1-Phenylureido-4,6-disulfo-8-naphthol | blaustichig rot |
| 528 | H | 3,6,8-Trisulfo-1-naphthol | rot |
| 529 | H | 3,6-Disulfo-2-naphthol | rot |
| 530 | H | 6,8-Disulfo-2-naphthol | orange |
| 531 | H | N-Acetoacetyl-3-sulfo-anilin | gelb |
| 532 | H | 1-(4'-Sulfophenyl)-3-carbethoxy-5-pyrazolon | gelb |
| 533 | H | 5-Sulfo-1,4-dimethyl-2-hydroxy-6-pyridon | gelb |
| 534 | H | 5-Sulfo-8-hydroxy-chinolin | orange |
| 535 | H | N-Acetoacetyl-2-carboxy-anilin | gelb |
| 536 | Sulfo | N-Ethyl-N-(3'-sulfo-benzyl)-anilin | orange |
| 537 | Sulfo | N,N-Bis-(ß-hydroxyethyl)-anilin | orange |
| 538 | Sulfo | 2-Sulfo-5-acetylamino-anilin | goldgelb |
| 539 | H | 1-(2'-Chlor-5'-phenyl)-3-methyl-5-pyrazolon | gelb |
| 540 | H | 1-(2',5'-Disulfo-phenyl)-3-methyl-5-pyrazolon | gelb |
| 541 | H | 1-(4',8'-Disulfo-naphth-2'-yl)-3-methyl-5-pyrazolon | gelb |
| 542 | H | 1-(4'-Sulfophenyl)-3-methyl-5-amino-pyrazol | gelb |

**Beispiele 543 bis 608**

In den nachfolgenden Tabellenbeispielen sind weitere erfindungsgemäße Azoverbindungen entsprechend der, in Form der freien Säure geschriebenen, allgemeinen Formel (G)

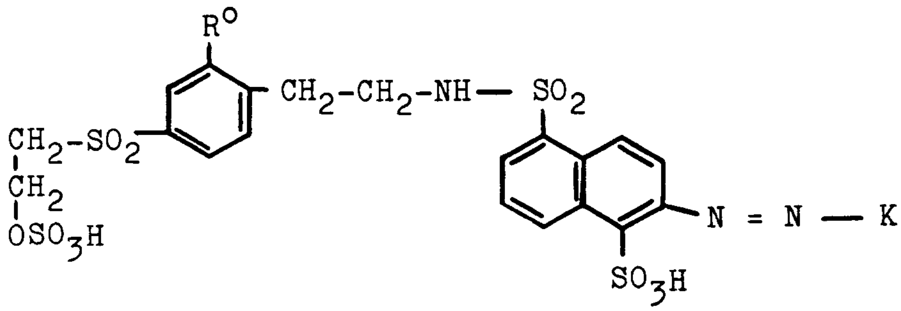

(G)

mit Hilfe ihrer Kupplungskomponente H-K beschrieben. Sie lassen sich in erfindungsgemäßer Weise, beispielsweise gemäß den obigen Ausführungsbeispielen durch Kupplung der angegebenen Kupplungskomponente mit dem Diazoniumsalz der aus Formel (G) ersichtlichen aromatischen Aminoverbindung entsprechend der allgemeinen Formel (5) herstellen und besitzen ebenfalls sehr gute faserreaktive Farbstoffeigenschaften. Sie liefern farbstarke Färbungen und Drucke auf den in der Beschreibung genannten Materialien, wie insbesondere Cellulosefasermaterialien, nach den für faserreaktive Farbstoffe üblichen Applikations- und Fixiermethoden mit den in dem jeweiligen Tabellenbeispiel angegebenen Farbton (hier auf Baumwolle) mit guten Echtheitseigenschaften; die in Klammern angegebenen Werte sind die Absorptionsmaxima ($\lambda_{max}$) in nm.

| Bsp. | Rest $R^o$ | Komponente H-K | Farbton |
|---|---|---|---|
| 543 | H | 2-Acetylamino-6-sulfo-8-naphthol | rotorange (495) |
| 544 | H | 4-Sulfo-1-naphthol | rotorange |
| 545 | H | 1-Benzoylamino-3,6-disulfo-8-naphthol | rot (529) |
| 546 | H | 1-Benzoylamino-4,6-disulfo-8-naphthol | rot (524) |
| 547 | H | 3-Sulfo-1-naphthol | rotorange |
| 548 | H | 3,6-Disulfo-1-naphthol | rotorange (492) |
| 549 | H | 5-Sulfo-1-naphthol | rotorange |
| 550 | H | 1-(4'-Sulfophenyl)-3-methyl-5-pyrazolon | gelb |
| 551 | Sulfo | N-Acetoacetyl-2-methoxy-5-methyl-4-(ß-sulfatoethylsulfonyl)-anilin | gelb |
| 552 | Sulfo | N-Acetoacetyl-2-methoxy-5-(ß-sulfatoethylsulfonyl)-anilin | gelb |
| 553 | H | N-Acetoacetyl-2,5-dimethoxy-4-(ß-sulfatoethylsulfonyl)-anilin | gelb |
| 554 | H | N-Acetoacetyl-3-methoxy-4-(ß-sulfatoethylsulfonyl)-anilin | gelb |
| 555 | H | N-Acetoacetyl-4-methoxy-3-(ß-sulfatoethylsulfonyl)-anilin | gelb |
| 556 | Sulfo | 1-(4'-ß-Sulfatoethylsulfonyl-phenyl)-3-methyl-5-pyrazolon | gelb |
| 557 | H | 1-(4'-ß-Sulfatoethylsulfonyl-phenyl)-3-carbethoxy-5-pyrazolon | gelb |
| 558 | H | 3-Acetylamino-6-sulfo-8-naphthol | rotorange |
| 559 | Sulfo | N,N-Bis-(ß-sulfatoethyl)-anilin | gelb |
| 560 | Sulfo | N-Ethyl-N-(ß-sulfatoethyl)-anilin | gelb |
| 561 | Sulfo | N,N-Bis-(ß-sulfatoethyl)-3-chlor-anilin | gelb |
| 562 | Sulfo | 1-(N-ß-Sulfatoethyl)-4-methyl-2-hydroxy-6-pyridon | gelb |

| Bsp. | Rest R° | Komponente H-K | Farbton |
|---|---|---|---|
| 563 | H | 1-Acetylamino-3,6-disulfo-8-naphthol | rot (520) |
| 564 | H | 1-Acetylamino-4,6-disulfo-8-naphthol | rot |
| 565 | Sulfo | 1-[4'-Chlor-6'-(4"-ß-sulfato-ethylsulfonyl-phenyl)-amino-s-triazin-2'-yl]-amino-3,6-disulfo-8-naphthol | rot |
| 566 | H | 1-[4'-Chlor-6'-(4"-ß-sulfato-ethylsulfonyl-phenyl)-amino-s-triazin-2'-yl]-amino-4,6-disulfo-8-naphthol | rot |
| 567 | Sulfo | 2-[4'-Chlor-6'-(4"-ß-sulfato-ethylsulfonyl-phenyl)-amino-s-triazin-2'-yl]-amino-6-sulfo-8-naphthol | rot |
| 568 | Sulfo | 3-[4'-Chlor-6'-(4"-ß-sulfato-ethylsulfonyl-phenyl)-amino-s-triazin-2'-yl]-amino-6-sulfo-8-naphthol | rot |
| 569 | H | 1-(4'-Chlor-6'-methoxy-s-triazin-2'-yl)-amino-3,6-disulfo-8-naphthol | rot |
| 570 | H | 1-(4'-Chlor-6'-methoxy-s-triazin-2'-yl)-amino-4,6-disulfo-8-naphthol | rot |
| 571 | H | 2-(4'-Chlor-6'-methoxy-s-triazin-2'-yl)-amino-6-sulfo-8-naphthol | rot |
| 572 | H | 3-(4'-Chlor-6'-methoxy-s-triazin-2'-yl)-amino-6-sulfo-8-naphthol | rot |
| 573 | H | 1-[4'-Fluor-6'-(4"-ß-sulfato-ethylsulfonyl-phenyl)-amino-s-triazin-2'-yl]-amino-3,6-disulfo-8-naphthol | rot |
| 574 | H | 1-[4'-Fluor-6'-(4"-ß-sulfato-ethylsulfonyl-phenyl)-amino-s-triazin-2'-yl]-amino-4,6-disulfo-8-naphthol | rot |
| 575 | H | 2-[4'-Fluor-6'-(4"-ß-sulfato-ethylsulfonyl-phenyl)-amino-s-triazin-2'-yl]-amino-6-sulfo-8-naphthol | rot |

| Bsp. | Rest Rº | Komponente H-K | Farbton |
|---|---|---|---|
| 576 | H | 3-[4'-Fluor-6'-(4"-ß-sulfato-ethylsulfonyl-phenyl)-amino-s-triazin-2'-yl]-amino-6-sulfo-8-naphthol | rot |
| 577 | H | 1-(4'-ß-Sulfatoethylsulfonyl-benzoyl)-amino-3,6-disulfo-8-naphthol | rot |
| 578 | H | 1-(4'-ß-Sulfatoethylsulfonyl-benzoyl)-amino-4,6-disulfo-8-naphthol | rot |
| 579 | H | 2-(4'-ß-Sulfatoethylsulfonyl-benzoyl)-amino-6-sulfo-8-naphthol | rot |
| 580 | H | 3-(4'-ß-Sulfatoethylsulfonyl-benzoyl)-amino-6-sulfo-8-naphthol | rot |
| 581 | H | 1-{ß-[4'-Chlor-6'-(4"-ß'-sulfatoethylsulfonyl-phenyl)-ethyl]-amino-s-triazin-2'-yl}-amino-3,6-disulfo-8-naphthol | rot |
| 582 | H | 1-{ß-[4'-Chlor-6'-(4"-ß'-sulfatoethylsulfonyl-phenyl)-ethyl]-amino-s-triazin-2'-yl}-amino-4,6-disulfo-8-naphthol | rot |
| 583 | H | 1-[4'-Chlor-6'-(3"-ß-sulfato-ethylsulfonyl-phenyl)-amino-s-triazin-2'-yl]-amino-3,6-disulfo-8-naphthol | rot |
| 584 | H | 1-[4'-Chlor-6'-(3"-ß-sulfato-ethylsulfonyl-phenyl)-amino-s-triazin-2'-yl]-amino-4,6-disulfo-8-naphthol | rot |
| 585 | H | 1-[4'-Chlor-6'-(4"-sulfophenyl)-amino-s-triazin-2'-yl]-amino-3,6-disulfo-8-naphthol | rot |
| 586 | H | 1-[4'-Chlor-6'-(4"-sulfophenyl)-amino-s-triazin-2'-yl]-amino-4,6-disulfo-8-naphthol | rot |
| 587 | H | 1-[4'-Chlor-6'-(2",5"-disulfo-phenyl)-amino-s-triazin-2'-yl]-amino-4,6-disulfo-8-naphthol | rot |

| Bsp. | Rest $R^O$ | Komponente H-K | Farbton |
|---|---|---|---|
| 588 | H | 1-[4'-Fluor-6'-(3",5"-disulfo-phenyl)-amino-s-triazin-2'-yl]-amino-3,6-disulfo-8-naphthol | rot |
| 589 | H | 1-[4'-Fluor-6'-(3",5"-disulfo-phenyl)-amino-s-triazin-2'-yl]-amino-4,6-disulfo-8-naphthol | rot |
| 590 | H | 6-Sulfo-2-naphthol | rot |
| 591 | H | 8-Sulfo-2-naphthol | rot |
| 592 | H | 1-Acetylamino-3,6-disulfo-8-naphthol | blaustichig rot |
| 593 | H | 1-Phenylureido-4,6-disulfo-8-naphthol | blaustichig rot |
| 594 | H | 3,6,8-Trisulfo-1-naphthol | rot |
| 595 | H | 3,6-Disulfo-2-naphthol | rot |
| 596 | H | 6,8-Disulfo-2-naphthol | orange |
| 597 | H | N-Acetoacetyl-3-sulfo-anilin | gelb |
| 598 | H | 1-(4'-Sulfophenyl)-3-carbethoxy-5-pyrazolon | gelb |
| 599 | Sulfo | 5-Sulfo-1,4-dimethyl-2-hydroxy-6-pyridon | gelb |
| 600 | Sulfo | 5-Sulfo-8-hydroxy-chinolin | orange |
| 601 | H | N-Acetoacetyl-2-carboxy-anilin | gelb |
| 602 | H | N-Ethyl-N-(3'-sulfo-benzyl)-anilin | orange |
| 603 | H | N,N-Bis-(ß-hydroxyethyl)-anilin | orange |
| 604 | H | 2-Sulfo-5-acetylamino-anilin | goldgelb |
| 605 | H | 1-(2'-Chlor-5'-sulfo-phenyl)-3-methyl-5-pyrazolon | gelb |
| 606 | H | 1-(2',5'-Disulfo-phenyl)-3-methyl-5-pyrazolon | gelb |
| 607 | H | 1-(4',8'-Disulfo-naphth-2'-yl)-3-methyl-5-pyrazolon | gelb |
| 608 | H | 1-(4'-Sulfophenyl)-3-methyl-5-amino-pyrazol | gelb |

**Beispiele 609 bis 638**

In den nachfolgenden Tabellenbeispielen sind weitere erfindungsgemäße Azoverbindungen entsprechend der, in Form der freien Säure geschriebenen, allgemeinen Formel (H)

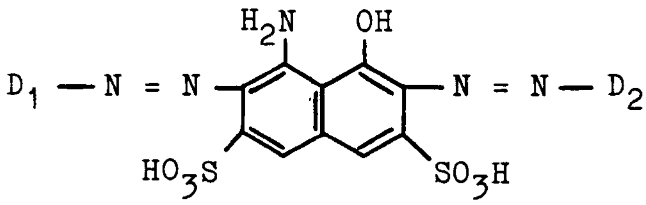

(H)

mit Hilfe ihrer Diazokomponenten $D_1$ und $D_2$ beschrieben. Sie lassen sich in erfindungsgemäßer Weise, beispielsweise gemäß den Ausführungsbeispielen 1 und 5, durch Kupplung der 1-Amino-8-naphthol-3,6-disulfonsäure mit den Diazoniumsalzen der aromatischen Aminoverbindungen $D_1$-$NH_2$ und $D_2$-$NH_2$ mit $D_1$ und $D_2$ der in dem jeweiligen Tabellenbeispiel angegebenen Bedeutungen zunächst im sauren Medium, das Diazoniumsalz von $D_1$-$NH_2$ betreffend, und sodann im schwach sauren bis neutralen Medium, das Diazoniumsalz von $D_2$-$NH_2$ betreffend, herstellen. Diese erfindungsgemäßen Disazoverbindungen besitzen ebenfalls sehr gute faserreaktive Farbstoffeigenschaften. Sie liefern farbstarke Färbungen und Drucke auf den in der Beschreibung genannten Materialien, wie insbesondere Cellulosefasermaterialien, nach den für faserreaktive Farbstoffe üblichen Applikations- und Fixiermethoden mit den in dem jeweiligen Tabellenbeispiel angegebenen Farbton (hier auf Baumwolle) mit guten Echtheitseigenschaften; die in Klammern angegebenen Werte sind die Absorptionsmaxima ($\lambda_{max}$) in nm.

| Bsp. | Rest $D^1$ | Rest $D^2$ | Farbton |
|---|---|---|---|
| 609 | 4-{N-[ß-(4'-ß'-Sulfato-ethylsulfonyl-phenyl)-ethyl]-amidocarbonyl}-phenyl | 2-Methoxy-(ß-sulfatoethyl-sulfonyl)-phenyl | marineblau (588) |
| 610 | dito | 2-Sulfo-4-(ß-sulfatoethyl-sulfonyl)-phenyl | marineblau (592) |
| 611 | dito | 2-Sulfo-5-(ß-sulfatoethyl-sulfonyl)-phenyl | marineblau (585) |
| 612 | dito | 1,5-Disulfo-naphth-2-yl | marineblau (597) |
| 613 | dito | 4-(ß-Sulfatoethyl-sulfonyl)-phenyl | marineblau (588) |
| 614 | dito | 4-{N-[ß-(4'-ß'-Sulfatoethylsulfo-nyl-phenyl)-ethyl]-amidocarbonyl}-phenyl | marineblau (585) |
| 615 | 2-Sulfo-5-(ß-sulfato-ethylsulfonyl)-phenyl | dito | marineblau |
| 616 | 4-(ß-Sulfatoethyl-sulfonyl)-phenyl | dito | marineblau |
| 617 | 1,5-Disulfo-naphth-2-yl | dito | marineblau |
| 618 | 2-Sulfo-4-(ß-sulfato-ethylsulfonyl)-phenyl | dito | marineblau |
| 619 | 4-{N-[ß-(4'-ß'-Sulfato-ethylsulfonyl-phenyl)-ethyl]-amidocarbonyl}-phenyl | 4-{N-[ß-(4'-ß'-Sulfatoethyl-sulfonyl-phenyl)-ethyl]-amidosulfo-nyl}-phenyl | marineblau |
| 620 | dito | 3-{N-[ß-(4'-ß'-Sulfatoethyl-sulfonyl-phenyl)-ethyl]-amidosulfo-nyl}-phenyl | marineblau (592) |
| 621 | dito | 3-{N-[ß-(4'-ß'-Sulfatoethylsulfo-nyl-phenyl)-ethyl]-amidocarbonyl}-phenyl | marineblau |

| Bsp. | Rest $D^1$ | Rest $D^2$ | Farbton |
|---|---|---|---|
| 622 | 3-{N-[ß-(4'-ß'-Sulfatoethylsulfonyl-phenyl)-ethyl]-amidocarbonyl}-phenyl | dito | marineblau |
| 623 | 3-{N-[ß-(4'-ß'-Sulfatoethylsulfonyl-phenyl)-ethyl]-amidosulfonyl}-phenyl | 3-{N-[ß-(4'-ß'-Sulfatoethylsulfonyl-phenyl)-ethyl]-amidosulfonyl}-phenyl | marineblau |
| 624 | 4-{N-[ß-(4'-ß'-Sulfatoethylsulfonyl-phenyl)-ethyl]-amidosulfonyl}-phenyl | 2-Methoxy-5-(ß-sulfatoethylsulfonyl)-phenyl | marineblau |
| 625 | dito | 3-(ß-Sulfatoethylsulfonyl)-phenyl | marineblau |
| 626 | dito | 4-(ß-Sulfatoethylsulfonyl)-phenyl | marineblau |
| 627 | dito | 2-Sulfo-5-(ß-sulfatoethylsulfonyl)-phenyl | marineblau |
| 628 | dito | 2-Sulfo-4-(ß-sulfatoethylsulfonyl)-phenyl | marineblau |
| 629 | dito | 1,5-Disulfo-naphth-2-yl | marineblau |
| 630 | 4-{N-[ß-(2'-Sulfo-4'-ß'-sulfatoethylsulfonyl-phenyl)-ethyl]-amidocarbonyl}-phenyl | 2-Methoxy-5-(ß-sulfatoethylsulfonyl)-phenyl | marineblau |
| 631 | dito | 3-(ß-Sulfatoethylsulfonyl)-phenyl | marineblau |
| 632 | dito | 4-(ß-Sulfatoethylsulfonyl)-phenyl | marineblau |
| 633 | 4-{N-[ß-(2'-Sulfo-4'-ß'-sulfatoethylsulfonyl-phenyl)-ethyl]-amidosulfonyl}-phenyl | dito | marineblau |
| 634 | dito | 3-(ß-Sulfatoethylsulfonyl)-phenyl | marineblau |

| Bsp. | Rest $D^1$ | Rest $D^2$ | Farbton |
|---|---|---|---|
| 635 | 3-(ß-Sulfatoethylsulfonyl)-phenyl | 3-{N-[ß-(4'-ß'-Sulfatoethylsulfonyl-phenyl)-ethyl]-amidosulfonyl}-phenyl | marineblau |
| 636 | 4-(ß-Sulfatoethyl-sulfonyl)-phenyl | dito | marineblau |
| 637 | dito | 4-(ß-Sulfatoethyl-sulfonyl)-phenyl | marineblau |
| 638 | 3-(ß-Sulfatoethyl-sulfonyl)-phenyl | 4-(ß-Sulfatoethyl-sulfonyl)-phenyl | marineblau |

## Patentansprüche

**Patentansprüche für folgende Vertragsstaaten : BE, CH, DE, FR, GB, IT, LI**

**1.** Eine wasserlösliche Azoverbindung der allgemeinen Formel (1)

$$D - N = N - K \quad (1)$$

in welcher bedeuten:

D ist ein Rest der allgemeinen Formel (2)

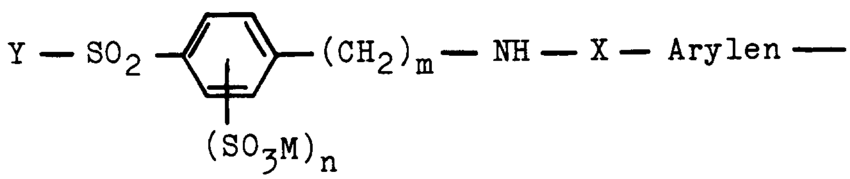

(2)

in welcher

Y die Vinylgruppe oder eine Gruppe der allgemeinen Formel (3)

$$-CH_2-CH_2-Z \quad (3)$$

ist, in welcher

Z ein Substituent ist, der durch ein Alkali unter Bildung der Vinylgruppe eliminierbar ist,

X die Carbonyl- oder Sulfonylgruppe ist,

Arylen ein Phenylen- oder Naphthylenrest ist, die beide durch eine Sulfogruppe, eine Alkylgruppe von 1 bis 4 C-Atomen, eine Alkoxygruppe von 1 bis 4 C-Atomen, eine Carboxygruppe oder ein Halogenatom substituiert sein können,

m die Zahl 1 oder 2 ist,

n für die Zahl Null oder 1 steht (wobei im Falle n = Null die Gruppe ein Wasserstoffatom bedeutet) und

M ein Wasserstoffatom oder ein salzbildendes Metallatom, bevorzugt ein Wasserstoffatom oder ein Alkalimetall, ist;

K ist ein Rest einer einfach ankuppelbaren wasserlöslichen Kupplungskomponente, die noch eine Azogruppe enthalten kann, oder der Rest einer doppelankuppelbaren wasserlöslichen Kupplungskomponente, jeweils aus der Reihe der Aminobenzole, der Phenole, insbesondere deren Sulfonsäuren und Carbonsäuren, der Naphthole, insbesondere deren Sulfonsäuren und Carbonsäuren, der Aminonaphthole, insbesondere deren Sulfonsäuren,

der Acylamino-naphthole, insbesondere deren Sulfonsäuren, mit dem Acylrest einer Alkan- oder Alkencarbonsäure mit jeweils 1 bis 4 bzw. 2 bis 4 C-Atomen im Alkyl- bzw. Alkenylrest oder einer aromatischen Carbonsäure oder einer aromatischen Sulfonsäure oder einer N-substituierten Carbaminsäure, oder aus der Reihe der Dihydroxynaphthalinsulfonsäuren, der Phenylazo- und Naphthylazo-aminonaphtholsulfonsäuren, der 5-Pyrazolone und 5-Aminopyrazole, der Acetoacetylarylide, der 2-Hydroxy-6-pyridone und der Hydroxychinoline, wobei K neben den in Farbstoffen üblichen Substituenten auch eine faserreaktive Gruppe enthalten kann.

**2.** Verbindung nach Anspruch 1, dadurch gekennzeichnet, daß
K ein Rest der allgemeinen Formel

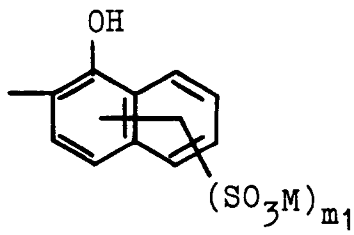

ist, in welcher $m_1$ für die Zahl 1, 2 oder 3 steht.
oder daß
K ein Rest der allgemeinen Formel

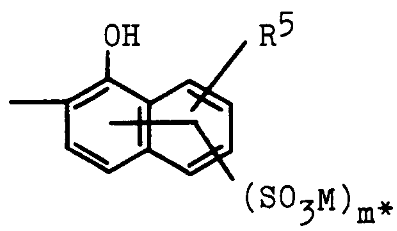

ist, in welcher $R^5$ die Benzoylaminogruppe oder eine Alkanoylaminogruppe von 2 bis 5 C-Atomen ist und m* für die Zahl 1 oder 2 steht.
oder daß
K ein Rest der allgemeinen Formel

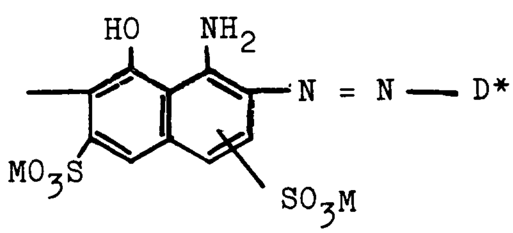

ist, in welcher D* ein Rest der allgemeinen Formel

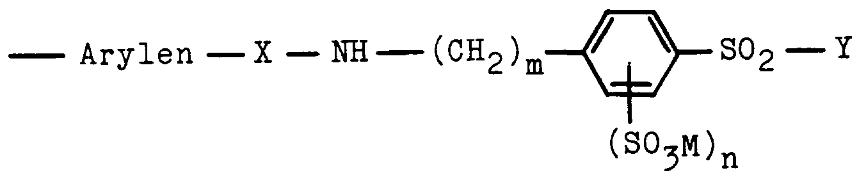

ist, worin Arylen, X, M, m, n und Y die in Anspruch 1 genannten Bedeutungen haben. oder daß K ein Rest der allgemeinen Formel

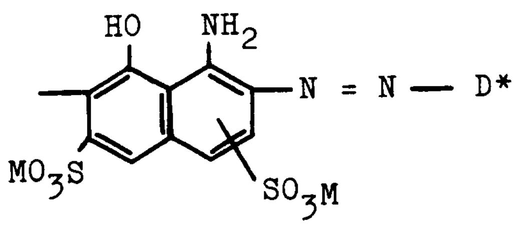

ist, in welcher M eine der in Anspruch 1 genannten Bedeutungen besitzt und D* ein Phenylrest ist, der durch 1, 2 oder 3 Substituenten aus der Gruppe Alkyl von 1 bis 4 C-Atomen, Alkoxy von 1 bis 4 C-Atomen, Chlor, Brom, Hydroxy, Carboxy, Sulfo, Carbamoyl, Sulfamoyl und Alkanoylamino von 2 bis 5 C-Atomen und/oder durch eine Gruppe der allgemeinen Formel $-SO_2-Y$ mit Y einer in Anspruch 1 genannten Bedeutung substituiert sein kann, oder ein Naphthylrest ist, der durch 1, 2 oder 3 Sulfogruppen oder durch 1 oder 2 Sulfogruppen und 1 oder 2 Gruppen der allgemeinen Formel $-SO_2-Y$ mit Y einer in Anspruch 1 genannten Bedeutung oder nur durch eine solche Gruppe $-SO_2-Y$ substituiert ist.

**3.** Verbindung nach Anspruch 1 oder 2, dadurch gekennzeichnet, daß n für die Zahl Null steht.

**4.** Verbindung nach mindestens einem der Ansprüche 1 bis 3, dadurch gekennzeichnet, daß m die Zahl 2 ist.

**5.** Verbindung nach mindestens einem der Ansprüche 1 bis 4, dadurch gekennzeichnet, daß Arylen ein unsubtituierter Phenylenrest ist.

**6.** Verbindung nach mindestens einem der Ansprüche 1 bis 5, dadurch gekennzeichnet, daß Y für die Vinylgruppe oder eine β-Sulfatoethyl-Gruppe steht.

**7.** Verfahren zur Herstellung einer Verbindung der in Anspruch 1 genannten und definierten allgemeinen Formel (1), dadurch gekennzeichnet, daß man eine Diazoniumverbindung einer Aminoverbindung der allgemeinen Formel (5)

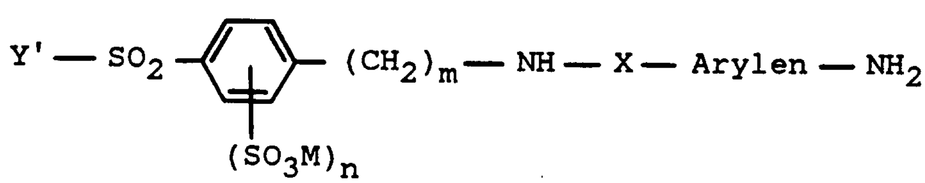

(5)

in welcher Arylen, X, M, m und n die in Anspruch 1 genannten Bedeutungen haben und Y' eine der Bedeutungen von Y besitzt oder die β-Hydroxyethylgrppe ist, mit einer Kupplungskomponente der allgemeinen Formel H-K mit K der in Anspruch 1 genannten Bedeutung kuppelt und gegebenenfalls die

gebildete Azoverbindung mit einer weiteren Diazoverbindung oder Kupplungskomponente umsetzt und gegebenenfalls die Gruppe Y', sofern sie für eine $\beta$-Hydroxyethyl-Gruppe stand, anschließend in eine Gruppe Y überführt.

**8.** Verwendung einer Verbindung entsprechend der allgemeinen Formel (1) von Anspruch 1 zum Färben (einschließlich Bedrucken) von hydroxy- und/oder carbonamidgruppenhaltigem Material, insbesondere Fasermaterial.

**9.** Verfahren zum Färben (einschließlich Bedrucken) von hydroxy- und/oder carbonamidgruppenhaltigem Material, insbesondere Fasermaterial, bei welchem man einen Farbstoff auf das Material aufbringt oder darin einbringt und ihn mittels Wärme und/oder mit Hilfe eines alkalisch wirkenden Mittels fixiert, dadurch gekennzeichnet, daß man als Farbstoff eine Verbindung entsprechend der allgemeinen Formel (1) von Anspruch 1 einsetzt.

**10.** Eine Verbindung, die der allgemeinen Formel (5)

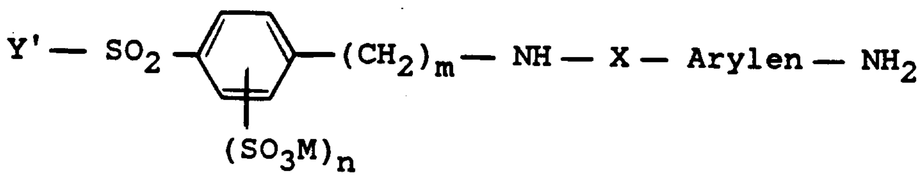

(5)

entspricht, in welcher

Y' die Vinylgruppe oder die $\beta$-Hydroxyethyl-Gruppe oder eine Gruppe der allgemeinen Formel (3)

$$-CH_2-CH_2-Z \quad (3)$$

bedeutet, in welcher

Z ein Substituent ist, der durch ein Alkali unter Bildung der Vinylgruppe eliminierbar ist,

X die Carbonyl- oder Sulfonylgruppe ist,

Arylen ein Phenylen- oder Naphthylenrest ist, die beide durch eine Sulfogruppe, eine Alkylgruppe von 1 bis 4 C-Atomen, eine Alkoxygruppe von 1 bis 4 C-Atomen, eine Carboxygruppe oder ein Halogenatom, substituiert sein können,

m die Zahl 1 oder 2, bevorzugt 2, ist,

n für die Zahl Null oder 1 steht (wobei im Falle von n = Null diese Gruppe ein Wasserstoffatom bedeutet), bevorzugt 1 ist, und

M ein Wasserstoffatom oder ein salzbildendes Metallatom, insbesondere ein Alkalimetallatom, ist.

**11.** Eine Verbindung nach Anspruch 10, in welcher Arylen für einen Benzolring steht und die Aminogruppe an den Benzolring in meta- oder para-Stellung zur Gruppe X gebunden ist.

**12.** Verfahren zur Herstellung der Verbindung der allgemeinen Formel (5) von Anspruch 10, dadurch gekennzeichnet, daß man ein Säurechlorid der allgemeinen Formel (6)

$$Cl-X-Arylen-NO_2 \quad (6)$$

in welcher Arylen und X die Anspruch 10 genannten Bedeutungen haben, mit einer Aminoverbindung der allgemeinen Formel (7)

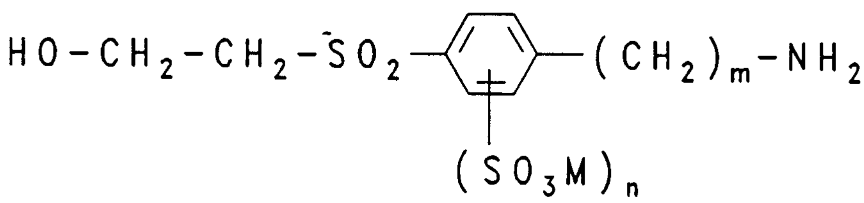

(7)

in welcher M, m und n die in Anspruch 10 genannten Bedeutungen haben, zur Verbindung der allgemeinen Formel (8)

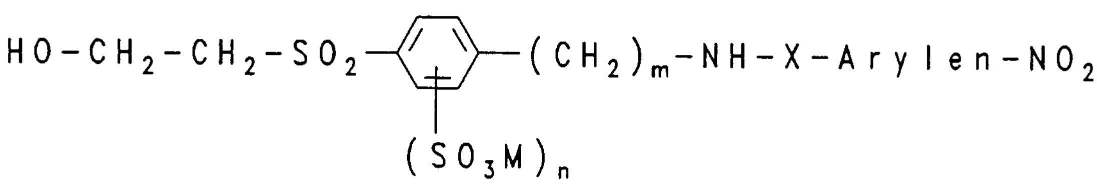

(8)

in welcher Arylen, X, M, m und n die in Anspruch 10 genannten Bedeutungen haben, umsetzt und die Verbindung der allgemeinen Formel (8) zur Aminoverbindung der allgemeinen Formel (5) reduziert und gewünschtenfalls die für den Formelrest Y' stehende $\beta$-Hydroxyethyl-Gruppe in eine andere Gruppe Y' überführt.

**13.** Verwendung einer Verbindung entsprechend der allgemeinen Formel (5) von Anspruch 10 zur Synthese von Farbstoffen, insbesondere Azofarbstoffen.

**Patentansprüche für folgenden Vertragsstaat : ES**

**1.** Verfahren zur Herstellung einer wasserlöslichen Azoverbindung der allgemeinen Formel (1)

$$D - N = N - K \quad (1)$$

in welcher bedeuten:

D ist ein Rest der allgemeinen Formel (2)

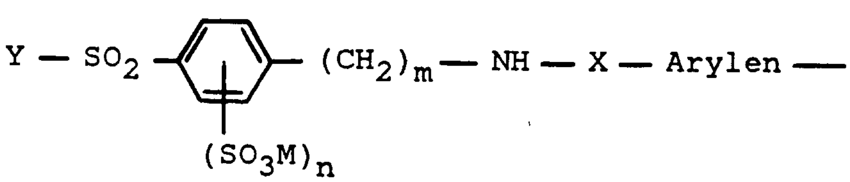

(2)

in welcher

Y die Vinylgruppe oder eine Gruppe der allgemeinen Formel (3)

$$- CH_2 - CH_2 - Z \quad (3)$$

ist, in welcher

Z ein Substituent ist, der durch ein Alkali unter Bildung der Vinylgruppe eliminierbar ist,

X die Carbonyl- oder Sulfonylgruppe ist,

Arylen ein Phenylen- oder Naphthylenrest ist, die beide durch eine Sulfogruppe, eine Alkylgruppe von 1 bis 4 C-Atomen, eine Alkoxygruppe von 1 bis 4 C-Atomen, eine Carboxygruppe oder ein Halogenatom substituiert sein können,

m die Zahl 1 oder 2 ist,

n für die Zahl Null oder 1 steht (wobei im Falle n = Null die Gruppe ein Wasserstoffatom bedeutet) und

M ein Wasserstoffatom oder ein salzbildendes Metallatom, bevorzugt ein Wasserstoffatom oder ein Alkalimetall ist;

K ist ein Rest einer einfach ankuppelbaren wasserlöslichen Kupplungskomponente, die noch eine Azogruppe enthalten kann, oder der Rest einer doppelankuppelbaren wasserlöslichen Kupplungskomponente, jeweils aus der Reihe der Aminobenzole, der Phenole, insbesondere deren Sulfonsäuren und Carbonsäuren, der Naphthole, insbesondere deren Sulfonsäuren und Carbonsäuren, der Aminonaphthole, insbesondere deren Sulfonsäuren, der Acylamino-naphthole, insbesondere deren Sulfonsäuren, mit dem Acylrest einer Alkan- oder Alkencarbonsäure mit jeweils 1 bis 4 bzw. 2 bis 4 C-Atomen im Alkyl- bzw. Alkenylrest oder einer aromatischen Carbonsäure oder einer aromatischen Sulfonsäure oder einer N-substituierten Carbaminsäure, oder aus der Reihe der Dihydroxynaphthalinsulfonsäuren, der Phenylazo- und Naphthylazo-aminonaphtholsulfonsäuren, der 5-Pyrazolone und 5-Aminopyrazole, der Acetoacetylarylide, der 2-Hydroxy-6-pyridone und der Hydroxychinoline, wobei K neben den in Farbstoffen üblichen Substituenten auch eine faserreaktive Gruppe enthalten kann;

dadurch gekennzeichnet, daß man eine Diazoniumverbindung einer Aminoverbindung der allgemeinen Formel (5)

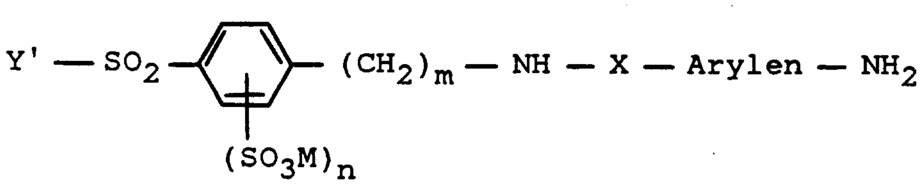

(5)

in welcher Arylen, X, M, m und n die oben genannten Bedeutungen haben und Y' eine der Bedeutungen von Y besitzt oder die $\beta$-Hydroxyethylgrppe ist, mit einer Kupplungskomponente der allgemeinen Formel H-K mit K der oben genannten Bedeutung kuppelt und gegebenenfalls die gebildete Azoverbindung mit einer weiteren Diazoverbindung oder Kupplungskomponente umsetzt und gegebenenfalls die Gruppe Y', sofern sie für eine $\beta$-Hydroxyethyl-Gruppe stand, anschließend in eine Gruppe Y überführt.

**2.** Verfahren nach Anspruch 1, dadurch gekennzeichnet, daß
K ein Rest der allgemeinen Formel

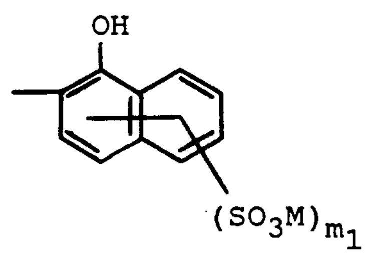

ist, in welcher $m_1$ für die Zahl 1, 2 oder 3 steht,
oder daß
K ein Rest der allgemeinen Formel

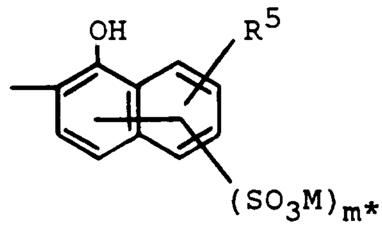

ist, in welcher $R^5$ die Benzoylaminogruppe oder eine Alkanoylaminogruppe von 2 bis 5 C-Atomen ist und m* für die Zahl 1 oder 2 steht,
oder daß
K ein Rest der allgemeinen Formel

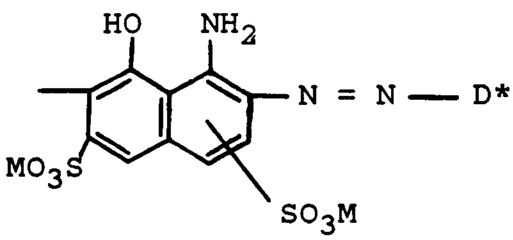

ist, in welcher D* ein Rest der allgemeinen Formel

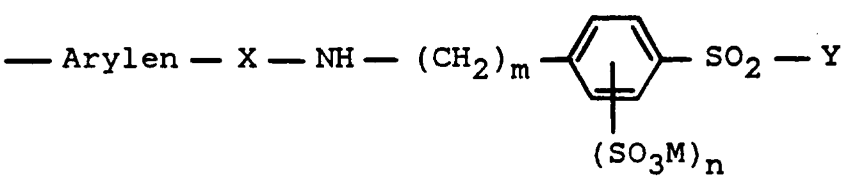

ist, worin Arylen, X, M, m, n und Y die in Anspruch 1 genannten Bedeutungen haben,
oder daß
K ein Rest der allgemeinen Formel

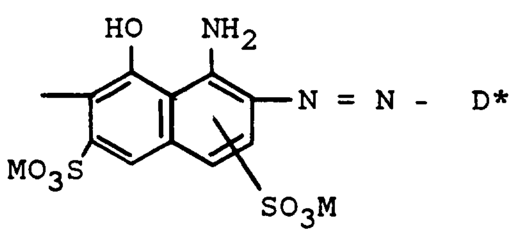

ist, in welcher M eine der in Anspruch 1 genannten Bedeutungen besitzt und D* ein Phenylrest ist, der durch 1, 2 oder 3 Substituenten aus der Gruppe Alkyl von 1 bis 4 C-Atomen, Alkoxy von 1 bis 4 C-Atomen, Chlor, Brom, Hydroxy, Carboxy, Sulfo, Carbamoyl, Sulfamoyl und Alkanoylamino von 2 bis 5 C-Atomen und/oder durch eine Gruppe der allgemeinen Formel $-SO_2-Y$ mit Y einer in Anspruch 1 genannten Bedeutung substituiert sein kann, oder ein Naphthylrest ist, der durch 1, 2 oder 3 Sulfogruppen oder durch 1 oder 2 Sulfogruppen und 1 oder 2 Gruppen der allgemeinen Formel $-SO_2-Y$ mit Y einer in Anspruch 1 genannten Bedeutung oder nur durch eine solche Gruppe $-SO_2-Y$ substituiert ist.

**3.** Verfahren nach Anspruch 1 oder 2, dadurch gekennzeichnet, daß n für die Zahl Null steht.

**4.** Verfahren nach mindestens einem der Ansprüche 1 bis 3, dadurch gekennzeichnet, daß m die Zahl 2 ist.

**5.** Verfahren nach mindestens einem der Ansprüche 1 bis 4, dadurch gekennzeichnet, daß Arylen ein unsubtituierter Phenylenrest ist.

**6.** Verfahren nach mindestens einem der Ansprüche 1 bis 5, dadurch gekennzeichnet, daß Y für die Vinylgruppe oder eine $\beta$-Sulfatoethyl-Gruppe steht.

**7.** Verfahren zum Färben (einschließlich Bedrucken) von hydroxy- und/oder carbonamidgruppenhaltigem Material, insbesondere Fasermaterial, bei welchem man einen Farbstoff auf das Material aufbringt oder darin einbringt und ihn mittels Wärme und/oder mit Hilfe eines alkalisch wirkenden Mittels fixiert, dadurch gekennzeichnet, daß man als Farbstoff eine Verbindung entsprechend der allgemeinen Formel (1) von Anspruch 1 einsetzt.

**8.** Verfahren zur Herstellung einer Verbindung, die der allgemeinen Formel (5)

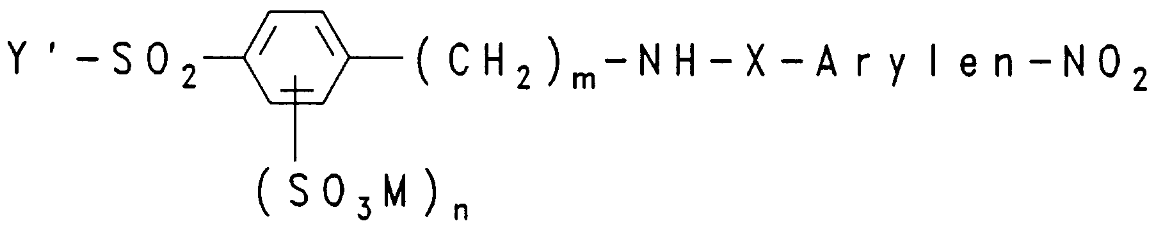

(5)

entspricht, in welcher

Y' die Vinylgruppe oder die $\beta$-Hydroxyethyl-Gruppe oder eine Gruppe der allgemeinen Formel (3)

$$-CH_2-CH_2-Z \quad (3)$$

bedeutet, in welcher

Z ein Substituent ist, der durch ein Alkali unter Bildung der Vinylgruppe eliminierbar ist,

X die Carbonyl- oder Sulfonylgruppe ist,

Arylen ein Phenylen- oder Naphthylenrest ist, die beide durch eine Sulfogruppe, eine Alkylgruppe von 1 bis 4 C-Atomen, eine Alkoxygruppe von 1 bis 4 C-Atomen, eine Carboxygruppe oder ein Halogenatom substituiert sein können,

m die Zahl 1 oder 2 ist, bevorzugt 2, ist,

n für die Zahl Null oder 1 steht (wobei im Falle von n = Null diese Gruppe ein Wasserstoffatom bedeutet) bevorzugt 1 ist, und

M ein Wasserstoffatom oder ein salzbildendes Metallatom, insbesondere ein Alkalimetallatom, ist,

dadurch gekennzeichnet, daß man ein Säurechlorid der allgemeinen Formel (6)

$$Cl-X-Arylen-NO_2 \quad (6)$$

in welcher Arylen und X die oben genannten Bedeutungen haben, mit einer Aminoverbindung der allgemeinen Formel (7)

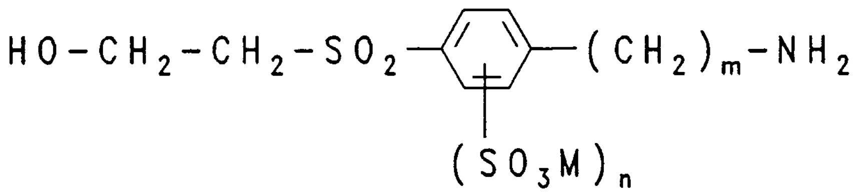

(7)

in welcher M, m und n die obengenannten Bedeutungen haben, zur Verbindung der allgemeinen Formel (8)

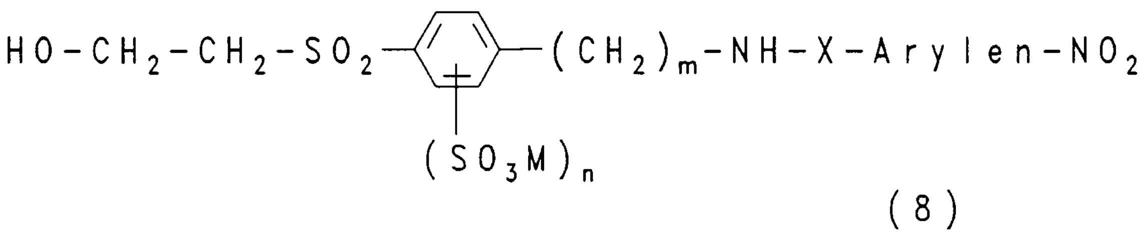

(8)

in welcher Arylen, X, M, m und n die obengenannten Bedeutungen haben, umsetzt und diese zur Aminoverbindung der allgemeinen Formel (5) reduziert und gewünschtenfalls die für den Formelrest Y' stehende $\beta$-Hydroxyethylgruppe in eine andere Gruppe Y' überführt.

**9.** Verfahren nach Anspruch 8, in welcher Arylen für einen Benzolring steht und die Aminogruppe an den Benzolring in meta- oder para-Stellung zur Gruppe X gebunden ist.

**Claims**

**Claims for the following Contracting States : BE, CH, DE, FR, GB, IT, LI**

**1.** A water-soluble azo compound of the formula (1)

D — N = N — K (1)

in which:

D is a radical of the formula (2)

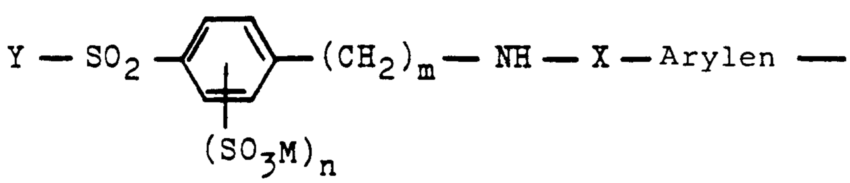

(2)

in which

Y is the vinyl group or a group of the formula (3)

$-CH_2-CH_2-Z$ (3)

in which

Z is a substituent, which can be removed by an alkali to form the vinyl group,

X is the carbonyl or sulfonyl group,

Arylen is a phenylene or naphthylene radical, both of which can be substituted by a sulfo group, an alkyl group having 1 to 4 carbon atoms, an alkoxy group having 1 to 4 carbon atoms, a carboxyl group or a halogen atom,

m is the number 1 or 2

n represents the number zero or 1 (and in the case where n = zero, the group is a

hydrogen atom), and

M is a hydrogen atom or a salt-forming metal atom, preferably a hydrogen atom or an alkali metal;

K is a radical of a water-soluble coupling component which can be coupled in one position and can also contain an azo group, or the radical of a water-soluble coupling component which can be coupled in two positions, in each case from the series comprising aminobenzenes, phenols, in particular sulfonic acids and carboxylic acids thereof, naphthols, in particular sulfonic acids and carboxylic acids thereof, aminonaphthols, in particular sulfonic acids thereof, and acylamino-naphthols, in particular sulfonic acids thereof, with the acyl radical of an alkane- or alkenecarboxylic acid having 1 to 4 or 2 to 4 carbon atoms in the alkyl or alkenyl radical respectively, or of an aromatic carboxylic acid or of an aromatic sulfonic acid, or of an N-substituted carbamic acid, or from the series comprising dihydroxynaphthalenesulfonic acids, phenylazo- and naphthylazo-aminonaphtholsulfonic acids, 5-pyrazolones and 5-aminopyrazoles, acetoacetyl arylides, 2-hydroxy-6-pyridones and hydroxyquinolines, it also being possible for K to contain, in addition to the substituents customary in dyestuffs, a fiber-reactive group.

**2.** A compound as claimed in claim 1, in which K is a radical of the formula

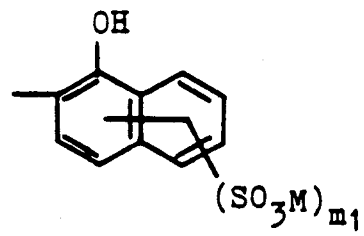

in which $m_1$ represents the number 1, 2 or 3, or in which K is a radical of the formula

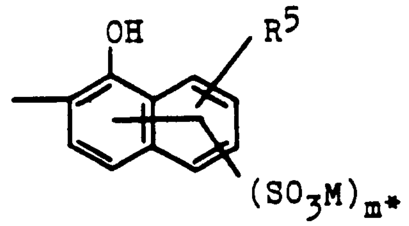

in which $R^5$ is the benzoylamino group or an alkanoylamino group having 2 to 5 carbon atoms and m* represents the number 1 or 2, or in which K is a radical of the formula

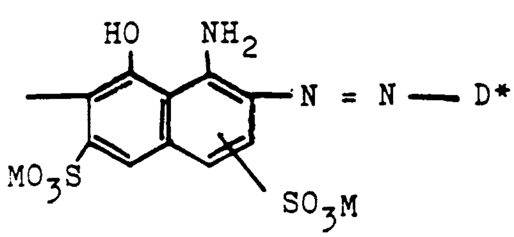

in which

D* is a radical of the formula

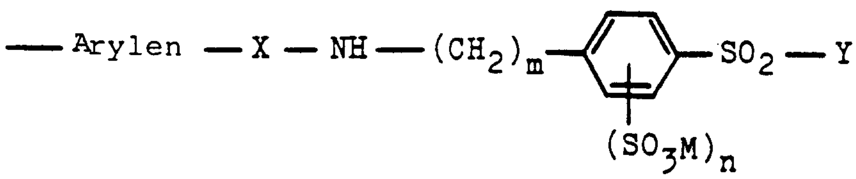

in which Arylen , X, M, m, n and Y have the meanings given in claim 1, or in which K is a radical of the formula

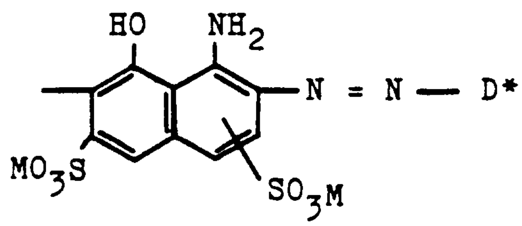

in which M has one of the meanings mentioned in claim 1 and D* is a phenyl radical, which can be substituted by 1, 2 or 3 substituents from the group comprising alkyl having 1 to 4 carbon atoms, alkoxy having 1 to 4 carbon atoms, chlorine, bromine, hydroxyl, carboxyl, sulfo, carbamoyl, sulfamoyl and alkanoylamino having 2 to 5 carbon atoms and/or by a group of the formula $-SO_2-Y$, where Y has a meaning given in claim 1, or is a naphthyl radical, which is substituted by 1, 2 or 3 sulfo groups or by 1 or 2 sulfo groups and 1 or 2 groups of the formula $-SO_2-Y$, where Y has a meaning given in claim 1, or by only one such group $-SO_2-Y$.

**3.** A compound as claimed in claim 1 or 2, in which n represents the number zero.

**4.** A compound as claimed in at least one of claims 1 to 3, in which m is the number 2.

**5.** A compound as claimed in at least one of claims 1 to 4, in which Arylen is an unsubstituted phenylene radical.

**6.** A compound as claimed in at least one of claims 1 to 5 in which Y represents the vinyl group or a $\beta$-sulfatoethyl group.

**7.** A process for the preparation of a compound of the formula (1) mentioned and defined in claim 1, which comprises coupling a diazonium compound of an amino compound of the formula (5)

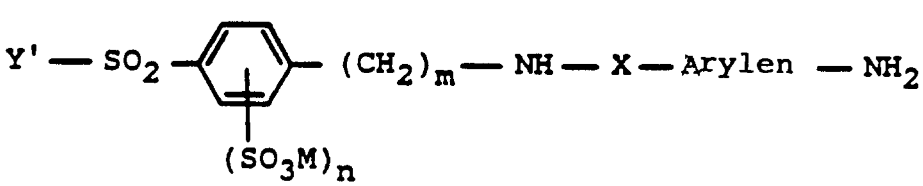

(5)

in which Arylen , X, M, m and n have the meanings given in claim 1 and Y' has one of the meanings of Y or is the $\beta$-hydroxyethyl group, with a coupling component of the formula H-K, where K has the meaning given in claim 1, and optionally reacting the azo compound formed with another diazo compound or coupling component and optionally then converting the group Y', as long as it was a $\beta$-hydroxyethyl group, into a group Y.

8. The use of a compound corresponding to the formula (1) of claim 1 for dyeing (including printing) material containing hydroxyl and/or carboxamide groups, in particular fiber material.

9. A process for dyeing (including printing) material containing hydroxyl and/or carboxamide groups, in particular fiber material, in which a dyestuff is applied to the material or incorporated therein and is fixed by means of heat and/or with the aid of an alkaline agent, which comprises using a compound corresponding to the formula (1) of claim 1 as the dyestuff.

10. A compound which corresponds to the formula (5)

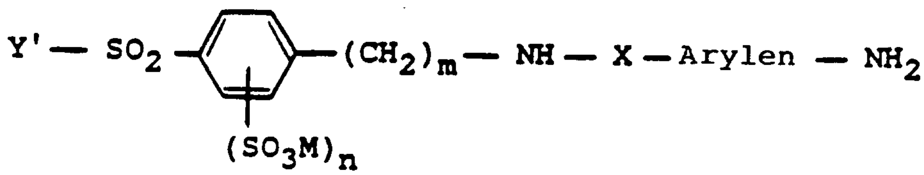

(5)

in which

Y' is the vinyl group or the $\beta$-hydroxyethyl group or a group of the formula (3)

$-CH_2-CH_2-Z$ (3)

in which

Z is a substituent, which can be removed by an alkali to form the vinyl group,
X is the carbonyl or sulfonyl group,
Arylen is a phenylene or naphthylene radical, both of which can be substituted by a sulfo group, an alkyl group having 1 to 4 carbon atoms, an alkoxy group having 1 to 4 carbon atoms, a carboxyl group or a halogen atom,
m is the number 1 or 2, preferably 2,
n represents the number zero or 1 (and in the case where n = zero, this group is a hydrogen atom), preferably 1, and
M is a hydrogen atom or a salt-forming metal atom, in particular an alkali metal atom.

11. A compound as claimed in claim 10, in which Arylen represents a benzene ring and the amino group is bonded to the benzene ring in the meta- or para-position relative to the group X.

12. A process for the preparation of a compound of claim 10, which comprises reacting an acid chloride of the formula (6)

$Cl-X-Arylen-NO_2$ (6)

in which Arylen and X have the meanings given in claim 10, with an amino compound of the general formula (7)

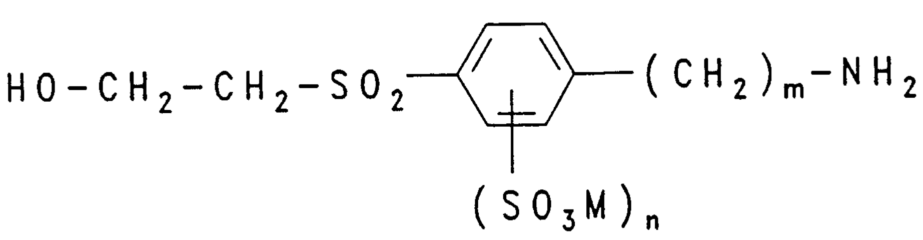

(7)

in which M, m and n have the meanings given in claim 10, to form the compound of the general formula (8)

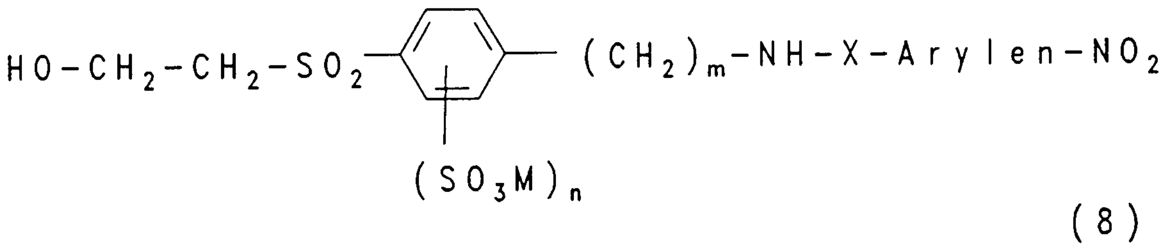

(8)

in which Arylen, X, M, m and n have the meanings given in claim 10, and reducing the compound of the general formula (8) to the amino compound of the general formula (5), and if desired converting the β-hydroxyethyl group represented by the formula radical Y' into another group Y'.

**13.** The use of a compound corresponding to the formula (5) of claim 10 for the synthesis of dyestuffs, in particular azo dyestuffs.

**Claims for the following Contracting State : ES**

**1.** A process for the preparation of a water-soluble azo compound of the formula (1)

$$D - N = N - K \quad (1)$$

in which:

D is a radical of the formula (2)

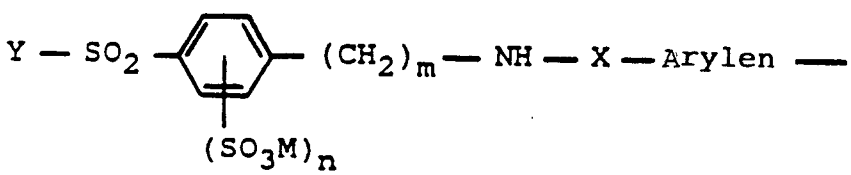

(2)

in which

Y is the vinyl group or a group of the formula (3)

$$- CH_2 - CH_2 - Z \quad (3)$$

in which

Z is a substituent, which can be removed by an alkali to form the vinyl group,

X is the carbonyl or sulfonyl group,

Arylen is a phenylene or naphthylene radical, both of which can be substituted by a sulfo group, an alkyl group having 1 to 4 carbon atoms, an alkoxy group having 1 to 4 carbon atoms, a carboxyl group or a halogen atom,

m is the number 1 or 2

n represents the number zero or 1 (and in the case where n = zero, the group is a hydrogen atom), and

M is a hydrogen atom or a salt-forming metal atom, preferably a hydrogen atom or an alkali metal;

K is a radical of a water-soluble coupling component which can be coupled in one position and can also contain an azo group, or the radical of a water-soluble coupling component which can be complied in two positions, in each case from the series comprising aminobenzenes, phenols, in particular sulfonic acids and carboxylic acids thereof, naphthols, in particular sulfonic acids and carboxylic acids thereof, aminonaphthols, in particular sulfonic acids thereof, and acylamino-naphthols, in particular sulfonic acids thereof, with the acyl radical of an alkane- or alkenecarboxylic acid having 1 to 4 or 2 to 4 carbon atoms in the alkyl or alkenyl radical respectively, or of an aromatic carboxylic acid or of

an aromatic sulfonic acid, or of an N-substituted carbamic acid, or from the series comprising dihydroxynaphthalenesulfonic acids, phenylazo- and naphthylazo-aminonaphtholsulfonic acids, 5-pyrazolones and 5-aminopyrazoles, acetoacetyl arylides, 2-hydroxy-6-pyridones and hydroxyquinolines, it also being possible for K to contain, in addition to the substituents customary in dyestuffs, a fiber-reactive group; which comprises coupling a diazonium compound of an amino compound of the formula (5)

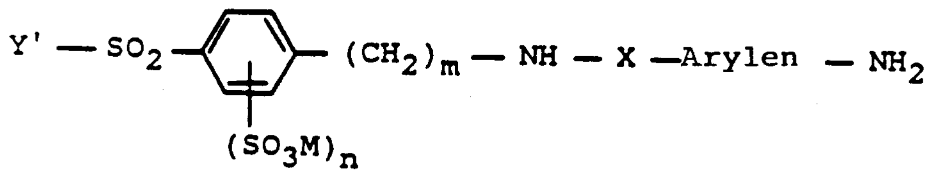

(5)

in which Arylen , X, M, m and n have the meanings given above and Y' has one of the meanings of Y or is the β-hydroxyethyl group, with a coupling component of the formula H-K, where K has the meaning given above, and optionally reacting the azo compound formed with another diazo compound or coupling component and optionally then converting tee group Y', as long as it was a β-hydroxyethyl group, into a group Y.

**2.** The process as claimed in claim 1, in which K is a radical of the formula

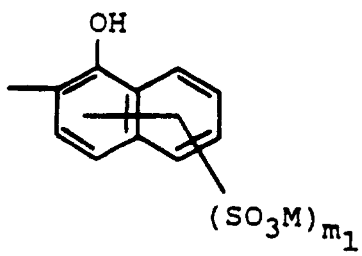

in which $m_1$ represents the number 1, 2 or 3, or in which K is a radical of the formula

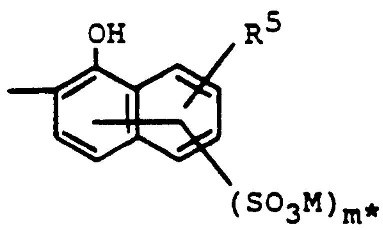

in which $R^5$ is the benzoylamino group or an alkanoylamino group having 2 to 5 carbon atoms and m* represents the number 1 or 2, or in which K is a radical of the formula

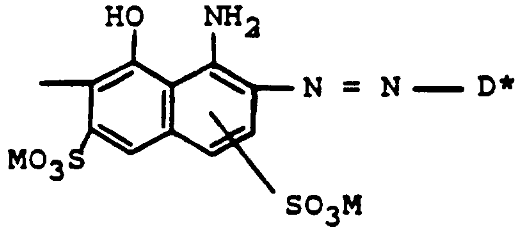

in which

D* is a radical of the formula

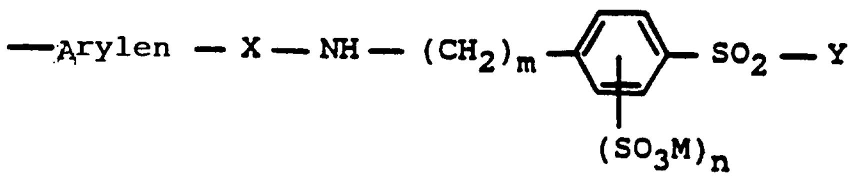

in which Arylen , X, M, m, n and Y have the meanings given in claim 1, or in which K is a radical of the formula

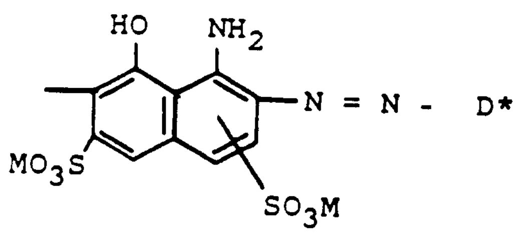

in which M has one of the meanings mentioned in claim 1 and D* is a phenyl radical, which can be substituted by 1, 2 or 3 substituents from the group comprising alkyl having 1 to 4 carbon atoms, alkoxy having 1 to 4 carbon atoms, chlorine, bromine, hydroxyl, carboxyl, sulfo, carbamoyl, sulfamoyl and alkanoylamino having 2 to 5 carbon atoms and/or by a group of the formula $-SO_2-Y$, where Y has a meaning given in claim 1, or is a naphthyl radical, which is substituted by 1, 2 or 3 sulfo groups or by 1 or 2 sulfo groups and 1 or 2 groups of the formula $-SO_2-Y$, where Y has a meaning given in claim 1, or by only one such group $SO_2-Y$.

3. The process as claimed in claim 1 or 2, in which n represents the number zero.

4. The process as claimed in at least one of claims 1 to 3, in which m is the number 2.

5. The process as claimed in at least one of claims 1 to 4, in which Arylen is an unsubstituted phenylene radical.

6. The process as claimed in at least one of claims 1 to 5 in which Y represents the vinyl group or a $\beta$-sulfatoethyl group.

7. A process for dyeing (including printing) material containing hydroxyl and/or carboxamide groups, in particular fiber material, in which a dyestuff is applied to the material or incorporated therein and is fixed by means of heat and/or with the aid of an alkaline agent, which comprises using a compound corresponding to the formula (1) of claim 1 as the dyestuff.

8. A process for the preparation of a compound which corresponds to the formula (5)

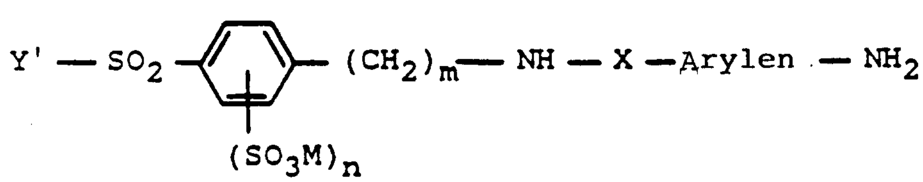

(5)

in which

Y' is the vinyl group or the $\beta$-hydroxyethyl group or a group of the formula (3)

$CH_2 - CH_2 - Z$ (3)

in which

Z is a substituent, which can be removed by an alkali to form the vinyl group,

X is the carbonyl or sulfonyl group,

Arylen is a phenylene or naphthylene radical, both of which can be substituted by a sulfo group, an alkyl group having 1 to 4 carbon atoms, an alkoxy group having 1 to 4 carbon atoms, a carboxyl group or a halogen atom,

m is the number 1 or 2, preferably 2,

n represents the number zero or 1 (and in the case where n = zero, this group is a hydrogen atom), preferably 1, and

M is a hydrogen atom or a salt-forming metal atom, in particular an alkali metal atom,

which comprises reacting an acid chloride of the formula (6)

$Cl - X - Arylen - NO_2$ (6)

in which Arylen and X have the meanings given above, with an amino compound of the general formula (7)

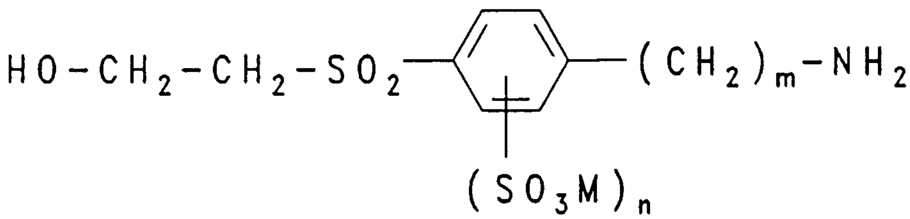

(7)

in which M, m and n have the above-mentioned meanings, to form the compound of the general formula (8)

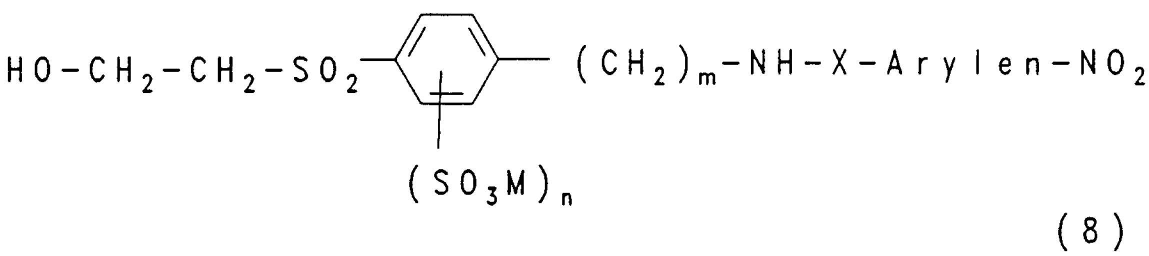

(8)

in which Arylen, X, M, m and n have the above-mentioned meanings, and reducing the compound of the general formula (8) to the amino compound of the general formula (5), and if desired converting the $\beta$-hydroxyethyl group represented by the formula radical Y' into another group Y'.

**9.** The process as claimed in claim 8, in which Arylen represents a benzene ring and the amino group is bonded to the benzene ring in the meta- or para-position relative to the group X.

**Revendications**

**Revendications pour les Etats contractants suivants : BE, CH, DE, FR, GB, IT, LI**

**1.** Composé azoïque soluble dans l'eau de formule générale (1)

$D - N = N - K$ (1)

dans laquelle :

D est un reste de formule générale (2)

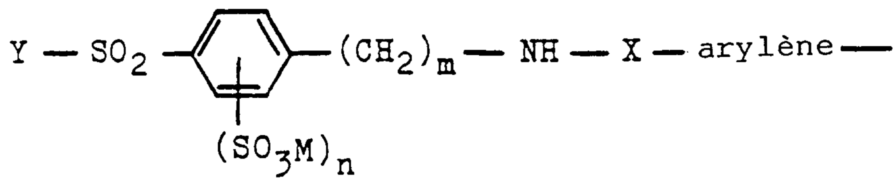

(2)

dans laquelle

Y est le groupe vinyle ou un groupe de formule générale (3)

$— CH_2 — CH_2 — Z$ (3)

dans laquelle

Z est un substituant qui peut être éliminé par un alcali avec la formation du groupe vinyle,

X est le groupe carbonyle ou sulfonyle,

l'arylène est un reste phénylène ou naphtylène qui peuvent être tous les deux substitués par un groupe sulfo, un groupe alkyle ayant de 1 à 4 atomes de C, un groupe alcoxy ayant de 1 à 4 atomes de C, un groupe carboxy ou un atome d'halogène,

m représente le nombre 1 ou 2,

n représente le nombre 0 ou 1 (de telle sorte que si n = 0 le groupe représente un atome d'hydrogène) et

M représente un atome d'hydrogène ou un atome de métal formant un sel, de préférence un atome d'hydrogène ou un métal alcalin;

K est un reste d'un composant de copulation soluble dans l'eau et pouvant copuler une fois, qui peut encore comporter un groupe azoïque, ou le reste d'un composant de copulation soluble dans l'eau apte à une double copulation, à chaque fois de la série des aminobenzènes, des phénols, en particulier de leurs acides sulfoniques et de leurs acides carboxyliques, des naphtols, en particulier de leurs acides sulfoniques et de leurs acides carboxyliques, des aminonaphtols, en particulier de leurs acides sulfoniques, des acylaminonaphtols, en particulier de leurs acides sulfoniques, avec le-reste acyle d'un acide carboxylique d'alcane ou d'alcène, le reste alkyle ou alcényle ayant chaque fois de 1 à 4 ou de 2 à 4 atomes de C, ou d'un acide carboxylique aromatique ou d'un acide sulfonique aromatique ou d'un acide carbamique substitué sur l'azote, ou de la série des acides dihydroxynaphtalène-sulfoniques, des acides phénylazo- et naphtylazo-aminonaphtolsulfoniques, des 5-pyrazolones et des 5-aminopyrazoles, des acétoacétylarylides, des 2-hydroxy-6-pyridones et des hydroxyquinoléines, où K, en plus des substituants habituels dans les colorants, peut aussi comporter un groupe réagissant avec des fibres.

**2.** Composé selon la revendication 1, caractérisé en ce que K est un reste de formule générale

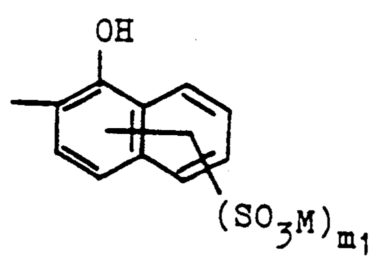

dans laquelle $m_1$ représente le nombre 1, 2 ou 3,
ou en ce que
K est un reste de formule générale

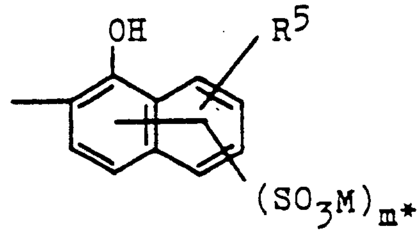

dans laquelle $R^5$ est le groupe benzoylamino ou un groupe alcanoylamino ayant de 2 à 5 atomes de C et m* représente le nombre 1 ou 2;
ou en ce que
K représente un reste de formule générale

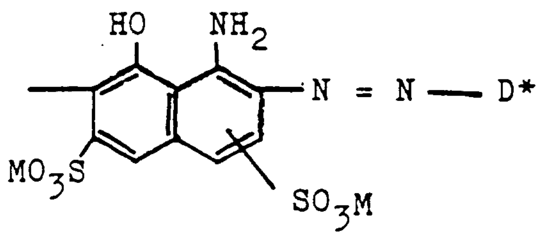

dans laquelle D* représente un reste de formule générale

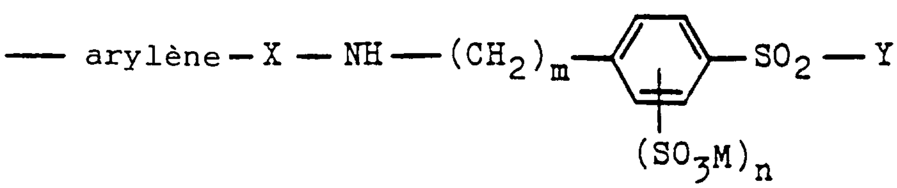

où l'arylène, X, M, m, n et Y ont les significations données à la revendication 1;
ou en ce que
K est un reste de formule générale

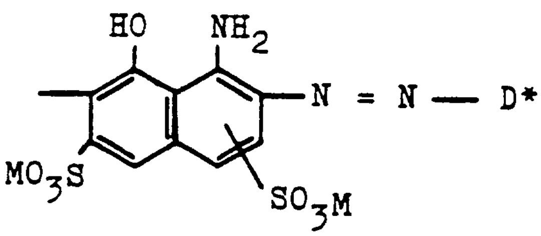

dans laquelle M a une des significations données à la revendication 1 et D* est un reste phényle qui peut être substitué par 1, 2 ou 3 substituants du groupe alkyle avec de 1 à 4 atomes de C, alcoxy avec de 1 à 4 atomes de C, chlore, brome, hydroxy, carboxy, sulfo, carbamoyle, sulfamoyle et alcanoylamino avec de 2 à 5 atomes de C et/ou par un groupe de formule générale $-SO_2-Y$, Y ayant une signification donnée dans la revendication 1, ou il est un reste naphtyle qui est substitué par 1, 2 ou 3 groupes sulfo ou par 1 ou 2 groupes sulfo et 1 ou 2 groupes de formule générale $-SO_2-Y$, Y ayant une signification donnée à la revendication 1, ou seulement par un tel groupe $-SO_2-Y$.

**3.** Composé selon la revendication 1 ou 2, caractérisé en ce que n représente le nombre 0.

**4.** Composé selon au moins une des revendications 1 à 3, caractérisé en ce que m est le nombre 2.

**5.** Composé selon au moins une des revendications 1 à 4, caractérisé en ce que l'arylène est un reste phénylène non substitué.

**6.** Composé selon au moins une des revendications 1 à 5, caractérisé en ce que Y représente le groupe vinyle ou un groupe $\beta$-sulfatoéthyle.

**7.** Procédé pour préparer un composé de formule générale (1) citée et définie dans la revendication 1, caractérisé en ce que l'on fait copuler un composé de diazonium d'un composé amino de formule générale (5)

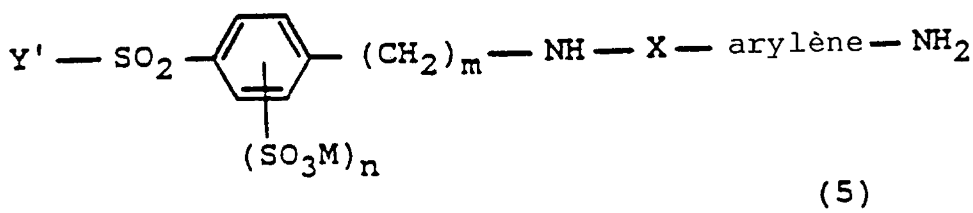

(5)

dans laquelle l'arylène, X, M, m et n ont les significations données dans la revendication 1 et Y' a une des significations de Y ou est le groupe $\beta$-hydroxyéthyle, avec un composant de copulation de formule générale H-K, K ayant la signification donnée dans la revendication 1, et en ce qu'éventuellement on fait réagir le composé azoïque formé avec un autre composé diazoïque ou un composant de copulation, et en ce qu'éventuellement on transforme ensuite le groupe Y', dans la mesure où il représente un groupe $\beta$-hydroxyéthyle, en un groupe Y.

**8.** Utilisation d'un composé correspondant à la formule générale (1) de la revendication 1 pour teindre (y compris par impression) une matière comportant des groupes hydroxy et/ou carboxamide, en particulier une matière fibreuse.

**9.** Procédé pour teindre (y compris par impression) une matière contenant des groupes hydroxy et/ou carboxamide, en particulier une matière fibreuse, dans lequel on applique un colorant sur la matière, ou on l'y introduit, et on fixe le colorant au moyen de la chaleur et/ou à l'aide d'un agent ayant une action alcaline, caractérisé en ce que l'on utilise comme colorant un composé correspondant à la formule générale (1) de la revendication 1.

**10.** Composé correspondant à la formule générale (5)

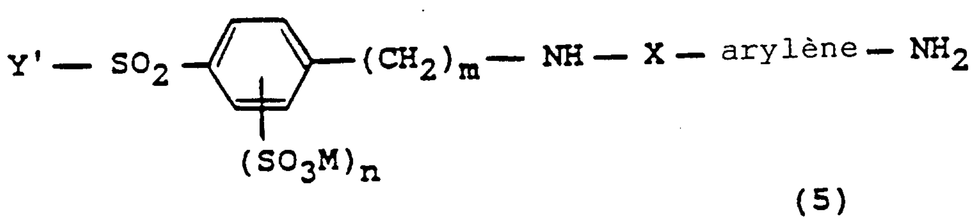

(5)

dans laquelle

Y' représente le groupe vinyle ou le groupe $\beta$-hydroxyéthyle ou un groupe de formule générale (3)

$$-CH_2-CH_2-Z \quad (3)$$

dans laquelle

Z est un substituant qui peut être éliminé par un alcali avec la formation du groupe vinyle,

X est le groupe carbonyle ou le groupe sulfonyle,

l'arylène est un reste phénylène ou naphtylène, qui peuvent être tous les deux substitués par un groupe sulfo, un groupe alkyle ayant de 1 à 4 atomes de C, un groupe alcoxy ayant de 1 à 4 atomes de C, un groupe carboxy ou un atome d'halogène,

m est le nombre 1 ou 2, de préférence 2,

n est le nombre 0 ou 1 (de telle sorte que si n = 0 ce groupe représente un atome d'hydrogène), de préférence 1, et

M est un atome d'hydrogène ou un atome de métal formant un sel, en particulier un atome de métal alcalin.

**11.** Composé selon la revendication 10, dans lequel 1' arylène représente un cycle benzénique et le groupe amino est lié au cycle benzénique en position méta ou para par rapport au groupe X.

**12.** Procédé pour produire le composé de formule générale (5) de la revendication 10, caractérisé en ce que l'on fait réagir un chlorure d'acide de formule générale (6)

$Cl — X — Arylen — NO_2$ (6)

dans laquelle l'arylène et X ont les significations données à la revendication 10 avec un composé amino de formule générale (7)

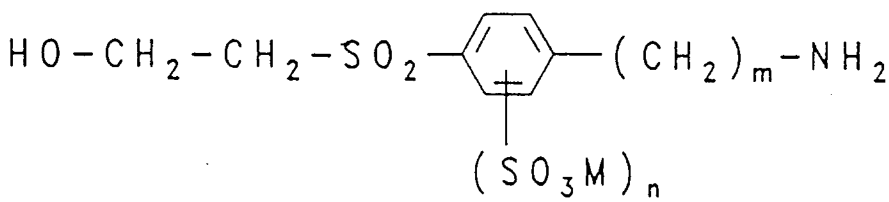

(7)

dans laquelle M, m et n ont les significations données à la revendication 10, pour obtenir un composé de formule générale (8)

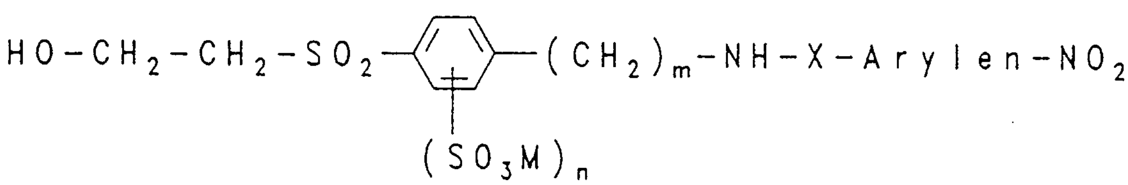

(8)

dans laquelle l'arylène, X, M, m et n ont les significations données à la revendication 10, et en ce qu'on réduit le composé de formule générale (8) pour obtenir un composé amino de formule générale (5) et en ce que, si on le désire, on transforme le groupe $\beta$-hydroxyéthyle, correspondant au reste Y' de la formule, en un autre groupe Y'.

**13.** Utilisation d'un composé correspondant à la formule générale (5) de la revendication 10 pour la synthèse de colorants, en particulier de colorants azoïques.

**Revendications pour l'Etat contractant suivant : ES**

**1.** Procédé pour préparer un composé azoïque soluble dans l'eau de formule générale (1)

D — N = N — K (1)

dans laquelle :

D est un reste de formule générale (2)

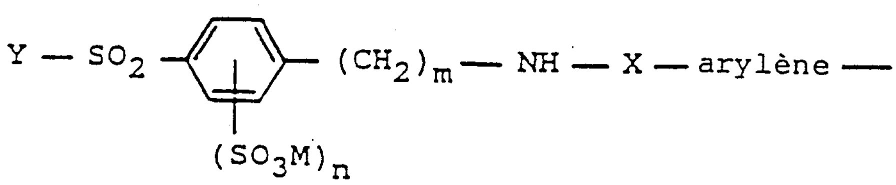

(2)

dans laquelle

Y est le groupe vinyle ou un groupe de formule générale (3)

$$-CH_2-CH_2-Z \quad (3)$$

dans laquelle

Z est un substituant qui peut être éliminé par un alcali avec la formation du groupe vinyle,

X est le groupe carbonyle ou sulfonyle,

l'arylène est un reste phénylène ou naphtylène qui peuvent être tous les deux substitués par un groupe sulfo, un groupe alkyle ayant de 1 à 4 atomes de C, un groupe alcoxy ayant de 1 à 4 atomes de C, un groupe carboxy ou un atome d'halogène,

m représente le nombre 1 ou 2,

n représente le nombre 0 ou 1 (de telle sorte que si n = 0 le groupe représente un atome d'hydrogène) et

M représente un atome d'hydrogène ou un atome de métal formant un sel, de préférence un atome d'hydrogène ou un métal alcalin;

K est un reste d'un composant de copulation soluble dans l'eau et pouvant copuler une fois, qui peut encore comporter un groupe azoïque, ou le reste d'un composant de copulation soluble dans l'eau apte à une double copulation, à chaque fois de la série des aminobenzènes, des phénols, en particulier de leurs acides sulfoniques et de leurs acides carboxyliques, des naphtols, en particulier de leurs acides sulfoniques et de leurs acides carboxyliques, des aminonaphtols, en particulier de leurs acides sulfoniques, des acylaminonaphtols, en particulier de leurs acides sulfoniques, avec le reste acyle d'un acide carboxylique d'alcane ou d'alcène, le reste alkyle ou alcényle ayant chaque fois de 1 à 4 ou de 2 à 4 atomes de C, ou d'un acide carboxylique aromatique ou d'un acide sulfonique aromatique ou d'un acide carbamique substitué sur l'azote, ou de la série des acides dihydroxynaphtalène-sulfoniques, des acides phénylazo- et naphtylazo-aminonaphtolsulfoniques, des 5-pyrazolones et des 5-aminopyrazoles, des acétoacétylarylides, des 2-hydroxy-6-pyridones et des hydroxyquinoléines, où K, en plus des substituants habituels dans les colorants, peut aussi comporter un groupe réagissant avec des fibres;

caractérisé en ce que l'on fait copuler un composé de diazonium d'un composé amino de formule générale (5)

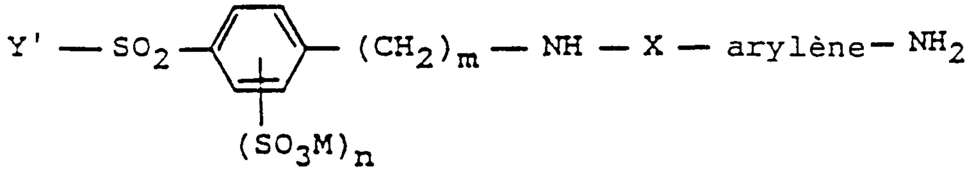

(5)

dans laquelle l'arylène, X, M, m et n ont les significations données précédemment et Y' a une des significations de Y ou est le groupe $\beta$-hydroxyéthyle, avec un composant de copulation de formule générale H-K, K ayant la signification donnée précédemment,

et en ce qu'éventuellement on fait réagir le composé azoïque formé avec un autre composé diazoïque ou un autre composant de copulation, et en ce qu'éventuellement on transforme ensuite le groupe Y', dans la mesure où il représente un groupe $\beta$-hydroxyéthyle, en un groupe Y.

**2.** Procédé selon la revendication 1, carctérisé en ce que K est un reste de formule générale

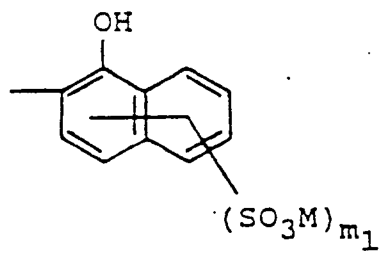

dans laquelle $m_1$ représente le nombre 1, 2 ou 3,
ou en ce que
K est un reste de formule générale

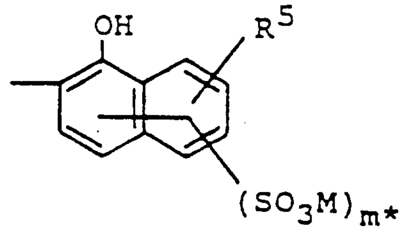

dans laquelle $R^5$ est le groupe benzoylamino ou un groupe alcanoylamino ayant de 2 à 5 atomes de C et m* représente le nombre 1 ou 2;
ou en ce que
K représente un reste de formule générale

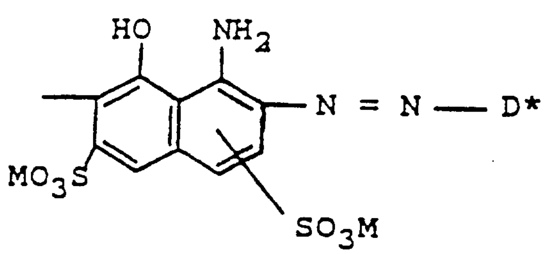

dans laquelle D* représente un reste de formule générale

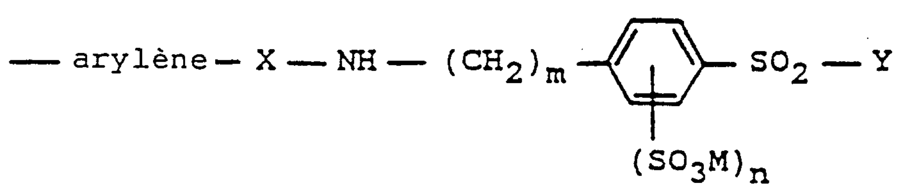

où l'arylène, X, M, m, n et Y ont les significations données à la revendication 1;
ou en ce que
K est un reste de formule générale

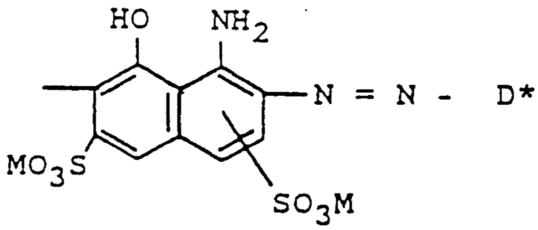

dans laquelle M a une des significations données à la revendication 1 et D* est un reste phényle qui peut être substitué par 1, 2 ou 3 substituants du groupe alkyle avec de 1 à 4 atomes de C, alcoxy avec de 1 à 4 atomes de C, chlore, brome, hydroxy, carboxy, sulfo, carbamoyle, sulfamoyle et alcanoylamino avec de 2 à 5 atomes de C et/ou par un groupe de formule générale $-SO_2-Y$, Y ayant une signification donnée dans la revendication 1, ou il est un reste naphtyle qui est substitué par 1, 2 ou 3 groupes sulfo ou par 1 ou 2 groupes sulfo et 1 ou 2 groupes de formule générale $-SO_2-Y$, Y ayant une signification donnée à la revendication 1, ou seulement par un tel groupe $-SO_2-Y$.

**3.** Procédé selon la revendication 1 ou 2, caractérisé en ce que n représente le nombre 0.

**4.** Procédé selon au moins une des revendications 1 à 3, caractérisé en ce que m est le nombre 2.

**5.** Procédé selon au moins une des revendications 1 à 4, caractérisé en ce que l'arylène est un reste phénylène non substitué.

**6.** Procédé selon au moins une des revendications 1 à 5, caractérisé en ce que Y représente le groupe vinyle ou un groupe β-sulfatoéthyle.

**7.** Procédé pour teindre (y compris par impression) une matière contenant des groupes hydroxy et/ou carboxamide, en particulier une matière fibreuse, dans lequel on applique un colorant sur la matière, ou on l'y introduit, et on fixe le colorant au moyen de la chaleur et/ou à l'aide d'un agent ayant une action alcaline, caractérisé en ce que l'on utilise comme colorant un composé correspondant à la formule générale (1) de la revendication 1.

**8.** Procédé pour obtenir un composé qui correspond à la formule générale (5)

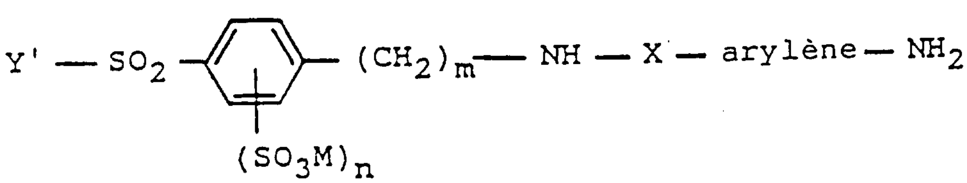

(5)

dans laquelle

Y' représente le groupe vinyle ou le groupe β-hydroxyéthyle ou un groupe de formule générale (3)

$$-CH_2-CH_2-Z \quad (3)$$

dans laquelle

Z est un substituant qui peut être éliminé par un alcali avec la formation du groupe vinyle,

X est le groupe carbonyle ou le groupe sulfonyle,

l'arylène est un reste phénylène ou naphtylène, qui peuvent être tous les deux substitués par un groupe sulfo, un groupe alkyle ayant de 1 à 4 atomes de C, un groupe alcoxy ayant de 1 à 4 atomes de C, un groupe carboxy ou un atome d'halogène,

m est le nombre 1 ou 2, de préférence 2,

n est le nombre 0 ou 1 (de telle sorte que si n = 0 ce groupe représente un atome

d'hydrogène), de préférence 1, et

M est un atome d'hydrogène ou un atome de métal formant un sel, en particulier un atome de métal alcalin;

caractérisé en ce que l'on fait réagir un chlorure d'acide de formule générale (6)

Cl — X — arylène — $NO_2$ (6)

dans laquelle l'arylène et X ont les significations données précédemment avec un composé amino de formule générale (7)

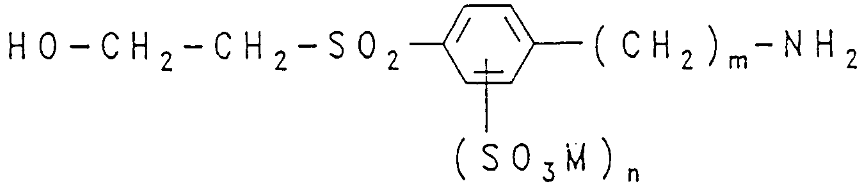

(7)

dans laquelle M, m et n ont les significations données précédemment, pour obtenir un composé de formule générale (8)

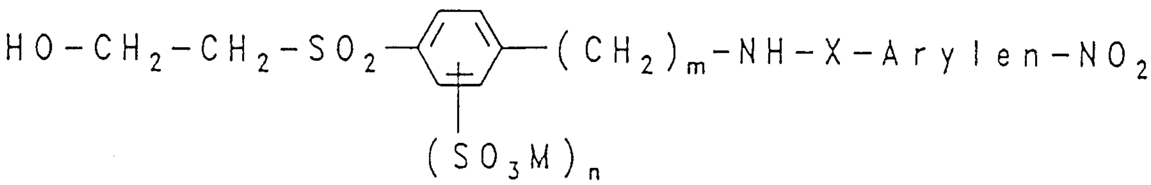

(8)

dans lequelle l'arylène, X, M, m et n ont les significations données précédemment, et en ce qu'on réduit ce dernier composé pour obtenir un composé amino de formule générale (5) et en ce que, si on le désire, on transforme le groupe $\beta$-hydroxyéthyle, correspondant au reste Y' de la formule, en un autre groupe Y'.

9. Procédé selon la revendication 8, dans lequel l'arylène représente un cycle benzénique et le groupe amino est lié au cycle benzénique en position méta ou para par rapport au groupe X.